(19) Europäisches Patentamt / European Patent Office / Office européen des brevets

(11) **EP 3 839 577 B1**

(12) **EUROPÄISCHE PATENTSCHRIFT**

(45) Veröffentlichungstag und Bekanntmachung des Hinweises auf die Patenterteilung:
**15.10.2025 Patentblatt 2025/42**

(21) Anmeldenummer: **19217564.4**

(22) Anmeldetag: **18.12.2019**

(51) Internationale Patentklassifikation (IPC):
*G01T 1/24* *(2006.01)*

(52) Gemeinsame Patentklassifikation (CPC):
**G01T 1/247**

(54) **VERFAHREN ZUR ERZEUGUNG EINES RÖNTGENBILDDATENSATZES**

METHOD FOR CREATING AN X-RAY IMAGE DATASET

PROCÉDÉ DE GÉNÉRATION D'UN ENSEMBLE DE DONNÉES DE RADIOGRAPHIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.06.2021 Patentblatt 2021/25**

(73) Patentinhaber: **Siemens Healthineers AG**
**91301 Forchheim (DE)**

(72) Erfinder:
• **Hupfer, Martin**
**91052 Erlangen (DE)**
• **Petersilka, Martin**
**91325 Adelsdorf (DE)**
• **Göderer, Edgar**
**91301 Forchheim (DE)**
• **Kreisler, Björn**
**91353 Hausen (DE)**

(74) Vertreter: **Siemens Healthineers**
**Patent Attorneys**
**Postfach 22 16 34**
**80506 München (DE)**

(56) Entgegenhaltungen:
DE-A1- 102012 224 209    US-A1- 2019 285 469
US-B2- 12 196 897    US-B2- 8 440 957
US-B2- 8 772 730

• HSIEH SCOTT S: "Coincidence Counters for Charge Sharing Compensation in Spectroscopic Photon Counting Detectors", IEEE TRANSACTIONS ON MEDICAL IMAGING, IEEE, USA, vol. 39, no. 3, 8 August 2019 (2019-08-08), pages 678 - 687, XP011775598, ISSN: 0278-0062, [retrieved on 20200302], DOI: 10.1109/ TMI.2019.2933986

**Beschreibung**

Die Erfindung betrifft ein Verfahren zur Erzeugung eines Röntgenbilddatensatzes, ein Röntgendetektorsystem mit einem photonenzählenden Röntgendetektor und ein medizinisches Bildgebungsgerät aufweisend ein Röntgendetektorsystem mit einem photonenzählenden Röntgendetektor.

Photonenzählende Röntgendetektoren finden in vielen bildgebenden Anwendungen Einsatz. So werden diese Röntgendetektoren beispielsweise in Computertomographen in der medizinischen Bildgebung genutzt, um ein tomographisches Röntgenbild eines Untersuchungsbereiches eines Patienten zu erzeugen.

Die Nutzung photonenzählender Detektoren in der Röntgenbildgebung bietet eine Reihe von Vorteilen gegenüber energieintegrierenden Detektoren. So ermöglichen sie eine hohe Ortsauflösung und eine intrinsisch energieaufgelöste Messung. Die Bildqualität heutiger photonenzählender Röntgendetektoren wird jedoch durch die endliche Ausdehnung der erzeugten Ladungswolken (sowie durch die Erzeugung charakteristischer Röntgenstrahlung) im Detektormaterial limitiert. Dies führt dazu, dass nicht immer die gesamte Energie des Röntgenphotons im getroffenen Pixel deponiert wird, sondern ein Teil der Energie in einem oder mehreren benachbarten Pixeln registriert wird. Als Folge werden zum einen Photonen unter der falschen Energie registriert, zum anderen können auch Photonen in benachbarten Pixeln mehrfach gezählt werden (= Koinzidenz). Diese Koinzidenzen verschlechtern nicht nur die spektralen Eigenschaften des Detektors, sondern führen auch ganz allgemein zu einer Verschlechterung der DQE (detektive Quanteneffizienz) des Detektors durch eine Erhöhung des Rauschens und eine Reduzierung der Ortsauflösung. Es handelt sich also um einen Effekt, welcher die Bildqualität für alle Anwendungen degradiert.

Der typische schaltungstechnische Ansatz zur Lösung des Problems besteht in der Implementierung sogenannter "charge summing"-Schaltungen ("Ladungssummations"-Schaltungen) in der Auswerteelektronik des Detektors. Hier wird während des Detektionsprozesses insbesondere im analogen Teil der Auswerteelektronik eines Pixels erkannt, dass Ladung in mehreren benachbarten Pixeln deponiert wurde und die gesamte Ladung aller Pixel wird einem Pixel (typischerweise dem mit der meisten Ladung oder dem schnellsten Anstieg des Stroms) zugeordnet. Dadurch werden sowohl Doppelzählungen verhindert als auch die ursprüngliche Ladung nahezu wiederhergestellt. Nachteilig an solchen Schaltungen ist, dass die Totzeit der Pixel durch solche Schaltungen massiv erhöht wird. Dadurch verschärft sich das Problem des sogenannten "Pulse-Pile Up" ("Aufeinanderstapeln der Pulse"), bei dem sich die Signale mehrerer Photonen überlagen und ebenfalls zu verfälschten Messungen führen. Eine gute Hochflussfähigkeit, wie z.B. in der Computertomographie gefordert, ist damit nicht mehr gegeben. Alternativ kann auch durch eine Erhöhung der Pixelgröße (z.B. auf > 0.3 mm Kantenlänge) der Verschlechterung der Energieauflösung und DQE entgegengewirkt werden, allerdings ebenfalls auf Kosten der Hochflussfähigkeit und zusätzlich noch auf Kosten des räumlichen Auflösungsvermögens.

In der DE 10 2011 077 859 B4 ist beispielsweise ein quantenzählender Strahlungsdetektor offenbart mit einem Array von Detektorelementen, die jeweils eine von der Energie auftreffender Strahlungsquanten abhängige Ladungsmenge erzeugen und zur Bildung größerer Detektoreinheiten in Gruppen benachbarter Detektorelemente eingeteilt sind, einer ersten Verarbeitungsstufe, durch die für jede der Gruppen jeweils ein elektrisches Signal bereitgestellt wird, das von der Summe der erzeugten Ladungsmengen der Detektorelemente der Gruppe abhängt, sowie einer zweiten Verarbeitungsstufe, durch die die auf die jeweiligen Gruppen auftreffenden Strahlungsquanten durch Auswertung der bereitgestellten elektrischen Signale gezählt werden, um für jede Gruppe ein Zählergebnis zu erhalten.

In "Coincidence counters for charge sharing compensation in spectroscopic photon counting detectors" von Scott S. Hsieh in IEEE Transactions on Medical Imaging (doi: 10.1109/TMI.2019.2933986) wird außerdem ein Koinzidenz-Zähler-Bin vorgeschlagen, dessen Implementierung existierenden Energie-Bins ähnelt.

Im Dokument DE 10 2021 224209 A1 ist ein zählender digitaler Röntgendetektor offenbart, welcher eine erste Schaltung und eine zweite Schaltung aufweist, wobei die erste Schaltung dazu ausgebildet ist, das in dem jeweiligen Pixelement direkt eingegangene Signal einzeln in ein Zählsignal zu wandeln und zu zählen und die zweite Schaltung dazu ausgebildet ist, das in dem jeweiligen Pixelement direkt eingegangene Signal gemeinsam mit koinzidierend auftretenden Signalen mindestens eines benachbarten Pixelelements in ein Zählsignal zu wandeln und zu zählen.

Aufgabe der Erfindung ist es eine Möglichkeit für eine verbesserte Erzeugung von Röntgenbilddatensätzen unter Beachtung einer Koinzidenzinformation bereitzustellen.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft ein Verfahren gemäß Anspruch 1.

Vorteilhaft kann mittels des erfindungsgemäßen Verfahrens durch die Bereitstellung der Koinzidenzinformation eine Berücksichtigung der Koinzidenzinformation im Schritt des Erzeugens ermöglicht werden und eine Reduktion der Bildqualität eines erzeugten Röntgenbilddatensatzes auf Grund auftretender Koinzidenzen in den Pixelelementen des Röntgendetektors reduziert werden. Die erfindungsgemäße Implementierung erlaubt dabei gleichzeitig negative Auswirkungen auf die Hochflussfähigkeit und verlängerte Totzeiten der Pixelelemente zu vermeiden, indem eine zeitaufwendige Echtzeitkorrektur während der Messung selbst umgangen wird. Darüber hinaus kann die Prozesskette zur Erzeugung des Röntgenbilddatensatzes vorteilhaft angepasst werden und gegebenenfalls unterschiedliche Korrekturmethoden oder auch Kombinationen von Korrekturmethoden basierend auf den bereitgestellten Anzahlen an Koinzidenz-Zählsignalen, gegebenenfalls auch nachträglich und wiederholt, auf die in jedem Pixelelement gezählte zumindest eine Anzahl an Zählsignalen oder auf einen darauf basierenden (vorläufigen) Röntgenbilddatensatz angewendet werden.

Dabei kann die Teilzahl der Vielzahl an Pixelelementen die gesamte Vielzahl an Pixelelementen umfassen. Die Vielzahl kann jedoch auch neben der Teilzahl an Pixelelementen anderweitig ausgebildete Pixelelemente umfassen. Diese können beispielsweise lediglich ausgebildet sein Zählsignale zu bilden und zu zählen. Indem lediglich ein Teil der Pixelelemente der Vielzahl an Pixelelementen ausgebildet ist Koinzidenz-Zählsignale zu bilden und zu zählen, kann beispielsweise eine vereinfachte Verschaltung der Pixelelemente ermöglicht werden.

Das zumindest eine weitere Pixelelement der Vielzahl an Pixelelementen, auf welchem ein zu zählendes Koinzidenz-Zählsignal basiert, kann von der zumindest einen Teilzahl der Vielzahl an Pixelelementen umfasst sein, d.h. selbst Teil der Teilzahl der Vielzahl an Pixelelementen sein. Es kann jedoch auch Ausbildungen geben, in welchem das zumindest eine weitere Pixelelement nicht Teil der mindestens Teilzahl der Vielzahl an Pixelelementen ist.

Im Rahmen des erfindungsgemäßen Verfahrens können auch mehr als eine Anzahl an Zählsignalen in einem jeweiligen Pixelelement der Vielzahl an Pixelelementen gezählt werden. Es können ebenso auch mehr als eine Anzahl an Koinzidenz-Zählsignalen in einem jeweiligen Pixelelement der Teilzahl der Vielzahl an Pixelelementen gezählt werden. Die mehreren Anzahlen an Zählsignalen und/oder mehreren Anzahlen an Koinzidenz-Zählsignalen können dann in das Erzeugen des Röntgenbilddatensatzes mit eingehen. Beispielsweise können in jedem Pixelelement der Vielzahl an Pixelelementen mehrere Anzahlen an Zählsignalen in Abhängigkeit mehrere für energieauflösende Messungen bereitgestellter Energieschwellen gezählt werden. Ebenso können in jedem Pixelelement der Teilzahl der Vielzahl an Pixelelementen mehrere Anzahlen an Koinzidenz-Zählsignalen in Abhängigkeit mehrere für energieauflösende Messungen bereitgestellter Energieschwellen gezählt werden. Darüber hinaus können in jedem Pixelelement der Teilzahl der Vielzahl an Pixelelementen eine Anzahl oder mehrere Anzahlen an Koinzidenz-Zählsignalen in Abhängigkeit mehrerer weiterer Pixelelemente der Vielzahl an Pixelelementen gezählt werden. Wird im Folgenden also von der zumindest einen Anzahl an Zählsignalen bzw. Koinzidenz-Zählsignalen gesprochen, kann dies umfassen, dass es darüber hinaus weitere Anzahlen geben kann, welche in ähnlicher Weise gezählt in das Erzeugen eingehen können. Wird im Folgenden allgemein von Koinzidenzinformation gesprochen, so umfasst dies zumindest die in einem jedem Pixelelement der Teilzahl der Vielzahl an Pixelelementen gezählte zumindest eine Anzahl an Koinzidenz-Zählsignale und kann darüber hinaus, wenn mehrere Anzahlen gezählt werden, die mehreren gezählten Anzahlen an Koinzidenz-Zählsignalen umfassen. Darüber hinaus kann die ermittelte Koinzidenzinformation außerdem eine oder mehrere von Pixelelementen der Teilzahl an Pixelelementen auf weitere Pixelelemente der Vielzahl an Pixelelemente übertragene Anzahlen an Koinzidenz-Zählsignalen umfassen. Wird im Folgenden allgemein von Zählinformation gesprochen, so umfasst dies zumindest die in einem jedem Pixelelement der Vielzahl an Pixelelementen gezählte zumindest eine Anzahl an Zählsignalen und kann darüber hinaus, wenn mehrere Anzahlen gezählt werden, die mehreren gezählten Anzahlen an Zählsignalen umfassen.

Gemäß einer Ausführungsvariante des erfindungsgemäßen Verfahrens geht im Schritt des Erzeugens die in den Pixelelementen der Teilzahl der Vielzahl an Pixelelementen gezählte zumindest eine Anzahl an Koinzidenz-Zählsignalen in die Datenvorverarbeitung vor einer Bildrekonstruktion, in die Bildrekonstruktion oder in einen der Bildrekonstruktion nachgelagerten Nachverarbeitungsschritt ein.

Die Erfinder haben erkannt, dass in einem jeden der Teilschritte die Koinzidenzinformation für Korrekturen hinsichtlich der auftretenden Koinzidenzen eingesetzt werden kann, um eine verbesserte Bildqualität zu ermöglichen. Es kann auch eine Kombination der drei Möglichkeiten vorliegen. Das heißt, die zumindest eine Anzahl an Koinzidenz-Zählsignalen kann sowohl in die Datenvorverarbeitung vor einer Bildrekonstruktion als auch in die Bildrekonstruktion und/oder in einen der Bildrekonstruktion nachgelagerten Nachverarbeitungsschritt eingehen. Daneben sind auch anderweitige Kombination möglich. Durch das Zählen und damit Bereitstellen der Koinzidenzinformation kann ein nachträgliches und wiederholtes Eingehen und ein Eingehen auch in unterschiedlichen dem Messprozess selbst nachgelagerten Verarbeitungsstufen des Erzeugens ermöglicht werden.

Das Eingehen der zumindest einen Anzahl an Koinzidenz-Zählsignalen in die Datenvorverarbeitung vor einer Bildrekonstruktion kann beispielsweise umfassen, dass die zumindest in jedem Pixelelement der Teilzahl der Vielzahl an Pixelelementen die zumindest eine Anzahl an Zählsignalen mittels der zumindest einen Anzahl an Koinzidenz-Zählsignalen angepasst wird. Die zumindest eine Anzahl an Koinzidenz-Zählsignalen kann auf die zumindest eine Anzahl an Zählsignalen angewendet werden, woraufhin vorteilhaft eine angepasste Anzahl an Zählsignalen für das Erzeugen bereitgestellt wird. Dadurch kann eine Korrektur der Anzahlen in Bezug auf auftretende Koinzidenzen ermöglicht werden. Die Bildrekonstruktion kann dann auf der angepassten zumindest einen Anzahl an Zählsignalen in jedem Pixelelement der Teilzahl an Pixelelementen basieren.

Beispielsweise kann zumindest in jedem Pixelelement der Teilzahl der Vielzahl an Pixelelementen die zumindest eine Anzahl an Koinzidenz-Zählsignalen von der zumindest einen Anzahl an Zählsignalen subtrahiert oder zu der zumindest einen Anzahl an Zählsignalen addiert werden. Eine Subtraktion oder Addition kann dabei eine gewichtete Subtraktion oder Addition umfassen. Das heißt, es kann lediglich einen Anteil oder ein Mehrfaches der zumindest einen Anzahl an Koinzidenz-Zählsignalen subtrahiert oder addiert werden. Es kann jedoch auch andere Implementierungen geben mittels derer zumindest eine Anzahl an Zählsignalen angepasst werden kann, beispielsweise eine Multiplikation, Division oder auch anderweitige, ggf. auch komplexere, Algorithmen. Vorteilhaft können durch Koinzidenzen beeinträchtige Anzahlen an Zählsignalen in den Pixelelementen direkt und einfach korrigiert werden und für die Bildrekonstruktion bereitgestellt werden.

Werden mehrere Anzahlen an Zählsignalen, beispielsweise in Abhängigkeit mehrerer Energieschwellen, in den Pixelelementen gezählt kann jede oder auch nur ein Teil der Anzahlen mit Hilfe der ermittelten Koinzidenzinformation angepasst werden. Werden ebenso mehrere Anzahlen an Koinzidenz-Zählsignalen ermittelt, beispielsweise in Abhängigkeit mehrerer Energieschwellen, können unterschiedliche Anzahlen an Koinzidenz-Zählsignalen auf unterschiedliche Anzahlen an Zählsignalen für ein Anpassen angewendet werden.

Beispielsweise kann der Schritt des Erzeugens auch das Anwenden einer trainierten Funktion umfassen, wobei die zumindest eine Anzahl an Koinzidenz-Zählsignale in zumindest einem Pixelelement der Teilzahl der Vielzahl an Pixelelementen als Eingabeparameter in die trainierte Funktion eingeht. Die trainierte Funktion kann beispielsweise im Schritt des Vorverarbeitens für eine Anpassung bzw. Korrektur der Anzahlen an Zählsignalen eingesetzt werden. Dabei kann die trainierte Funktion durch ein Verfahren des maschinellen Lernens trainiert sein. Insbesondere kann die trainierte Funktion ein neuronales Netzwerk, insbesondere ein faltendes neuronales Netzwerk (engl. convolutional neural network, CNN) bzw. ein Netzwerk umfassend eine Faltungsschicht (engl. convolutional layer) sein.

Eine trainierte Funktion bildet Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Ausgabedaten auf die Trainingseingabedaten angewendet wird. Im Allgemeinen wird auch eine trainierbare Funktion, d.h. eine Funktion mit noch nicht angepassten einen oder mehreren Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk, wobei die Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (engl. deep neural network, deep artificial neural network). Weiterhin sind auch insbesondere andere Algorithmen des maschinellen Lernens als trainierte Funktion einsetzbar. Die trainierte Funktion kann beispielsweise mittels einer Rückpropagation trainiert sein. Die Trainingseingabedaten können beispielweise eine Vielzahl an gemessenen und/oder simulierten Trainingsbilddatensätze aufweisend eine Beeinträchtigung durch auftretende Koinzidenzen umfassen. Die Trainingsausgabedaten können beispielweise gemessene und/oder simulierte Trainingsbilddatensätze umfassen, in welchen Koinzidenzen unterdrückt wurden, beispielsweise durch geeignete Pixelschaltungen oder durch geeignete Annahmen in der Simulation.

Durch Anwenden eines künstlichen Intelligenzsystems, d.h. einer trainierten Funktion, können alle relevanten Einflussgrößen für das Anwenden der Koinzidenzinformation berücksichtigt werden, auch solche, für die ein Anwender keinen Zusammenhang abschätzen kann. Insbesondere kann mittels einer trainierten Funktion nach der Trainingsphase eine Korrektur der Zählsignale mit Hilfe der Koinzidenz-Zählsignale besonders verlässlich und zeiteffizient automatisiert ermöglicht werden.

Das Eingehen der ermittelten zumindest einen Anzahl an Koinzidenz-Zählsignalen kann auch vor einer Übertragung der ermittelten zumindest einen Anzahl an Zählsignale von einem Röntgendetektor zu einer Erzeugungseinheit, welche für eine Bildrekonstruktion ausgebildet ist, durchgeführt werden oder aber auch erst nach einer Übertragung der Zählsignale von dem Röntgendetektor an eine Erzeugungseinheit. Das heißt, die Koinzidenzinformation kann etwa im Rahmen eines "on-board processing" (On-Board-Verarbeitung) auf die zumindest eine Anzahl an Zählsignalen angewendet werden, so dass lediglich die angepasste zumindest eine Anzahl an Zählsignalen vom Röntgendetektor ausgelesen und zu der Erzeugungseinheit übertragen werden muss. Dadurch kann vorteilhaft Übertragungsbandbreite gespart werden.

Das Eingehen der zumindest einen Anzahl an Koinzidenz-Zählsignalen in die Bildrekonstruktion des Röntgenbilddatensatzes kann beispielsweise umfassen, dass die Koinzidenzinformation in einen iterativen Rekonstruktionsalgorithmus eingeht. Beispielsweise kann die Koinzidenzinformation bei der Vorwärtsprojektion im Rahmen einer modellbasierten iterativen Rekonstruktion verwendet werden und zu einer verbesserten Bildrekonstruktion durch das Bereitstellen der zusätzlichen Information beitragen. Daneben kann es auch anderweitige Möglichkeiten geben.

Das Eingehen der in jedem Pixelelement der zumindest einen Anzahl an Koinzidenz-Zählsignalen in einen der Bildrekonstruktion nachgelagerten Nachverarbeitungsschritt kann beispielsweise umfassen, dass sowohl basierend auf der ermittelten Koinzidenzinformation Koinzidenz-Bilddaten als auch auf der Zählinformation vorläufige Bilddaten mittels eines Rekonstruktionsalgorithmus erzeugt werden. Die Koinzidenz-Bilddaten können dann auf die vorläufigen Bilddaten zur Verbesserung der Bildqualität angewendet werden, um einen finalen, verbesserten Röntgenbilddatensatz zu erzeugen. Gemäß einer Variante des Verfahrens kann es vorgesehen sein, dass basierend auf der zumindest einen Anzahl an gezählten Zählsignalen in jedem Pixelelement der Vielzahl an Pixelelementen ein vorläufiger Bilddatensatz erzeugt wird, und dass basierend auf der zumindest einen Anzahl an gezählten Koinzidenz-Zählsignalen in jedem Pixelelement der Teilzahl der Vielzahl an Pixelelementen ein Koinzidenz-Bilddatensatz erzeugt wird, welcher im Schritt des Erzeugens des Röntgenbilddatensatzes auf den vorläufigen Bilddatensatz angewendet wird. Eine besonders einfache Variante des Anwendens kann dabei beispielsweise eine Linearkombination der Koinzidenz-Bilddaten mit den vorläufigen Bilddaten umfassen. Daneben sind jedoch auch anderweitige Implementierungen möglich.

Vorteilhaft kann zeitnah ein erster vorläufiger Bilddatensatz bereitgestellt werden. Vorteilhaft kann bedarfsgemäß, beispielsweise nach einer ersten Begutachtung des vorläufigen Bilddatensatzes, und variabel basierend auf den rekonstruierten Bilddaten eine Verbesserung der Bildqualität mittels der Koinzidenzinformation ermöglicht werden. Vorteilhaft kann die Verrechnung bereits rekonstruierter Bilddatensätze eine robuste und zeiteffiziente Bereitstellung des finalen Bilddatensatzes ermöglichen, welcher eine verbesserte Bildqualität aufweist.

Erfindungsgemäß weist jedes Pixelelement der Vielzahl an Pixelelementen eine Anzahl an Komparatoren mit jeweils einer einstellbaren Energieschwelle auf, wobei in jedem Pixelelement der Vielzahl an Pixelelementen basierend auf einem Ausgangssignal zumindest eines Komparators der Anzahl an Komparatoren eines jeweiligen Pixelelements die zumindest eine Anzahl an Zählsignalen gezählt wird. Jedes Pixelelement der Vielzahl an Pixelelementen weist zumindest einen Komparator mit einer einstellbaren Energieschwelle auf. Jedes Pixelelement der Vielzahl kann mehrere mit Komparatoren mit jeweils einer einstellbaren Energieschwelle aufweisen. Das Ausgangssignal eines jeweiligen Komparators der Anzahl an Komparatoren kann dann dem Zählsignal entsprechen, welches mittels eines mit dem jeweiligen Komparator signaltechnisch gekoppelten Zählers, auch Zählelement genannt, gezählt wird. Das Zählsignal kann jedoch auch auf einem weiterverarbeiteten oder angepassten Ausgangssignals basieren. Beispielsweise kann ein Element zur Verhinderung einer Paralyse bei hohen Röntgenflüssen zwischen dem Signalausgang des Komparators und dem Zählelement zwischengeschaltet sein, welches bei einer dauerhafter Überschreitung der Komparatorschwelle weitere Zählereignisse induziert, welche mittels des Zählelements gezählt werden können. Die Anzahl an Zählsignalen kann außerdem mittels des Zählelements zumindest vorläufig bis zu einer Auslese des Zählerstandes gespeichert werden.

Außerdem wird erfindungsgemäß in einem jeweiligen Pixelelement der Teilzahl der Vielzahl an Pixelelementen basierend auf dem Ausgangssignal zumindest eines Komparators der Anzahl an Komparatoren des jeweiligen Pixelelements der Teilzahl der Vielzahl an Pixelelementen und zumindest auf dem Ausgangssignal eines Komparators der Anzahl an Komparatoren des zumindest einen weiteren Pixelelements der Vielzahl an Pixelelementen die zumindest eine Anzahl an Koinzidenz-Zählsignalen gezählt. Der zumindest eine Komparator der Anzahl an Komparatoren des jeweiligen Pixelelements der Teilzahl der Vielzahl an Pixelelementen, auf welchem das Koinzidenz-Zählsignal basiert, kann demjenigen Komparator der Anzahl an Komparatoren entsprechen, auf welchem das Zählsignal in dem Pixelelement der Teilzahl basiert. Das Koinzidenz-Zählsignal und das Zählsignal, dessen Anzahlen im jeweiligen Pixelelement der Teilzahl der Vielzahl an Pixelelementen gezählt werden, können jedoch auch auf Ausgangssignalen von unterschiedlichen Komparatoren des jeweiligen Pixelelements basieren.

Für die Bildung der Koinzidenz-Zählsignale kann der Komparator der Anzahl an Komparatoren des jeweiligen Pixelelements der Teilzahl an Pixelelementen und der Komparator der Anzahl an Komparatoren des zumindest einen weiteren Pixelelements der Vielzahl an Pixelelementen, auf welchem das Koinzidenz-Zählsignal basiert, dazu mit einer Koinzidenzlogik signaltechnisch verknüpft sein. Die Koinzidenzlogik kann dazu ausgebildet sein, bei Auftreten von zumindest zwei koinzidierend auftretenden Signalen ein Ausgangsignal bereitzustellen, welches mittels eines mit der Koinzidenzlogik signaltechnisch gekoppelten Zählers als Koinzidenz-Zählsignal gezählt werden kann.

Das erfindungsgemäße Zählen der Zählsignale und das Zählen der Koinzidenz-Zählsignale basierend auf den Ausgangssignalen von zumindest einem Komparator eines jeweiligen Pixelelement stellt eine vorteilhaft zweckmäßige Möglichkeit der Bereitstellung der für die Erzeugung eines verbesserten Röntgenbilddatensatzes notwendigen Koinzidenz- bzw. Zählinformationen dar, welche unnötige Totzeiten des Röntgendetektors vermeidet.

Vorzugsweise ist der Röntgendetektor außerdem für energieaufgelöste Messungen ausgebildet und weist mehr als einen, beispielsweise zwei, drei, vier oder mehr, Komparatoren mit jeweils einer einstellbaren Energieschwelle auf, wobei basierend auf den Ausgangssignalen von mehr als einem Komparator jeweils eine Anzahl an Zählsignalen gezählt werden kann. In dieser Variante weist jedes Pixelelement der Vielzahl an Pixelelementen eine Mehrzahl an Komparatoren auf, wobei basierend auf den jeweiligen Ausgangssignalen von mehr als einem Komparator jeweils eine Anzahl an Zählsignalen basierend auf den dazugehörigen Ausgangssignalen gezählt werden kann. Basierend auf den mehreren Anzahlen an Zählsignalen kann vorteilhaft ein energieaufgelöster Röntgenbilddatensatz erzeugt werden.

Es können ebenso auch mehrere Anzahlen an Koinzidenz-Zählsignalen basierend auf den Ausgangssignalen von mehreren Komparatoren der Anzahl an Komparatoren des jeweiligen Pixelelements der Teilzahl der Vielzahl an Pixelelementen und zumindest auf dem Ausgangssignal eines Komparators der Anzahl an Komparatoren des zumindest einen weiteren Pixelelements der Vielzahl an Komparatoren gezählt werden. Das heißt, jedes Pixelelement der Teilzahl der Vielzahl an Pixelelementen kann eine Mehrzahl an Komparatoren aufweisen, wobei basierend auf den jeweiligen Ausgangssignalen von mehr als einem Komparator jeweils zumindest eine Anzahl an Koinzidenz-Zählsignalen mit zumindest einem weiteren Pixelelement der Vielzahl an Pixelelementen gezählt wird.

Vorteilhaft kann eine detailliertere Koinzidenzinformation in Abhängigkeit mehrerer Energieschwellen zu einer verbesserten Korrektur sowie zu erweiterten Korrekturmöglichkeiten führen, welche durch die Bereitstellung lediglich einer einzelnen Anzahl an Koinzidenz-Zählsignalen nicht zugänglich wäre. So können bei Bereitstellung mehrere Energieschwellen, die mehreren darauf basierenden Anzahlen an Zählsignalen verbessert mittels mehrerer Anzahlen an Koinzidenz-Zählsignalen in Abhängigkeit der gleichen Energieschwellen korrigiert werden. Außerdem kann die Auswertung detaillierterer Koinzidenzinformationen in Form von mehreren Anzahlen an Koinzidenz-Zählsignalen in Abhängigkeit mehrerer Energieschwellen verbessert Hinweise auf eine Ortsinformation eintreffender Röntgenphotonen oder eine verbesserte spektrale Information der eintreffenden Röntgenphotonen und damit erweiterte Korrekturmöglichkeiten eröffnen.

Gemäß einer weiteren Ausführungsform des erfindungsgemäßen Verfahrens weist für das zweite Zählen der zumindest einen Anzahl an Koinzidenz-Zählsignalen die einstellbare Energieschwelle des zumindest einen Komparators der Anzahl an Komparatoren des jeweiligen Pixelelements der Teilzahl der Vielzahl an Pixelelementen und die einstellbare Energieschwelle des zumindest einen Komparators der Anzahl an Komparatoren des zumindest einen weiteren Pixelelements der Vielzahl an Pixelelementen, auf welchen die Koinzidenz-Zählsignale basieren, einen gleichen Energieschwellwert auf. Das bedeutet im Wesentlichen, dass Koinzidenz-Zählsignale gezählt werden, welche auf koinzidierend auftretenden Signalen basieren, welche sowohl in dem jeweiligen betrachteten Pixelelement der Teilzahl der Vielzahl an Pixelelementen als auch in dem zumindest einen weiteren Pixelelement der Vielzahl an Pixelelement jeweils eine Energieschwelle aufweisend denselben Energieschwellwert überschritten haben. Dies kann auch als (energie-) symmetrische Überschreitung an Energieschwellen oder (energie-) symmetrische Koinzidenz im betrachteten und dem zumindest einen weiteren Pixelelement bezeichnet werden.

Vorteilhaft erlaubt der Zugang zu (energie-)symmetrischen Überschreitungen an Energieschwellen von koinzidierend auftretenden Signalen mit einem überschaubaren schaltungstechnischen Aufwand Korrekturen, welche das Erreichen einer deutlich verbesserten Bildqualität eines Röntgenbilddatensatzes, beispielsweise ein verbessertes Rauschen, ermöglichen. Beispielsweise erlaubt die Koinzidenzinformation über symmetrische Überschreitungen an Energieschwellen in einfacher Weise eine Anpassung der zumindest einen gezählten Anzahl an Zählsignalen in den Pixelelementen, so dass Doppelzählungen von Photonenereignissen zumindest zu einem großen Teil korrigiert werden können.

Bei einer Bereitstellung von mehreren Komparatoren mit jeweils einer einstellbaren Energieschwelle kann bevorzugt zumindest eine symmetrische Koinzidenz hinsichtlich der niedrigsten Energieschwelle berücksichtigt werden. Es kann zumindest eine Anzahl an Koinzidenz-Zählsignalen in Abhängigkeit des auf den niedrigsten Energieschwellwert eingestellten zumindest einen Komparators der Anzahl an Komparatoren des jeweiligen Pixelelements der Teilzahl der Vielzahl an Pixelelementen und des auf den niedrigsten Energieschwellwert eingestellten zumindest einen Komparators der Anzahl an Komparatoren des zumindest einen weiteren Pixelelements gezählt werden. Die Koinzidenzinformation hinsichtlich der symmetrischen Überschreitung der niedrigsten Energieschwelle kann vorteilhaft die Anzahl aller auftretenden Koinzidenzen zwischen den beteiligten Pixelelementen widerspiegeln. Jedoch stehen darauf basierend nur Verbesserungsmöglichkeiten zur Verfügung und es kann derart kein Hinweis auf eine verbesserte Zuordnung der Koinzidenzen zu einem spezifischen Pixelelement abgeleitet werden.

Die Information über symmetrische Überschreitungen der Energieschwellen kann außerdem vorteilhaft genutzt werden, um die spektrale Information der Daten zu verbessern. Hier liegt die Überlegung zugrunde, dass die Wahrscheinlichkeit von Koinzidenzen für Photonen höherer Energie größer ist als für niederenergetischere Photonen. Daraus folgt, dass diese Photonen vermehrt in den unteren Energieschwellen mehrfach gezählt, und damit höher gewichtet werden. Durch Eingehen der Koinzidenzinformation in das Erzeugen kann dies durch eine entsprechende Korrektur vorteilhaft reduziert werden. Beispielsweise kann die Korrektur eine Umverteilung der gezählten Anzahlen basierend auf zumindest einem Teil der gezählten symmetrischen Koinzidenzen, d.h. zumindest einem Anteil der gezählten Anzahl an Koinzidenz-Zählsignalen basierend auf einer symmetrischen Überschreitung, hin zu einer höherenergetischeren Energieschwelle umfassen.

Erfindungsgemäß ist vorgesehen, dass für das zweite Zählen der zumindest einen Anzahl an Koinzidenz-Zählsignalen die einstellbare Energieschwelle des zumindest einen Komparators der Anzahl an Komparatoren des jeweiligen Pixelelements der Teilzahl der Vielzahl an Pixelelementen und die einstellbare Energieschwelle des zumindest einen Komparators der Anzahl an Komparatoren des zumindest einen weiteren Pixelelements der Vielzahl an Pixelelementen, auf welchen die Koinzidenz-Zählsignale basieren, unterschiedliche Energieschwellwerte aufweisen. Das bedeutet im Wesentlichen, dass Koinzidenz-Zählsignale gezählt werden, welche auf Signalen basieren, welche in dem jeweiligen Pixelelement der Teilzahl an Pixelelementen eine Energieschwelle mit einem anderen Energieschwellwert, beispielsweise einen höher- oder einen niederenergetischeren, überschritten haben, als in dem zumindest einen weiteren Pixelelement der Vielzahl an Pixelelementen. Beispielsweise kann ein Koinzidenz-Zählsignal basierend auf einem Komparator des jeweiligen Pixelelements der Teilzahl an Pixelelementen aufweisend eine Energieschwelle mit einem ersten Energieschwellwert und einem Komparator des weiteren Pixelelements der Vielzahl an Pixelelementen aufweisend einen zweiten Energieschwellwert gebildet werden. Dies kann auch als (energie-)unsymmetrische Überschreitung an Energieschwellen, oder auch (energie-)unsymmetrische Koinzidenz, im betrachteten und zumindest einem weiteren Pixelelement, bezeichnet werden.

Vorteilhaft erlaubt der Zugang zu (energie-)unsymmetrischen Überschreitungen an Energieschwellen von koinzidierend auftretenden Signalen verbessert Hinweise auf eine Ortsinformation eintreffender Röntgenstrahlung. Das heißt, es kann daraus verbessert ein Hinweis auf dasjenige Pixelelement abgeleitet werden, in welchem ein Großteil der Energie eines Röntgenphotons deponiert wurde. Vorteilhaft können darauf basierend weitergehende Verbesserungen der Bildqualität ermöglicht werden, welche die Möglichkeit der besseren Lokalisation einbeziehen. Vorteilhaft können die Anzahlen an Koinzidenz-Zählsignalen, welche auf unterschiedlichen Energieschwellen basieren, besonders vorteilhaft eingesetzt werden, um ein verbesserte Ortsauflösung zu erreichen.

In einer vorteilhaften Ausgestaltung der Erfindung ist außerdem vorgesehen, dass in jedem Pixelelement der Teilzahl an Pixelelementen sowohl zumindest eine Anzahl an Koinzidenz-Zählsignalen gezählt wird, welche auf dem gleichen Energieschwellwert basiert, und zumindest eine Anzahl an Koinzidenz-Zählsignal gezählt wird, welche auf unterschiedlichen Energieschwellwerten basiert.

Diese Ausführungsvariante liefert vorteilhaft eine besonders detaillierte Koinzidenzinformation, wobei ein kombiniertes Eingehen der Koinzidenzinformation hinsichtlich eines (energie- )symmetrischen oder (energie-)unsymmetrischen Überschreitens von Energieschwellen in das Erzeugen besonders vorteilhaft für die Verbesserung der Bildqualität eingesetzt werden kann.

In bevorzugten Ausführungsvarianten werden Koinzidenz-Zählsignale in jedem Pixelelement der Teilzahl an Pixelelementen mit zwischen einem und 24 weiteren Pixelelementen der Vielzahl an Pixelelementen gebildet. Bevorzugt umfasst das zumindest eine weitere Pixelelement ein in dem durch die Anordnung der Vielzahl an Pixelelemente aufgespannten, üblicherweise orthogonalen, matrixartigen Pixelraster zumindest ein direkt benachbartes Pixelelement des jeweiligen Pixelelements der Teilzahl an Pixelelementen oder ein diagonal benachbartes Pixelelement der Vielzahl an Pixelelementen.

Vorteilhaft werden zumindest mit denjenigen weiteren Pixelelementen Koinzidenz-Zählsignale gebildet, in welchen mit hoher Wahrscheinlichkeit koinzidierende Signale auftreten. Dies kann zumindest die vier direkt benachbarten Pixelelementen oder die vier direkt benachbarten Pixelelementen gemeinsam mit den diagonal benachbarten Pixelelementen umfassen. Beispielsweise werden Koinzidenz-Zählsignale in jedem Pixelelement der Teilzahl der Vielzahl an Pixelelementen zumindest mit den vier direkt benachbarten weiteren Pixelelementen der Vielzahl an Pixelelementen gebildet. Es kann jedoch auch eine andere Auswahl und/oder Anzahl an weiteren Pixelelementen vorgesehen sein. Es können beispielsweise übernächste Nachbarn, d.h. benachbarte Pixelelemente der direkt benachbarten Pixelelemente des betrachteten Pixelelements, hinsichtlich koinzidierend auftretender Signale betrachtet werden. Die Betrachtung und Einbeziehung von übernächsten Nachbarn, kann insbesondere dann vorteilhaft sein, wenn vorgesehen ist, Signale von mehreren Pixelelementen für eine Erzeugung des Röntgenbilddatensatzes zusammenzufassen, oder bei geringen Pixelgrößen, bei welchen koinzidierend auftretende Signale auch über die durch die Pixelelemente vorgegebenen Abstände hinweg in größerem Ausmaß zu erwarten sind.

Die Anzahl und Auswahl an weiteren Pixelelementen, auf welchen das Koinzidenz-Zählsignal basiert, kann innerhalb der Teilzahl der Vielzahl an Pixelelementen variieren. Jedoch geht mit einer höheren Anzahl an weiteren Pixelelementen eine komplexere Verschaltung der Pixelelemente untereinander einher.

Gemäß einer weiteren Ausführungsform des erfindungsgemäßen Verfahrens werden Koinzidenz-Zählsignale in jedem Pixelelement der Teilzahl an Pixelelementen mit zwischen einem und 24 weiteren Pixelelementen der Vielzahl an Pixelelementen gebildet, wobei die im Schritt des zweiten Zählens für jedes Pixelelement der Teilzahl an Pixelelementen gezählte zumindest eine Anzahl an Koinzidenz-Zählsignalen auf koinzidierend auftretenden Signalen aller weiteren Pixelelemente basiert.

In dieser Ausführungsform kann jede in einem Pixelelement der Teilzahl an Pixelelementen gezählte Anzahl an Koinzidenz-Zählsignalen jeweils der Summe der in den ein bis 24 weiteren Pixelelementen gebildeten Koinzidenz-Zählsignale entsprechen. Das heißt die jeweilige Anzahl an Koinzidenz-Zählsignalen wird um eine Zähleinheit erhöht, wenn zumindest in einem der ein bis 24 weiteren Pixelelementen ein koinzidierendes Signal auftritt. Dahinter steht die Erkenntnis, dass die Anzahl der Koinzidenzen eines Pixelelements mit beliebigen Nachbarn bereits einen Großteil der Information tragen kann, welche vorteilhaft für Korrekturen eingesetzt werden kann. Gleichzeitig kann dadurch eine einfache signaltechnische Verschaltung gewährleistet werden.

Alternativ kann in einer weiteren Ausführungsform des erfindungsgemäßen Verfahrens vorgesehen sein, dass Koinzidenz-Zählsignale in jedem Pixelelement der Teilzahl der Vielzahl an Pixelelementen mit zwischen einem und 24 weiteren Pixelelementen der Vielzahl an Pixelelementen gebildet werden, wobei im Schritt des zweiten Zählens jeweils zumindest eine Anzahl an Koinzidenz-Zählsignalen mit jedem der weiteren Pixelelementen gezählt wird.

In dieser Ausführungsform wird in dem Pixelelement der Teilzahl der Vielzahl an Pixelelementen für jedes der weiteren Pixelelemente einzeln zumindest eine Anzahl an Koinzidenz-Zählsignalen gezählt. Vorteilhaft steht eine möglichst detaillierte, pixelweise Koinzidenzinformation zur Verfügung, die eine Richtungsinformation der auftretenden Koinzidenzen relativ zu dem betrachteten Pixelelement der Teilzahl an Pixelelementen trägt. Diese detaillierte, pixelweise Koinzidenzinformation kann besonders vorteilhaft für eine verbesserte Ortsinformation, d.h. verbesserte Lokalisation des Ursprungsorts der auftretenden gezählten Signale, eingesetzt werden. Jedoch geht damit ein erhöhter signaltechnischer Aufwand und eine erhöhte, ggf. von einem Röntgendetektor zu übertragende Datenmenge einher.

In einer weiteren Ausgestaltungsvariante des Verfahrens umfasst das Verfahren außerdem den Schritt

- Übertragen zumindest einer in einem Pixelelement der Teilzahl der Vielzahl an Pixelelementen gezählten Anzahl an Koinzidenz-Zählsignalen auf zumindest eines der weiteren Pixelelemente, mit welchem in dem jeweiligen Pixelelement der Teilzahl ein Koinzidenz-Zählsignal gebildet wird,
- wobei der Schritt des Erzeugens außerdem auf der zumindest einer übertragenen Anzahl an Koinzidenz-Zählsignalen basiert.

Dieser Variante liegt insbesondere die Überlegung zugrunde, dass die Koinzidenzinformation zwischen beteiligten Pixelelementen als (pixel-)symmetrisch gelten kann. Wird in einem ersten Pixelelement der Teilzahl der Vielzahl eine Anzahl an Koinzidenz-Zählsignalen eines Pixelelements mit einem zweiten Pixelelement der Vielzahl gezählt, kann die gezählte Anzahl ebenso auf das zweite Pixelelement übertragen werden, und in dem zweiten Pixelelement für eine korrespondierende Anzahl an Koinzidenz-Zählsignalen des zweiten Pixelelements mit dem ersten Pixelelement eingesetzt werden.

Diese Variante erlaubt für Pixelelemente der Vielzahl an Pixelelementen, welche nicht Teil der Teilzahl der Vielzahl an Pixelelementen sind, Anzahlen an Koinzidenz-Zählsignalen zu ermitteln und Koinzidenzinformation zu sammeln, welche in dem Verfahren zur Erzeugung eines Röntgenbilddatensatz und seiner Varianten in analoger Weise wie eine gezählte Anzahl an Koinzidenz-Zählsignalen eingesetzt werden kann. Beispielsweise kann in einer matrixartigen Anordnung der Vielzahl an Pixelelemente nur jeweils jedes zweite Pixelelement der Vielzahl Teil der Teilzahl und damit ausgebildet sein, zumindest eine Anzahl an Koinzidenz-Zählsignalen zu zählen. Für den Rest der Vielzahl an Pixelelementen kann dann eine Übertragung der Koinzidenzinformation stattfinden.

Diese Variante erlaubt ebenso, ein redundantes Zählen an Koinzidenz-Zählsignalen in den Pixelelementen der Teilzahl der Vielzahl an Pixelelementen zu reduzieren oder die gesammelte Koinzidenzinformation in einem Pixelelement der Teilzahl der Vielzahl zu erweitern. Die in einem Pixelelement der Teilzahl der Vielzahl an Pixelelementen ermittelte Koinzidenzinformation kann mittels einer Übertragung erweitert oder vervollständigt werden, ohne dass diese Anzahl in dem Pixelelement selbst gezählt werden muss. Beispielsweise kann jedes Pixelelement der Vielzahl zumindest jeweils eine Anzahl an Koinzidenz-Zählsignalen mit seinem nördlich benachbarten Pixel zählen. Die Koinzidenzinformation eines jeden Pixelelements hinsichtlich seines südlichen Nachbarn liegt dann korrespondierend entsprechend jeweils im südlichen benachbarten Pixelelement vor und kann für die Anwendung des Verfahrens zur Erzeugung auf das jeweilige Pixelelement übertragen werden. Derart kann in jedem Pixelelement der Teilzahl selbst jeweils nur ein Teil der für ein Verfahren zur Erzeugung eines Röntgenbilddatensatz gewünschten Koinzidenzinformation gezählt werden, wobei durch Übertragung eine Vervollständigung ermöglicht werden kann. Vorteilhaft kann dadurch der Aufwand in der signaltechnischen Verschaltung der Pixelelemente vereinfacht werden.

Eine auf ein Pixelelement der Vielzahl an Pixelelementen übertragene Anzahl kann in dem Verfahren zur Erzeugung eines Röntgenbilddatensatzes und seiner Varianten in analoger Weise eingesetzt werden, wie eine gezählte Anzahl. Wird im Folgenden also von einer Anzahl an Koinzidenz-Zählsignalen oder von Koinzidenzinformation gesprochen, so kann dies eine gezählte und/oder eine übertragene Anzahl an Koinzidenz-Zählsignalen umfassen, außer es wird im Text explizit unterschieden. Eine übertragene Anzahl an Koinzidenzinformation kann im Schritt des Erzeugens in die Datenvorverarbeitung vor einer Bildrekonstruktion, in die Bildrekonstruktion oder in einen der Bildrekonstruktion nachgelagerten Nachverarbeitungsschritt eingehen. Beispielsweise kann eine auf ein Pixelelement übertragene Anzahl an Koinzidenz-Zählsignalen eingesetzt werden, um eine gezählte Anzahl an Zählsignalen in diesem Pixelelement anzupassen. Beispielsweise kann eine auf ein Pixelelement übertragene zumindest eine Anzahl an Koinzidenz-Zählsignalen von einer in dem Pixelelement gezählten Anzahl an Zählsignalen subtrahiert oder zu der zumindest einen Anzahl an Zählsignalen addiert werden. Beispielsweise kann ein Koinzidenz-Bilddatensatz auch auf einer übertragenen Anzahl an Koinzidenz-Zählsignalen basieren. Beispielsweise kann auch eine übertragene Anzahl an Koinzidenz-Zählsignalen als Eingabeparameter in eine trainierte Funktion eingehen. Beispielsweise kann eine übertragene Anzahl an Koinzidenz-Zählsignalen in die Bildrekonstruktion eingehen.

Bevorzugt liegt einer übertragenen Anzahl eine pixelweise Koinzidenzinformation zugrunde, wobei die zu übertragende Anzahl auf Koinzidenz-Zählsignalen basiert, welche lediglich zwischen dem Pixelelement, von welchem die Anzahl übertragen wird, und dem Pixelelement, auf welches die Anzahl übertragen wird, gebildet werden. Das heißt, in dieser Variante wird im Schritt des zweiten Zählens jeweils zumindest eine Anzahl an Koinzidenz-Zählsignalen mit jedem der weiteren Pixelelemente gezählt, sofern mehrere weitere Pixelelemente für die Bildung von Koinzidenz-Zählsignalen vorgesehen sind. Derart ist eine eindeutigere Zuordnung möglich und eine direkte Korrespondenz der Anzahlen verbessert gewährleistbar.

Es kann jedoch Varianten des Verfahrens geben, wobei, sofern mehrere, weitere Pixelelemente für die Bildung von Koinzidenz-Zählsignalen vorgesehen sind, eine Anzahl an Koinzidenz-Zählsignalen übertragen wird, welche auf koinzidierend auftretenden Signalen aller weiteren Pixelelemente basiert. Das heißt die übertragene Anzahl an Koinzidenz-Zählsignalen kann der Summe der mit den weiteren Pixelelementen gebildeten Koinzidenz-Zählsignale entsprechen. Hier kann die Annahme zugrunde liegen, dass zumindest für Pixelelemente, welche in nächster Nähe zu dem Pixelelement angeordnet sind, von welchem eine Anzahl übertragen wird, und damit ähnlichen Bedingungen ausgesetzt sind, auch basierend auf einer Summe zumindest eine Abschätzung einer Anzahl an Koinzidenz-Zählsignalen möglich sein kann, welche in einem Verfahren zur Erzeugung eines Röntgenbilddatensatzes angewendet werden kann. In einer Weiterbildung dieser Variante kann beispielsweise vorsehen, dass im Schritt des Übertragens eine trainierte Funktion angewendet wird, welche eine optimierte Abschätzung der übertragenen Anzahlen basierend auf Summen-Anzahlen, beispielsweise unter Berücksichtigung auch der gezählten Anzahlen an Zählsignalen und der relativen Anordnung der Pixelelemente zueinander, ermöglich. Eine solche trainierte Funktion kann beispielweise trainiert sein mittels Trainingsdaten, umfassend eine pixelweise Koinzidenzinformation und eine summierte Koinzidenzinformation, so dass nach einem Training mittels der trainierten Funktion die pixelweise Koinzidenzinformation abgeschätzt werden kann.

In einer Variante des Verfahrens wird für jedes Pixelelement der Vielzahl an Pixelelementen zumindest eine gezählte oder eine übertragene Anzahl an Koinzidenz-Zählsignalen ermittelt. Vorteilhaft kann in jedem Pixelelement der Vielzahl an Pixelelementen ermittelte Koinzidenzinformation zur Verfügung gestellt werden, so dass optimale Bedingungen für das Erzeugen des Röntgenbilddatensatz bereitgestellt werden können.

In einer weiteren Ausführungsform des erfindungsgemäßen Verfahrens ist durch die Anordnung der Vielzahl an Pixelelemente ein erstes matrixartiges Pixelraster definiert. Im Schritt des Erzeugens wird basierend auf dem Pixelraster ein Subpixelraster an mit den Pixelelementen der Vielzahl an Pixelelementen überlappenden, Subpixeln definiert, wobei das Subpixelraster zumindest entlang einer Rasterdimension einen relativ zum Pixelraster reduzierten Rasterabstand aufweist.

Im allgemeinen Fall kann das ursprüngliche Pixelraster $A_1 \, x \, A_2$ auf ein neues Subpixelraster $B_1 \, x \, B_2$ mit $B_1 \geq A_1$ und $B_2 \geq A_2$ übertragen werden, wobei für zumindest eine der Beziehungen $B > A$ gilt. $A_1$ und $A_2$ bzw. $B_1$ und $B_2$ beschreiben dabei jeweils die Anzahl der Pixelelemente im Pixelraster bzw. die Anzahl der Subpixel im Subpixelraster entlang der zwei Raumdimensionen des jeweiligen Rasters. Das heißt, es wird zumindest eine Dimension des Subpixelrasters feiner dargestellt als das Pixelraster. Der Rasterabstand kann jedoch entlang der Rasterdimensionen variieren.

Weiterhin wird jedem Subpixel zumindest eine virtuelle Anzahl an Zählsignalen zugeordnet, wobei die jeweilige zumindest eine virtuelle Anzahl zumindest eines Teils der Subpixel auf zumindest einer gezählten oder übertragenen Anzahl an Koinzidenz-Zählsignalen eines mit dem jeweiligen Subpixel überlappenden Pixelelements basiert. Basierend auf der den virtuellen Subpixeln jeweils zugeordneten virtuellen Anzahl an Zählsignalen wird dann der Röntgenbilddatensatz erzeugt.

Diese Variante geht dabei erfindungsgemäß davon aus, dass die zumindest eine Anzahl an Koinzidenz-Zählsignalen in den Pixelelementen der Teilzahl der Vielzahl an Pixelelementen mit jedem der verknüpften weiteren Pixelelemente einzeln gezählt oder ermittelt wird. Das heißt, es kann für die Zuordnung der virtuellen Anzahl eine (gezählte oder übertragene) pixelweise Koinzidenzinformation zur Verfügung gestellt werden. Eine pixelweise Koinzidenzinformation beinhaltete eine Richtungsinformation über die Koinzidenzen und damit die Möglichkeiten einer verbesserten Lokalisation der auftretenden Koinzidenzen.

Vorzugsweise wird dabei die zumindest eine Anzahl an Koinzidenz-Zählsignalen außerdem mit zumindest einem der direkt benachbarten Pixelelemente gezählt.

Die zumindest eine virtuelle Anzahl an Zählsignalen kann dabei für zumindest einen Teil der Subpixel lediglich auf zumindest einer gezählten oder übertragenen Anzahl an Koinzidenz-Zählsignalen eines mit dem jeweiligen Subpixel überlappenden Pixelelements basieren. Die zumindest eine virtuelle Anzahl an Zählsignalen kann auch auf zumindest einer Anzahl an Koinzidenz-Zählsignalen und auf zumindest einer Anzahl an Zählsignalen eines mit dem Subpixel überlappenden Pixelelements basieren. Eine zugeordnete virtuelle Anzahl kann auch lediglich auf zumindest einer Anzahl an Zählsignalen eines überlappenden Pixelelements basieren.

Vorteilhaft kann die Richtungsinformation der ermittelten Koinzidenzinformation zu einer verbesserten Übertragung der Anzahlen auf die virtuellen Subpixel eingesetzt werden und damit eine verbesserte Ortsauflösung ermöglichen.

Vorzugsweise ist dabei vorgesehen, dass die einem jeweiligen Subpixel zugeordnete virtuelle Anzahl an Zählsignalen abhängig ist von der relativen Position des virtuellen Subpixels zu einem mit dem Subpixel überlappenden Pixelelement. Dahinter steht die Annahme, dass die Koinzidenzinformationen mit einer Richtungsinformation zu einer verbesserten Lokalisation der Zählsignale auf einem feineren Subpixelraster eingesetzt werden kann. So wird angenommen, dass alle Photonen, welche ein Pixelelement im Pixelraster zentral treffen (und damit mit höherer Wahrscheinlichkeit keine koinzidierenden Signale in weiteren Pixelelementen erzeugen), einem entsprechend das betrachtete Pixelelement zentral überlappendem Subpixel zugeordnet werden können. Dagegen können Koinzidenzevents denjenigen Subpixeln zugeordnet werden, welche randnäher mit dem ursprünglichen Pixelelement überlappen oder zwischen, beispielsweise, zwei ursprünglichen Pixelelementen liegen.

In Verfahrensvarianten kann dazu vorgesehen sein, dass die einem Subpixel zugeordnete virtuelle Anzahl an Zählsignalen insbesondere abhängig ist vom relativen Abstand des virtuellen Subpixels zum Zentrum eines überlappenden Pixelelements. Dabei können beispielsweise Koinzidenz-Zählsignale, welche aus einer symmetrischen Überschreitung resultieren Subpixeln zugeordnet werden, welche einen größeren Abstand zum Zentrum des überlappenden Pixelelements aufweisen und solche Koinzidenz-Zählsignale, welche aus einer unsymmetrischen Überschreitung resultieren, solchen Subpixeln zugeordnet werden, welche einen relativ dazu geringeren Abstand zum Zentrum des überlappenden Pixelelements aufweisen. Vorteilhaft kann dadurch eine verbesserte Lokalisation der Zählsignale im Subpixelraster und damit eine verbesserte Ortsauflösung ermöglicht werden.

In Verfahrensvarianten kann dabei vorgesehen sein, dass die Fläche der virtuellen Subpixel im Subpixelraster gleich ist. Vorteilhaft ist eine zweckmäßige und einfache Implementierung bereitgestellt, welche eine zeiteffiziente Bereitstellung des Röntgenbilddatensatzes ermöglicht.

Weiterhin kann dabei vorgesehen sein, dass die Fläche der virtuellen Subpixel im Subpixelraster unterschiedlich ist. Vorteilhaft kann eine flexiblere Implementierung bereitgestellt werden, welche weitere Randbedingungen oder Annahmen für eine verfeinerte Lokalisation der gezählten Anzahlen ermöglicht.

Es kann bei der Wahl der Subpixelgrößen des Subpixelrasters beispielsweise vorgesehen sein, die Pixelgröße d.h. die Pixelfläche der Subpixel, in Abhängigkeit von zumindest einem physikalischen Parameter zu wählen. Der physikalische Parameter kann insbesondere in Verbindung mit der generierten Ladungsverteilung im Konverterelement durch die eintreffende Röntgenstrahlung stehen. Indem Annahmen über beispielsweise Durchmesser der generierten Ladungswolke oder Reichweite der Fluoreszenzen in die Wahl der Pixelfläche einbezogen werden, kann vorteilhaft ein qualitativ hochwertigere Bildqualität ermöglicht werden. Vorteilhaft kann dadurch ein möglichst konstantes Verhältnis von Statistik zu Pixelfläche im Subpixelraster erreicht werden, um einen gleichmäßigen und hochqualitativen Bildeindruck zu ermöglichen.

Die Erfindung betrifft außerdem ein Röntgendetektorsystem gemäß Anspruch 16.

Dabei kann die Teilzahl an Pixelelementen die gesamte Vielzahl an Pixelelementen umfassen. Die Vielzahl kann jedoch auch neben der Teilzahl an Pixelelementen anderweitig ausgebildete Pixelelemente umfassen.

Die Erfindung betrifft außerdem ein medizinisches Bildgebungsgerät umfassend ein erfindungsgemäßes Röntgendetektorsystem. Das medizinische Bildgebungsgerät kann beispielsweise ein CT-Gerät, ein C-Bogenröntgengerät oder ein Angiographie-Röntgengerät umfassen. Es sind daneben jedoch auch andere medizinische Bildgebungsgeräte möglich, welche ausgebildet sind einen zweidimensionalen oder auch dreidimensionalen Bilddatensatz eines Objekts oder Patienten basierend auf Röntgenstrahlung zu erzeugen.

Die Vorteile des Verfahrens und seiner Varianten können dabei direkt ebenso auf das Röntgendetektorsystem und das medizinische Bildgebungsgerät übertragen werden.

Im Rahmen der Erfindung können insbesondere Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehr-fach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das medizinische Bildgebungsgerät das medizinische Bildgebungsgerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:

Fig. 1 ein Röntgendetektorsystem aufweisend einen Röntgendetektor mit einer Vielzahl an Pixelelementen,

Fig. 2 einen schematischen Verfahrensablauf eines Verfahrens zur Erzeugung eines Röntgenbilddatensatz,

Fig. 3 und Fig. 4 jeweils eine schematische Veranschaulichung einer exemplarischen, signaltechnischen Verschaltung eines Pixelelements der Teilzahl der Vielzahl an Pixelelementen in unterschiedlichen Ausführungsformen,

Fig. 5 eine Veranschaulichung eines basierend auf dem Pixelraster der Vielzahl an Pixelelementen definiertes Subpixelrasters an mit den Pixelelementen überlappenden Subpixeln,

Fig. 6 eine Veranschaulichung der Auswirkung des Verfahrens zur Erzeugung eines Röntgenbilddatensatz auf eine Energiedeposition durch Röntgenstrahlung innerhalb eines durch die Pixelelemente vorgegebenen Pixelrasters bei Einführung eines Subpixelrasters,

Fig. 7 eine Veranschaulichung eines basierend auf dem Pixelraster der Pixelelemente definiertes Subpixelrasters in einer weiteren Ausführungsform,

Fig. 8 ein medizinisches Bildgebungsgerät.

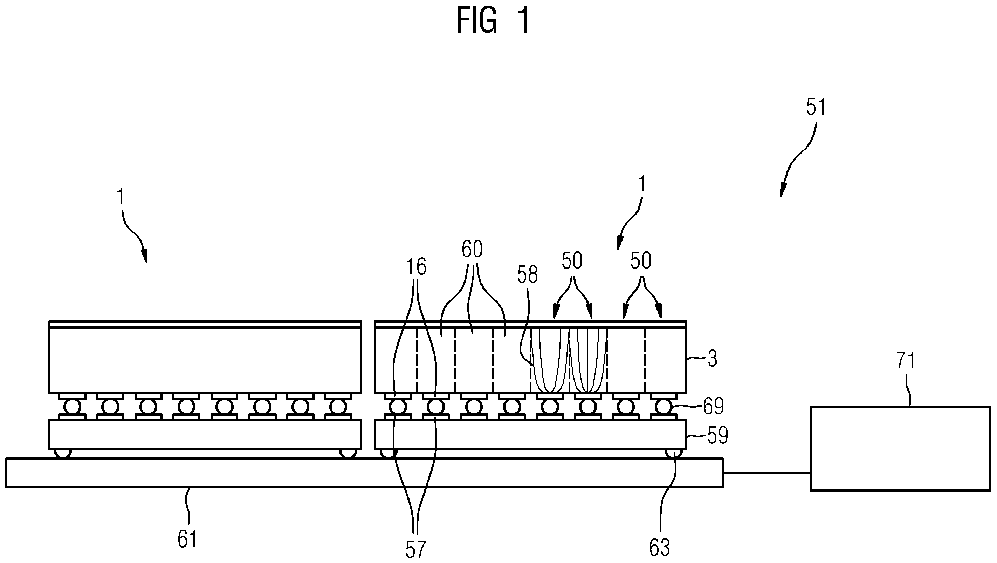

Die Fig. 1 zeigt schematisch ein exemplarisches Röntgendetektorsystem 51 in einer Seitenansicht, ausgebildet ein erfindungsgemäßes Verfahren auszuführen und aufweisend einen Röntgendetektor 1 mit einer Vielzahl an Pixelelementen 50.

Das in Fig. 1 exemplarisch gezeigte Röntgendetektorsystem 51 weist in der gezeigten Seitenansicht zwei sichtbare Röntgendetektoren 1 auf. Ein Röntgendetektorsystem 51 kann jedoch auch nur einen Röntgendetektor 1 oder auch mehr als zwei Röntgendetektoren 1 aufweisen. In einer vorteilhaften Ausführungsform weist das Röntgendetektorsystem 51 eine Anordnung einer Mehrzahl an Röntgendetektoren 1 in Form einer zweidimensionalen Matrix auf. Jeder der Röntgendetektoren 1 kann wiederum eine zweidimensionale, matrixartige Anordnung der einem Röntgendetektor 1 zugeordneten Vielzahl an Pixelelementen 50 aufweisen. Die Anzahl der Vielzahl an Pixelelemente 50 kann beispielsweise im Bereich von 100 bis mehrere Tausend liegen.

Der im Rahmen der Erfindung verwendete Röntgendetektor 1 kann auch als (photonen-)zählender oder direktkonvertierender Röntgendetektor 1 bezeichnet werden. Der gezeigte Röntgendetektor 1 weist ein Konverterelement 3 auf. Das Konverterelement 3 kann als flächenhafter Direktkonverter, beispielsweise aufweisend CdTe, CZT, CdZnTeSe, CdTeSe, CdMnTe, InP, TlBr2, HgI2, GaAs, Si, amorphes Selen (a-Se) oder andere als Konvertermaterial, ausgebildet sein. Auf der Oberseite weist das Konverterelement 3 eine erste Elektrode 18 (auch Top-Elektrode genannt) auf. Die Unterseite des Konverterelements 3 weist eine Mehrzahl an Sensor-Pixelelektroden 16 auf. Eine jeweilige Sensor-Pixelelektrode 16 ist in dem hier dargestellten, beispielhaften Röntgendetektor 1 über (elektrisch) leitende Verbindungen 69 und Auswerte-Pixelelektroden 57 jeweils mit einer pixelweisen Pixelelektronik der Pixelelemente 50 in der Auswerteeinheit 59 verbunden. Die pixelweise Pixelelektronik ist für eine pixelweise Weiterverarbeitung der in Abhängigkeit von der eintreffenden Röntgenstrahlung eingegangenen Signale ausgebildet. Die Auswerteeinheit 59 kann beispielsweise in Form eines ASIC ("application specific integrated circuit") ausgebildet sein. Die leitenden Verbindungen 69 können beispielsweise als Lotkugeln (bump bonds) oder Lotmaterial in Verbindung mit Kupfersäulen (copper pillars) oder auch anderweitig ausgebildet sein. In anderen Ausgestaltungsvarianten kann die Verbindung auch anderweitig ausgebildet sein, beispielsweise in Form einer Leitklebeverbindung oder ähnlichem. Die gemeinsame Anzahl der Sensor-Pixelelektroden 16, die Anzahl der leitenden Verbindungen 69, die Anzahl der Auswerte-Pixelelektroden 57, die Anzahl der pixelweisen Pixelelektroniken in der Auswerteeinheit 59 und die Anzahl der Pixelelemente 50 eines Röntgendetektors 1 sind in der Regel gleich. Das elektrische Feld, angedeutet durch die Feldlinien 58, zwischen der Top-Elektrode 18 und einer Sensor-Pixelelektrode 16 bestimmt ein einem jeweiligen Pixelelement 50 des Röntgendetektors 1 zugeordnetes sensitives Detektionsvolumen 60 im Konverterelement 3, angedeutet durch die gestrichelten Linien im Konverterelement 3. Die Einheit aus einer Sensor-Pixelelektrode 16, einer leitenden Verbindung 69, einer Pixelelektrode 57, einer pixelweisen Pixelelektronik in der Auswerteeinheit 59 und gegebenenfalls einem zugeordneten Detektionsvolumen 60 kann dann als Pixelelement 50, auch Detektionselement genannt, bezeichnet werden.

Die Auswerteeinheit 59 ist in diesem Ausführungsbeispiel weiterhin über Verbindungen 63 und einer peripheren Elektronik 61 mit einer Erzeugungseinheit 71 verbunden. Die periphere Elektronik 61 kann beispielsweise zur Sammlung der gemessenen Daten der Mehrzahl an Auswerteeinheiten 59 und gegebenenfalls auch einer Vorverarbeitung der gemessenen Daten vor einer Übertragung an die Erzeugungseinheit 71 dienen.

Darüber hinaus kann der Röntgendetektor 1 oder das Röntgendetektorsystem 51 auch noch weitere, hier nicht gezeigte Komponenten umfassen.

Eintreffende Röntgenstrahlung wird im Konvertermaterial des Konverterelements 3 in Abhängigkeit der durch die eintreffende Röntgenstrahlung lokal deponierten Energie in Ladungsträger konvertiert, d.h. in ein elektrisches Signal umgewandelt, worauf basierend in der pixelweisen Pixelelektronik desjenigen Pixelelements 50, in dessen zugeordneten Detektionsvolumen 60 Ladungsträger erzeugt wurden, ein Signal, üblicherweise ein elektrischer Puls, beispielsweise ein Ladungspuls, erzeugt und weiterverarbeitet wird. Die Auswerteeinheit 59 stellt pixelweise Pixelelektroniken für die pixelweise Verarbeitung eines über die Sensor-Pixelelektroden 16 bzw. Pixelelektroden 57 eines jeweiligen Pixelelements 50 direkt eingegangenen Signals bereit.

In einem photonenzählenden Röntgendetektor 1 wird üblicherweise ein elektrischer Puls erzeugt, dessen Höhe und/oder Länge der deponierten Energie des eingetroffenen Röntgenquants im Konvertermaterial entspricht. Der elektrische Puls wird dann als ein Zählereignis in einem Pixelelement 50 registriert und in eine digitale Speichereinheiten eines Zählers 13, auch Zählelement 13 genannt, eingeordnet, d.h. als (Pixel-)Zählsignal gezählt, wenn der erzeugte elektrische Puls im Pixelelement 50 oberhalb eines definierten Schwellwerts, d.h. einer einstellbaren Energieschwelle S in der Pixelelektronik des Pixelelements 50, liegt. In anderen Worten überschreitet das generierte Signal in einem Pixelelement 50 die eingestellte Energieschwelle S wird der Zählerstand des damit verknüpften Zählelements 13 des Pixelelements 50 um eine Zähleinheit erhöht.

Die einstellbare Energieschwelle S ist dabei in der Regel über einen Komparator 19 auf einen Energieschwellwert einstellbar. Der Energieschwellwert einer Energieschwelle S kann prinzipiell auch fest analog vorgegeben sein, wird aber im Allgemeinen über z. B. einen DAC (Digital-Analog-Wandler = digital-to-analog-converter) angelegt und ist damit in einem gewissen Bereich variabel einstellbar. Die Energieschwelle S kann entweder pixelweise lokal (mittels des Komparators und des DAC), für Gruppen von Pixelelementen oder auch global im Röntgendetektor 1 für alle Pixelelemente 50 des Röntgendetektors 1 einstellbar sein. Im dem Falle, dass zwei, drei oder mehr einstellbare Energieschwellen S in einem Pixelelement 50 für energieaufgelöste Messungen bereitgestellt sind, wird das erzeugte elektrische Signal entsprechend der unterschiedlichen, vordefinierten Energieschwellen S in eine oder mehrere Zählelemente 13, welche jeweils mit einer Energieschwelle S verknüpft sind, eingeordnet, d.h. gezählt.

Durch Charge-Sharing oder Fluoreszenzen kann ein Aufteilen der deponierten Energie eines einzelnen Ereignisses, d.h. eines eintreffenden Röntgenphotons, auf zwei oder mehr Pixelelemente 50, bzw. deren Detektionsvolumen 60, auftreten. Das heißt, es können in Detektionsvolumina 60, welche mehr als einem Pixelelement 50 zugeordnet sind, Ladungsträger generiert werden. Entsprechend kann es auftreten, dass ein eintreffendes Photon in mehr als einem Pixelelement 50 koinzidierend Signale erzeugt und Mehrfachzählungen in mehreren Pixelelementen 50 auftreten. Solche Mehrfachzählungen können zu Störeffekten im Röntgenbilddatensatz, beispielsweise zu einem erhöhten Rauschen, zu einer verminderten Ortsauflösung und/oder zu einer verminderten Energieauflösung führen.

Die Vielzahl an Pixelelementen 50 des erfindungsgemäßen Röntgendetektors 1 ist nun jeweils ausgebildet ein Zählsignal basierend auf einem in einem Pixelelement 50 der Vielzahl an Pixelelementen 50 direkt eingegangenem Signal zu bilden und darauf basierend zumindest eine Anzahl T an Zählsignalen zu zählen. Zumindest eine Teilzahl der Vielzahl an Pixelelementen 50 des erfindungsgemäßen Röntgendetektors 1 ist außerdem jeweils dazu ausgebildet, ein Koinzidenz-Zählsignal zu bilden und darauf basierend zumindest eine Anzahl C an Koinzidenz-Zählsignalen zu zählen. Dabei basiert ein in einem jeweiligen Pixelelement 50 der Teilzahl der Vielzahl an Pixelelementen 50 gebildetes Koinzidenz-Zählsignal auf einem in dem jeweiligen Pixelelement 50 der Teilzahl an Pixelelementen 50 direkt eingegangenem Signal und auf einem koinzidierend auftretenden Signal mindestens eines weiteren Pixelelements 50 der Vielzahl an Pixelelementen 50. Das zumindest eine weitere Pixelelement 50 der Vielzahl an Pixelelementen 50, auf welchem das Koinzidenz-Zählsignal basiert, kann, muss aber nicht notwendigerweise, von der zumindest einen Teilzahl der Vielzahl an Pixelelementen 50 umfasst sein.

Die zumindest Teilzahl der Vielzahl an Pixelelementen 50 kann außerdem die gesamte Vielzahl an Pixelelementen 50 umfassen. Das heißt, der Röntgendetektor 1 kann eine Vielzahl an Pixelelementen 50 aufweisen, wobei jedes Pixelelement 50 der Vielzahl ausgebildet sein kann, ein Zählsignal und ein Koinzidenz-Zählsignal zu bilden. Die Vielzahl an Pixelelementen 50 kann jedoch auch neben der Teilzahl der Vielzahl an Pixelelementen 50 anderweitig ausgebildete Pixelelemente 50 umfassen. Diese können beispielsweise lediglich ausgebildet sein Zählsignale zu bilden und zu zählen.

Im dem Falle, dass zwei, drei oder mehr einstellbare Energieschwellen S in einem jeweiligen Pixelelement 50 der Vielzahl an Pixelelementen 50 für energieaufgelöste Messungen bereitgestellt sind, kann ein jeweiliges Pixelelement 50 der Vielzahl an Pixelelementen 50 ausgebildet sein, entsprechend auch mehrere Anzahlen T an Zählsignalen in Abhängigkeit der bereitgestellten eingestellten Energieschwellen S zu zählen. Ebenso kann der erfindungsgemäße Röntgendetektor 1 bzw. ein jeweiliges Pixelelement 50 der Teilzahl der Vielzahl an Pixelelementen 50 ausgebildet sein, mehrere Anzahlen C an Koinzidenz-Zählsignalen, beispielsweise in Abhängigkeit der beteiligen Energieschwellen S und/oder in Abhängigkeit des oder der beteiligten weiteren Pixelelemente 50, zu zählen.

Die Erzeugungseinheit 71 ist dazu ausgebildet basierend auf der in der Vielzahl an Pixelelementen 50 jeweils gezählten zumindest einen Anzahl T an Zählsignalen und der in jedem Pixelelement 50 der Vielzahl der Teilzahl an Pixelelementen 50 zumindest einen Anzahl C an Koinzidenz-Zählsignalen einen Röntgenbilddatensatz zu erzeugen.

Die Erzeugungseinheit kann außerdem ausgebildet sein zumindest eine in einem Pixelelement 50 der Teilzahl der Vielzahl an Pixelelementen 50 gezählte Anzahl an Koinzidenz-Zählsignalen auf zumindest eines der weiteren Pixelelemente 50, mit welchem in dem jeweiligen Pixelelement ein Koinzidenz-Zählsignal gebildet wird, zu übertragen. Das Erzeugen kann dann außerdem auf der zumindest einen übertragenen Anzahl an Koinzidenz-Zählsignalen basieren. Eine übertragene Anzahl kann in analoger Weise wie eine korrespondierende gezählte Anzahl in das Verfahren eingehen.

Die Erzeugungseinheit kann ausgebildet sein für ein Vorverarbeiten der Daten, d.h. ein Vorverarbeiten der Anzahlen vor einer Bildrekonstruktion, für ein Rekonstruieren des Röntgenbilddatensatzes oder eines vorläufigen Bilddatensatzes basierend auf den, optional vorverarbeiten, Daten und/oder für ein Nachverarbeiten eines rekonstruierten, vorläufigen Bilddatensatzes zur Erzeugung des endgültigen Röntgenbilddatensatzes. Es ist aber auch möglich, dass beispielweise zumindest der Schritt des Vorverarbeitens teilweise oder ganz im Sinne eines "on-board processings" (On-Board-Verarbeitung) von dem Röntgendetektor oder einer diesem zugeordneten peripheren Elektronik 61 bereits vor einer Übertragung an eine, möglicherweise physikalisch getrennt angeordnete, Erzeugungseinheit 71ausgeführt wird.

Die Erzeugungseinheit 71 kann weiterhin dazu ausgebildet sein, den Röntgenbilddatensatz mittels einer Schnittstelle auszugeben. Der Röntgenbilddatensatz kann beispielsweise an eine Ausgabeeinheit 49 in Form eines Monitors für eine Darstellung des Röntgenbilddatensatzes für einen Anwender oder an eine Speichereinheit für die Speicherung des Röntgenbilddatensatz ausgegeben werden.

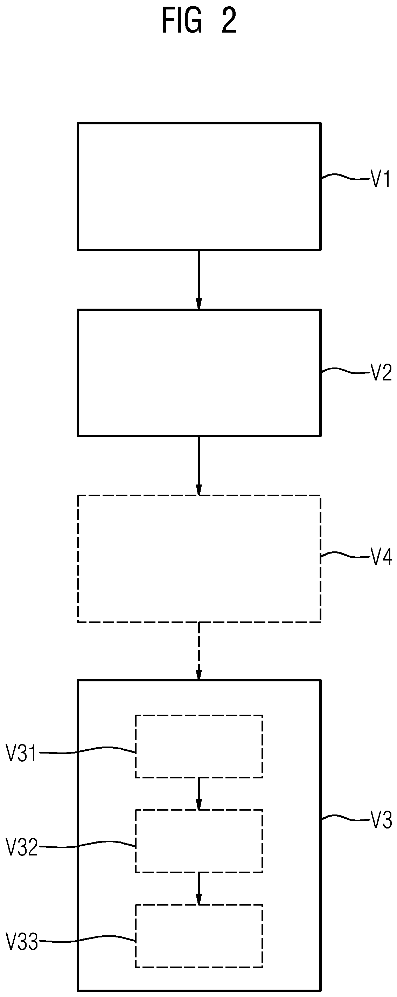

Die Fig. 2 zeigt einen schematischen Verfahrensablauf eines Verfahrens zur Erzeugung eines Röntgenbilddatensatz mittels eines Röntgendetektors 1 aufweisend ein Konverterelement 3, welches ausgebildet ist Röntgenstrahlung in ein elektrisches Signal umzuwandeln, und mit einer Vielzahl an Pixelelementen 50, jeweils ausgebildet ein Zählsignal basierend auf einem in einem Pixelelement 50 der Vielzahl an Pixelelementen 50 direkt eingegangenem Signal zu bilden und wobei zumindest eine Teilzahl der Vielzahl an Pixelelementen 50 ausgebildet ist, ein Koinzidenz-Zählsignal, welches auf einem in dem Pixelelement 50 der Teilzahl der Vielzahl an Pixelelementen 50 direkt eingegangenem Signal und auf einem koinzidierend auftretendem Signal mindestens eines weiteren Pixelelements 50 der Vielzahl an Pixelelementen basiert, zu bilden.

Das Verfahren umfasst den Schritt des ersten Zählens V1 zumindest einer Anzahl T an Zählsignalen in Abhängigkeit der eintreffenden Röntgenstrahlung in jedem Pixelelement 50 der Vielzahl an Pixelelementen 50. Das Verfahren umfasst weiterhin den Schritt des zweiten Zählens V2 zumindest einer Anzahl C an Koinzidenz-Zählsignalen in jedem Pixelelement 50 der Teilzahl der Vielzahl an Pixelelementen 50, welches auf dem in dem Pixelelement 50 der Teilzahl an Pixelelementen 50 direkt eingegangenem Signal und auf einem koinzidierend auftretendem Signal mindestens eines weiteren Pixelelements 50 der Vielzahl an Pixelelementen 50 basiert.

Die Anzahl T an Zählsignalen kann dabei während eines Belichtungszeitfenster oder Auslesezeitfenster gezählt werden. Dabei kann das Belichtungszeitfenster dem Zeitfenster entsprechen, während dem der Röntgendetektor 1 bzw. das jeweilige Pixelelement 50 der Vielzahl an Pixelelementen 50 mit Röntgenstrahlung belichtet wird. Ein Auslesezeitfenster kann beispielsweise dem Zeitfenster entsprechen, zwischen einem ersten Auslesen der gezählten Anzahlen und einem, zeitlich darauf folgenden, zweiten Auslesen der gezählten Anzahlen, welche seit dem ersten Auslesen gezählt wurden. Die gezählte Anzahl an Zählsignalen kann ein Maß für eine Intensität der Röntgenstrahlung liefern, welche während des Belichtungszeitfensters oder Auslesezeitfensters auf das Detektionsvolumen 60 des Pixelelements 50 der Vielzahl an Pixelelementen 50 trifft. Basierend auf der zumindest einen Anzahl T an Zählsignalen kann beispielsweise ein Röntgenbilddatensatz erzeugt werden, zu welchem lediglich die Röntgenphotonen der Röntgenstrahlung beitragen, welche jeweils direkt auf einem Pixelelement 50 (bzw. dem Detektionsvolumen 60, welches dem Pixelelement 50 zugeordnet ist) eingetroffen sind.

Die zumindest eine Anzahl C an Koinzidenz-Zählsignalen kann insbesondere im Wesentlichen zeitgleich mit der zumindest einen Anzahl T an Zählsignalen gezählt werden. Die zumindest eine Anzahl C an Koinzidenz-Zählsignalen kann insbesondere während desselben Belichtungszeitfenster oder Auslesezeitfenster gezählt werden wie die zumindest eine Anzahl T an Zählsignalen. Durch das Zählen der zumindest einen Anzahl C an Koinzidenz-Zählsignalen kann ein Röntgenbilddatensatz erzeugt werden, bei welchem koinzidierend in z. B. benachbarten, Pixelelementen 50 der Vielzahl an Pixelelementen 50 auftretende Signale mit in das Erzeugen des Röntgenbilddatensatzes einbezogen werden können. Dadurch können insbesondere Störeffekte auf die Anzahl T an Zählsignalen in den Pixelelementen 50 der Vielzahl an Pixelelementen 50, beispielsweise durch Fluoreszenzen oder Charge-Sharing, kompensiert bzw. korrigiert werden. Vorteilhaft kann dadurch eine verbesserte Bildqualität erreicht werden. Insbesondere kann dadurch ein verringertes Rauschen erreicht werden, beispielsweise quantitativ ausgedrückt durch ein verbessertes Signal-zu-Rausch-Verhältnis (SNR). Es kann auch je nach Korrektur oder Anwendung der Koinzidenzinformation eine verbesserte Ortsauflösung und/oder eine verbesserte spektrale Information ermöglicht werden.

Im Rahmen der Schritte des ersten Zählens V1 und/oder des zweiten Zählens V2 können insbesondere auch mehr als eine Anzahl T an Zählsignalen und/oder mehr als eine Anzahl C an Koinzidenz-Zählsignalen in jedem der Pixelelemente 50 der Vielzahl an Pixelelementen 50 bzw. der Teilzahl der Vielzahl an Pixelelementen 50 ermittelt werden. Beispielsweise können in Abhängigkeit mehrere bereitgestellter Energieschwellen S in einem jeweiligen Pixelelement 50 der Vielzahl an Pixelelementen 50 mehrere Anzahlen T an Zählsignalen und/oder in einem jeweiligen Pixelelement 50 der Teilzahl an Pixelelementen 50 mehrere Anzahlen C an Koinzidenz-Zählsignalen gezählt werden.

Es können auch mit mehr als einem weiteren Pixelelement 50 der Vielzahl an Pixelelementen 50 Koinzidenz-Zählsignale gebildet werden. Vorzugsweise werden in jedem Pixelelement 50 der Teilzahl der Vielzahl an Pixelelementen 50 mit zwischen einem und 24 weiteren Pixelelementen 50 der Vielzahl an Pixelelementen 50 Koinzidenz-Zählsignale gebildet. Bevorzugt umfassen die 1 bis 24 weiteren Pixelelemente ein direkt benachbartes, ein diagonal benachbartes Pixelelement der Vielzahl an Pixelelementen oder einen übernächsten Nachbar des Pixelelements der Teilzahl der Vielzahl an Pixelelementen.

Dabei kann die im Schritt des zweiten Zählens V2 für jedes Pixelelement 50 der Teilzahl an Pixelelementen 50 gezählte zumindest eine Anzahl C an Koinzidenz-Zählsignalen auf koinzidierend auftretenden Signalen aller weiteren, beteiligten Pixelelemente 50 basieren. Das heißt, die zumindest eine Anzahl an Koinzidenz-Zählsignalen kann eine Summe der koinzidierend auftretenden Signale des betrachteten Pixelelements 50 der Teilzahl an Pixelelementen 50 und der 1 bis 24 weiteren beteiligten Pixelelementen 50 der Vielzahl an Pixelelementen 50 repräsentieren. Alternativ dazu kann im Schritt des zweiten Zählens S2 auch jeweils zumindest eine Anzahl C an Koinzidenz-Zählsignalen mit jedem der 1 bis 24 weiteren Pixelelementen 50 und damit mehrere Anzahlen C an Koinzidenzzählsignalen in Abhängigkeit der beteiligten Pixelelemente 50 gezählt werden.

Das Verfahren kann außerdem den Schritt des Übertragens V4 umfassen, wobei zumindest eine in einem Pixelelement 50 der Teilzahl der Vielzahl an Pixelelementen 50 gezählten Anzahl C an Koinzidenz-Zählsignalen auf zumindest eines der weiteren Pixelelemente 50, mit welchem in dem jeweiligen Pixelelement 50 ein Koinzidenz-Zählsignal gebildet wird, übertragen wird. Der Schritt des Erzeugens V3 kann dann auf zumindest einer übertragenen Anzahl C an Koinzidenz-Zählsignalen basieren.

Diese Variante erlaubt, auch für Pixelelemente 50 der Vielzahl an Pixelelementen 50, welche nicht Teil der Teilzahl der Vielzahl an Pixelelementen 50 sind, Anzahlen an Koinzidenz-Zählsignalen zu ermitteln und Koinzidenzinformation zu sammeln, welche in dem Verfahren zur Erzeugung eines Röntgenbilddatensatz und seiner Varianten in analoger Weise wie eine in dem Pixelelement 50 gezählte Anzahl an Koinzidenz-Zählsignalen eingesetzt werden kann. Beispielsweise ist in einer matrixartigen Anordnung der Vielzahl an Pixelelemente 50 nur jeweils jedes zweite Pixelelement 50 der Vielzahl an Pixelelementen 50 Teil der Teilzahl und damit ausgebildet, jeweils zumindest eine Anzahl C an Koinzidenz-Zählsignalen zumindest mit seinen vier direkt benachbarten Pixelelementen 50 zu zählen. Für den Rest der Vielzahl an Pixelelementen 50 kann die korrespondierende Koinzidenzinformation dann durch eine Übertragung V4 ermittelt werden. Es sind daneben auch anderweitige Ausführungsvarianten möglich.

Diese Variante erlaubt ebenfalls, ein redundantes Zählen an Koinzidenz-Zählsignalen in den Pixelelementen 50 der Teilzahl der Vielzahl an Pixelelementen 50 zu reduzieren oder die gesammelte Koinzidenzinformation in einem Pixelelement 50 der Teilzahl der Vielzahl durch ein Übertragen von Anzahlen zu erweitern.

Beispielsweise zählt jedes Pixelelement 50 der Vielzahl jeweils eine Anzahl C an Koinzidenz-Zählsignalen mit seinem, in einem exemplarischen, orthogonalen Pixelraster, nördlich und westlichen direkt benachbarten und den diagonal benachbarten nord-westlichen und südwestlichen Pixelelement 50. Die Koinzidenzinformation eines jeweiligen Pixelelements 50 zu den restlichen direkt benachbarten oder diagonal benachbarten Pixelelementen 50 kann dann mittels einer Übertragung von Anzahlen von diesen Pixelelementen 50 ermittelt werden.

Eine auf ein Pixelelement 50 übertragene Anzahl C an Koinzidenz-Zählsignalen kann in dem Verfahren zur Erzeugung eines Röntgenbilddatensatzes und seiner beschriebenen Varianten in analoger Weise eingesetzt werden, wie als wenn die entsprechende Anzahl an Koinzidenz-Zählsignalen in dem Pixelelement 50 selbst gezählt worden wäre.

Bevorzugt liegt einer übertragenen Anzahl C an Koinzidenz-Zählsignalen eine pixelweise Koinzidenzinformation zugrunde, d.h. die zu übertragende Anzahl basiert auf Koinzidenz-Zählsignalen, welche lediglich zwischen dem Pixelelement 50, von welchem die Anzahl übertragen wird, und dem Pixelelement 50, auf welches die Anzahl übertragen wird, gebildet werden. Es kann jedoch Varianten des Verfahrens geben, wobei, sofern mehrere weitere Pixelelemente 50 für die Bildung von Koinzidenz-Zählsignalen vorgesehen sind, eine Anzahl C an Koinzidenz-Zählsignalen übertragen wird, welche auf koinzidierend auftretenden Signalen aller weiteren Pixelelemente 50 basiert. Hier kann die Annahme zugrunde liegen, dass zumindest für Pixelelemente 50, welche in nächster Nähe angeordnet sind, und damit ähnlichen Bedingungen ausgesetzt sind, auch basierend auf einer Summe zumindest eine Abschätzung einer Anzahl C an Koinzidenz-Zählsignalen möglich sein kann, welche in einem Verfahren zur Erzeugung eines Röntgenbilddatensatzes vorteilhaft angewendet werden kann.

In Verfahrensvarianten kann insbesondere für jedes Pixelelement 50 der Vielzahl an Pixelelementen 50 zumindest eine gezählte oder eine übertragene Anzahl C an Koinzidenz-Zählsignalen ermittelt werden, so dass für jedes Pixelelement 50 der Vielzahl an Pixelelementen 50 Koinzidenzinformation zur Verfügung steht.

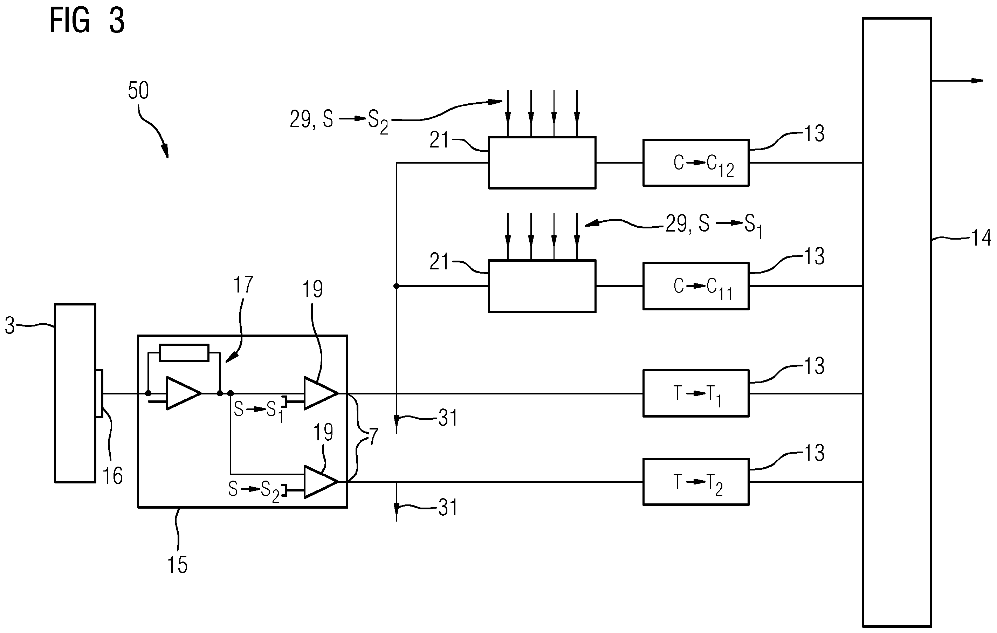

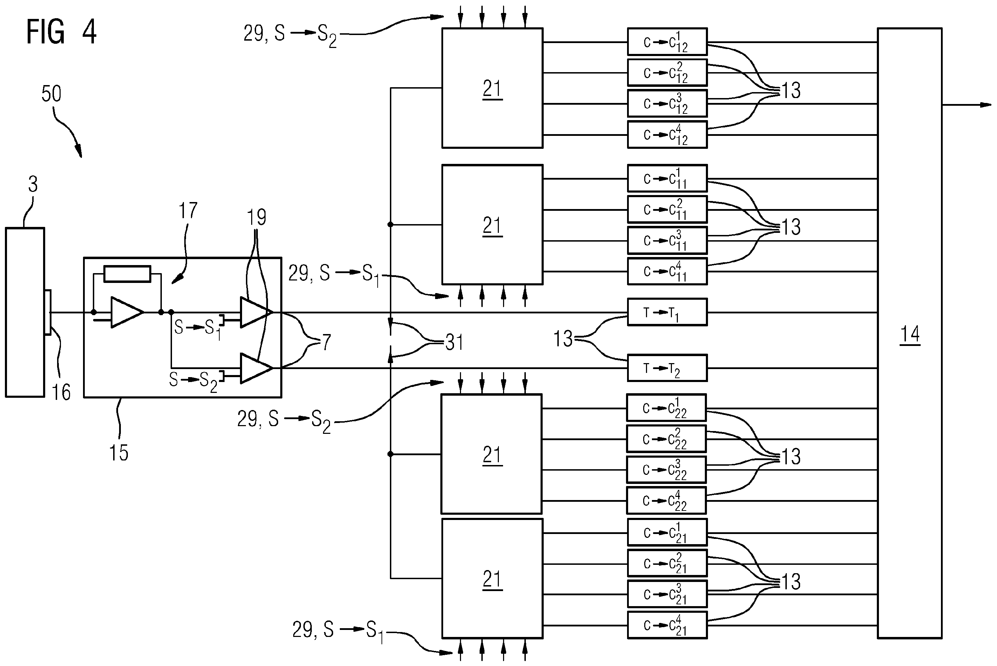

Eine vorteilhafte Repräsentation der gesammelten Koinzidenzinformation eines jeweiligen Pixelelements 50 in Form gezählter und/oder übertragener Anzahlen C an Koinzidenz-Zählsignalen kann mittels einer, zumindest teilweise gefüllten, Koinzidenzmatrix $\hat{C}$ bereitgestellt werden. Die gezählten und/oder übertragenen Anzahlen C an Koinzidenz-Zählsignalen können dann als Einträge der zumindest teilweise gefüllten Koinzidenzmatrix $\hat{C}$ eines jeweiligen Pixelelements 50 dienen. Die Koinzidenzmatrix $\hat{C}$ kann dann die gesammelte Koinzidenzinformation eines jeweiligen betrachteten Pixelelements 50 in Abhängigkeit des zumindest einen, bevorzugt der mehreren, weiteren Pixelelementen 50 der Vielzahl an Pixelelementen 50, und/oder in Abhängigkeit der einen oder der mehreren Energieschwellen S (basierend auf welcher bzw. welchen Koinzidenz-Zählsignale gebildet werden) repräsentieren. Die Dimensionen einer solchen Koinzidenzmatrix $\hat{C}$ und deren Befüllung ist abhängig von der konkreten Implementierung der signaltechnischen Verschaltung der Pixelelemente 50 des Röntgendetektors 1 und/oder der Möglichkeiten der Übertragung von Anzahlen C auf zumindest ein weiteres Pixelelement 50. Eine solche Koinzidenzmatrix $\hat{C}$ wird in der Figurenbeschreibung in Bezug auf die Figuren 3 und 4 näher ausgeführt. Das in Fig. 2 dargestellte Verfahren zur Erzeugung eines Röntgenbilddatensatz umfasst neben den Schritten des ersten Zählens V1 und des zweiten Zählens V2 außerdem den Schritt des Erzeugens V3 des Röntgenbilddatensatzes basierend auf der in jedem Pixelelement 50 der Vielzahl an Pixelelementen 50 gezählten zumindest einen Anzahl T an Zählsignalen und der in jedem Pixelelement 50 der Teilzahl der Vielzahl an Pixelelementen 50 gezählten zumindest einen Anzahl C an Koinzidenz-Zählsignalen. Dies kann außerdem auf einer auf einer gezählten Anzahl C an Koinzidenz-Zählsignalen basierenden übertragene Anzahl C an Koinzidenz-Zählsignalen basieren. Die Koinzidenz-Zählsignale gehen dabei erfindungsgemäß in das Erzeugen V3 mit ein. Werden mehrere Anzahlen T an Zählsignalen und/oder mehrere Anzahlen C an Koinzidenz-Zählsignalen ermittelt, d.h. gezählt oder übertragen, können entsprechend auch mehrere Anzahlen in das Erzeugen miteingehen. Basierend auf einer Anzahl C an Koinzidenz-Zählsignale können beispielsweise die gezählten Anzahlen T an Zählsignalen korrigiert werden, die Anzahlen C an Koinzidenz-Zählsignale können als Randbedingung in einer Rekonstruktion, als zusätzliche Bildinformation oder auch anderweitig in das Erzeugen V3 des Röntgenbilddatensatzes eingehen.

Der erzeugte Röntgenbilddatensatz kann insbesondere ein dreidimensionaler Bilddatensatz (3D Bilddatensatz) oder ein zweidimensionaler Bilddatensatz (2D Bilddatensatz) sein. Ein 2D Bilddatensatz erlaubt eine zweidimensionale, insbesondere räumlich zweidimensionale, Darstellung. Ein 3D Bilddatensatz erlaubt insbesondere eine dreidimensionale, insbesondere räumlich dreidimensionale, Darstellung. Ein dreidimensionaler Bilddatensatz kann auch als eine Anzahl an Schichtbilddatensätzen dargestellt werden, wobei ein Schichtbilddatensatz dann jeweils eine zweidimensionale, insbesondere räumlich zweidimensionale, Darstellung einer jeweiligen Schicht des 3D Bilddatensatzes erlaubt. Ein 3D Bilddatensatz umfasst in der Regel mehrere Voxel, auch Bildpunkte genannt. Analog dazu kann ein zweidimensionaler Bilddatensatz mehrere (Bild-)Pixel, ebenso auch Bildpunkte genannt, umfassen. Dabei kann jeder Bildpunkt jeweils einen Wert, insbesondere einen Bildwert, aufweisen, beispielsweise einen Grauwert und/oder einen RGB-Farbwert und/oder einen Intensitätswert. Die Bildwerte der Voxel bzw. Pixel des erzeugten Röntgenbilddatensatzes können dann insbesondere auf den in der Vielzahl an Pixelelementen 50 gezählten jeweils zumindest einen Anzahl T an Zählsignalen und den zumindest in der Teilzahl der Vielzahl an Pixelelementen gezählten jeweils zumindest einen gezählten Anzahl C an Koinzidenz-Zählsignalen basieren.

Wird das Verfahren beispielsweise mittels eines Computertomographie-Geräts durchgeführt, kann das Erzeugen zumindest das Rekonstruieren eines 3D Bilddatensatzes oder eines 2D Bilddatensatzes in Form eines Schichtbilddatensatzes mittels eines CT-Rekonstruktionsalgorithmus, beispielsweise in Form einer gefilterten Rückprojektion oder eines iterativen CT-Rekonstruktionsalgorithmus, umfassen.

Der Schritt des Erzeugens V3 kann in Teilschritte untergliedert sein. Im in Fig. 2 dargestellten schematischen Verfahrensablauf ist angedeutet, dass das Erzeugen V3 beispielsweise einen Teilschritt V31 des Vorverarbeitens der Daten vor einer Bildrekonstruktion, einen Teilschritt V32 des Rekonstruierens eines Röntgenbilddatensatzes oder eines vorläufigen Bilddatensatzes basierend auf den ggf. vorverarbeiten Daten und einen Teilschritt des Nachverarbeitens V33 des rekonstruierten Röntgenbilddatensatzes umfassen kann. Zumindest in einen der Teilschritte kann die im Schritt des zweiten Zählens V2 gezählte oder die im Schritt des Übertragens V4 ermittelte Koinzidenzinformation eingehen. Insbesondere kann in jeden der Teilschritte oder auch in mehreren der Teilschritte die im Schritt des zweiten Zählens V2 oder die im Schritt des Übertragens V4 ermittelte Koinzidenzinformation, basierend auf der in jedem Pixelelement 50 der Teilzahl an Pixelelementen gezählten zumindest einen Anzahl C an Koinzidenz-Zählsignale, eingehen.

Der Schritt V31 des Vorverarbeitens kann unter anderem ein Linearisieren oder ein Logarithmieren der Zählinformation umfassen. Es können Korrekturen durchgeführt werden, beispielsweise eine sogenannte "Gain-Korrektur" oder "Offset-Korrektur" zur Angleichung gegebenenfalls leicht unterschiedlichen Ansprechverhaltens der Pixelelemente untereinander bei Bestrahlung mit Röntgenstrahlung oder eine Korrektur bezüglich defekter Pixelelemente. Gemäß einer Variante des Verfahrens kann in einem Schritt V31 des Vorverarbeitens in jedem Pixelelement 50 der Teilzahl der Vielzahl an Pixelelementen 50 die zumindest eine gezählte Anzahl T an Zählsignalen mittels der zumindest einen Anzahl C an Koinzidenz-Zählsignalen angepasst werden.

Der Schritt V32 des Rekonstruierens kann unter anderem beispielsweise eine Bildrekonstruktion basierend auf einer gefilterten Rückprojektion oder basierend auf einem iterativen Rekonstruktionsalgorithmus umfassen. Eine Ausgestaltungsvariante des Verfahrens zur Erzeugung des Röntgenbilddatensatzes kann dabei vorsehen, dass die jeweilige in einem Pixelelement gezählte oder übertragene zumindest eine Anzahl C an Koinzidenz-Zählsignalen in einen iterativen Rekonstruktionsalgorithmus als zusätzliche Information eingeht.

Der Schritt V33 des Nachverarbeitens kann unter anderem beispielsweise eine Fensterung der Bildwerte des Röntgenbilddatensatzes, weitere Korrekturen, beispielsweise hinsichtlich Metallartefakte, oder eine Segmentierung von im Röntgenbilddatensatz enthaltenen Strukturen umfassen. In einer Ausgestaltungsvariante des Verfahrens zur Erzeugung des Röntgenbilddatensatzes kann der Schritt V3 des Erzeugens umfassen, dass basierend auf der zumindest einen Anzahl T an gezählten Zählsignalen in jedem Pixelelement 50 der Vielzahl an Pixelelementen 50 zumindest ein vorläufiger Bilddatensatz erzeugt wird und basierend auf der zumindest einen Anzahl C an Koinzidenz-Zählsignalen in jedem Pixelelement 50 der Teilzahl an Pixelelementen 50 zumindest ein Koinzidenz-Bilddatensatz erzeugt wird, wobei in dem Teilschritt V33 des Nachverarbeitens der zumindest eine Koinzidenz-Bilddatensatz auf den zumindest einen vorläufigen Bilddatensatz angewendet wird. Der Koinzidenz-Bilddatensatz kann dabei auch auf einer übertragenen Anzahl C an Koinzidenz-Zählsignalen basieren.

Beispiele für das Eingehen der Koinzidenzinformation in den Schritt des Erzeugens werden im Folgenden noch konkreter ausgeführt. Zuvor zeigen die Figuren 3 und 4 jeweils eine schematische Veranschaulichung einer exemplarischen signaltechnischen Verschaltung eines Pixelelements 50 der Teilzahl an Pixelelementen 50 eines Röntgendetektors 1 in unterschiedlichen Ausführungsformen. Die exemplarisch gezeigten Beispiele zeigen dabei Möglichkeiten einer Verschaltung, welche es erlauben können, ein Zählsignal basierend auf einem in dem gezeigten Pixelelement 50 direkt eingegangenem Signal zu bilden und darauf basierend zumindest eine Anzahl T an Zählsignalen zu zählen, sowie ein Koinzidenz-Zählsignal basierend auf dem in dem gezeigten Pixelelement 50 direkt eingegangenem Signal und auf einem koinzidierend auftretendem Signal mindestens eines weiteren Pixelelements 50 der Vielzahl an Pixelelementen 50 zu bilden und darauf basierend zumindest eine Anzahl C an Koinzidenz-Zählsignalen zu zählen. Die Ausführungsformen sind dabei exemplarisch zur Veranschaulichung der grundlegenden Funktionsweise gewählt. Darüber hinaus sind weitere, davon abweichende Verschaltungen der Pixelelemente 50 im Rahmen der Erfindung möglich, welche ebenso das Zählen eines Koinzidenz-Zählsignal und das Zählen eines Zählsignals erlauben. In den Figuren ist außerdem lediglich jeweils die signaltechnische Verschaltung eines exemplarisches Pixelelement 50 der Teilzahl an Pixelelementen 50 und lediglich in einer stark abstrahierten Form mit den für eine Veranschaulichung notwendigen Komponenten angedeutet. Die Verschaltung kann jedoch leicht auch auf die anderen Pixelelemente 50 der mindestens Teilzahl an Pixelelementen 50 übertragen werden. Daneben können auch weitere Verarbeitungskomponenten in einem Pixelelement 50, d.h. in der pixelweisen Pixelelektronik eines Pixelelements 50, vorgesehen sein.

In Fig. 3 weist das exemplarisch dargestellte Pixelelement 50 der Teilzahl an Pixelelementen 50 eine Wandlungsvorrichtung 15 mit zumindest einem Signalverstärker 17 und eine Anzahl, in diesem exemplarischen Fall zwei, Komparatoren 19 auf. Der Signaleingang der Wandlungsvorrichtung 15, insbesondere des Signalverstärkers 17, ist mit dem zugeordneten sensitiven Detektionsvolumen im Konverterelement 3 über die Sensor-Pixelelektrode 16 signaltechnisch gekoppelt. Der Signalverstärker 17 verstärkt das direkt in dem Pixelelement 50 über die Sensor-Pixelelektrode 16 eingegangene und durch eintreffende Röntgenstrahlung mittels des Konverterelements 3 erzeugte elektrische Signal für die folgende Weiterverarbeitung.

Die Komparatoren 19 des gezeigten Pixelelements 50 weisen jeweils eine einstellbare Energieschwelle S auf. Die einstellbaren Energieschwellen S unterschiedlicher Komparatoren 19 können auf unterschiedliche Energieschwellwerte eingestellt sein. In der Figur ist dies insbesondere durch die Angabe $S \to S_1$ und $S \to S_2$ verdeutlicht, womit im Wesentlichen ausgedrückt werden soll, dass die Komparatoren zwei unterschiedlich eingestellte Energieschwellen $S \to S_1$ und $S \to S_2$ mit unterschiedlichen Energieschwellwerten aufweisen. Im Folgenden kann auch von einer ersten Energieschwelle $S \to S_1$ und einer zweiten Energieschwelle $S \to S_2$ des Pixelelements 50 gesprochen werden. Beispielsweise weist die Energieschwelle $S \to S_1$ einen, bezogen auf die Energie der eintreffenden Röntgenstrahlung, niedrigeren Energieschwellwert auf als die Energieschwelle $S \to S_2$. Üblicherweise sind die Energieschwellen S unterschiedlicher Pixelelemente 50 der Vielzahl an Pixelelementen 50 jeweils auf dieselben Energieschwellwerte eingestellt. Es kann jedoch auch andere Ausführungen geben.

Mittels der Komparatoren 19 wird das in dem betrachtete Pixelelement 50 über die Sensor-Pixelelektrode 16 direkt eingegangene Signal mit den jeweiligen Energieschwellen $S \to S_1$ und $S \to S_2$ der Komparatoren 19 verglichen. Bei Überschreiten einer entsprechenden Energieschwelle S wird am Signalausgang 7 desjenigen Komparators 19, dessen Energieschwelle S überschritten wurde, ein Ausgangssignal gebildet, welches als Zählsignal dient. Werden beide Energieschwellen $S \to S_1$ und $S \to S_2$ überschritten, wird jeweils ein Zählsignal an beiden Signalausgängen 7 der Komparatoren 19 gebildet.

Gemäß einer Ausführung des Röntgendetektors 1 wird in jedem Pixelelement 50 der Teilzahl an Pixelelementen 50 basierend auf dem Ausgangssignal am Signalausgang 7 zumindest eines Komparators 19 der Anzahl an Komparatoren eines jeweiligen Pixelelements 50 die zumindest eine Anzahl T an Zählsignalen und basierend auf dem Ausgangssignal 7 zumindest eines Komparators 19 der Anzahl an Komparatoren des jeweiligen Pixelelements 50 der Teilzahl an Pixelelementen 50 und zumindest auf dem Ausgangssignal 7 eines Komparators 19 der Anzahl an Komparatoren des zumindest einen weiteren Pixelelements 50 der Vielzahl an Pixelelementen 50 die zumindest eine Anzahl C an Koinzidenz-Zählsignalen gezählt. Im Allgemeinen kann dabei in einem Pixelelement 50 der Teilzahl derjenige Komparator 19, auf welchem die zumindest eine Anzahl T an Zählsignalen basiert, von demjenigen Komparators 19 der Anzahl an Komparatoren, auf welchem die zumindest eine Anzahl C an Koinzidenz-Zählsignalen basiert, verschieden sein. Es kann jedoch auch, wie in diesem Beispiel, der gleiche Komparator 19 sein.

Im gezeigten Beispiel in Fig. 3 sind die Signalausgänge 7 der Komparatoren 19 jeweils mit einem Zählelement 13 signaltechnisch verknüpft. Ein jeweiliges Zählelement 13 ist ausgebildet eine Anzahl T an Zählsignalen, basierend auf dem Ausgangssignal eines jeweils verknüpften Komparators 19 zu zählen. In dem gezeigten Fall wird basierend auf den Ausgangssignalen der zwei Komparatoren 19 jeweils eine Anzahl T an Zählsignalen gezählt. Das heißt, im gezeigten Fall zählt eines der Zählelemente 13 eine Anzahl $T \to T_1$ an Zählsignalen in Abhängigkeit der Energieschwelle $S \to S_1$ und ein weiteres Zählelement 13 eine Anzahl $T \to T_2$ an Zählsignalen in Abhängigkeit der Energieschwelle $S \to S_2$.

Im gezeigten Fall in Fig. 3 ist der Komparator 19 aufweisend die Energieschwelle $S \to S_1$ außerdem mit zwei Koinzidenzlogiken 21 signaltechnisch verknüpft. In anderen Ausführungen kann auch nur eine oder noch weitere Koinzidenzlogiken 21 vorgesehen sein. Bevorzugt ist vorgesehen, dass der Signalausgang 7 zumindest einer der Komparatoren 19 der Anzahl an Komparatoren 19 eines Pixelelements 50 der Teilzahl an Pixelelementen 50 mit zumindest einer Koinzidenzlogik 21 signaltechnisch verknüpft ist. Basierend auf dem Ausgangssignal der jeweiligen Koinzidenzlogik 21 kann dann mittels eines weiteren, mit der jeweiligen Koinzidenzlogik 21 verknüpften Zählelements 13 eine Anzahl C an Koinzidenz-Zählsignalen gezählt werden.

Eine jeweilige Koinzidenzlogik 21 ist ausgebildet ein Koinzidenz-Zählsignal, welches auf dem in dem Pixelelement 50 der Teilzahl an Pixelelementen 50 direkt eingegangenem Signal und auf einem koinzidierend auftretenden Signal mindestens eines weiteren Pixelelements 50 der Vielzahl an Pixelelementen basiert, zu bilden. Eine jeweilige Koinzidenzlogik 21 ist dazu außerdem mit zumindest einem weiteren Pixelelement 50 der Vielzahl an Pixelelementen über zumindest einen weiteren Signaleingang 29 der Koinzidenzlogik 21 verknüpft. Insbesondere ist eine jeweilige Koinzidenzlogik 21 gemäß einer bevorzugten Ausbildungsform ausgebildet, basierend auf dem Ausgangssignal 7 des zumindest einen mit der Koinzidenzlogik 21 gekoppelten Komparators 19 des betrachteten Pixelelements 50 und basierend zumindest auf dem Ausgangssignal eines Komparators 19 zumindest eines weiteren, hier nicht dargestellten, Pixelelements 50 ein Koinzidenz-Zählsignal zu bilden und an einem Signalausgang der Koinzidenzlogik 21 bereitzustellen. Das heißt, in dieser Ausführungsform ist ein jeweiliger Signaleingang 29 der Koinzidenzlogik 21 jeweils mit einem Komparator 19 der Anzahl an Komparatoren eines weiteren Pixelelements 50 der Vielzahl an Pixelelementen 50 signaltechnisch gekoppelt. Im konkret gezeigten Beispiel in Fig. 3 ist eine jeweilige Koinzidenzlogik 21 exemplarisch mit vier weiteren Pixelelementen 50 der Vielzahl an Pixelelementen signaltechnisch verknüpft. Dazu weist eine jeweilige Koinzidenzlogik 21 vier Signaleingänge 29 auf, welche jeweils mit einem weiteren Pixelelement 50 der Vielzahl an Pixelelementen 50 signaltechnisch gekoppelt ist.

In dem gezeigten Fall ist die untere der Koinzidenzlogiken 21 mit einem Komparator 19 der Anzahl an Komparatoren des jeweiligen weiteren Pixelelements 50 verknüpft, welcher die Energieschwelle $S \to S_1$ aufweist (angedeutet in Fig 3. durch den Hinweis $S \to S_1$ bezogen auf die Signalausgänge 29 der in der Figur unteren Koinzidenzlogik 21). Entsprechend entspricht die mittels des mit dieser Koinzidenzlogik 21 verknüpften Zählelements 13 gezählte Anzahl C an Koinzidenz-Zählsignalen derjenigen Anzahl an koinzidierend auftretenden Signalen, welche in dem betrachteten Pixelelement 50 und zumindest in einem der weiteren mit der Koinzidenzlogik 21 verknüpften Pixelelemente 50 die Energieschwelle $S \to S_1$ überschritten haben. In anderen Worten, in diesem Fall wird eine Anzahl $C \to C_{11}$ an Koinzidenz-Zählsignalen der Energieschwelle $S \to S_1$ des betrachteten Pixelelements 50 mit der Energieschwelle $S \to S_1$ zumindest eines der weiteren Pixelelemente 50 der Vielzahl an Pixelelementen 50 gezählt.

Ausgehend von der Annahme, dass die Energieschwelle $S \to S_1$ in jedem Pixelelement 50 der Vielzahl an Pixelelementen 50 auf denselben Energieschwellwert eingestellt ist, weist gemäß einer bevorzugten Ausbildungsvariante dann die einstellbare Energieschwelle S des zumindest einen Komparators 19 der Anzahl an Komparatoren des betrachteten Pixelelements 50 der Teilzahl an Pixelelementen 50 und die einstellbare Energieschwelle S des zumindest einen Komparators 19 der Anzahl an Komparatoren des zumindest einen weiteren Pixelelements 50 der Vielzahl an Pixelelementen 50 (in dem gezeigten Beispiel jeweils der vier weiteren Pixelelemente 50) den gleichen Energieschwellwert auf. Das heißt, in diesem Fall wird eine Anzahl $C \to C_{11}$ an Koinzidenz-Zählersignalen basierend auf der gleichen Energieschwelle $S \to S_1$ der jeweils beteiligten Pixelelemente 50 der Vielzahl an Pixelelementen 50 gezählt. Dies kann auch als (energie-) symmetrische Überschreitung der Energieschwellen oder (energie-)symmetrische Koinzidenz bezeichnet werden.

In dem in Fig. 3 gezeigten Fall ist erfindungsgemäß die obere der Koinzidenzlogiken 21 jeweils über einen Signaleingang 29 mit einem Komparator 19 der Anzahl an Komparatoren des jeweiligen weiteren Pixelelements 50 verknüpft, welcher die Energieschwelle $S \to S_2$ aufweist (angedeutet in Fig 3. durch den Hinweis $S \to S_2$ bezogen auf die Signalausgänge 29 der in der Figur oberen Koinzidenzlogik 21). In anderen Worten, in diesem Fall wird eine Anzahl $C \to C_{12}$ an Koinzidenz-Zählsignalen der Energieschwelle $S \to S_1$ des betrachteten Pixelelements 50 der Teilzahl an Pixelelementen 50 mit der Energieschwelle $S \to S_2$ des zumindest einen weiteren Pixelelements 50 der Vielzahl an Pixelelementen (in diesem Beispiel der vier weiteren Pixelelemente 50) gezählt. Entsprechend entspricht eine basierend auf dem Ausgangssignal dieser Koinzidenzlogik 21 gezählte Anzahl $C \to C_{12}$ an Koinzidenz-Zählsignalen derjenigen Anzahl an koinzidierend auftretenden Signalen, welche in dem betrachteten Pixelelement 50 der Vielzahl an Pixelelementen 50 die Energieschwelle $S \to S_1$ und in zumindest einem der weiteren verknüpften Pixelelementen 50 die Energieschwelle $S \to S_2$ überschritten haben.

Ausgehend von der Annahme, dass die Energieschwellen $S \to S_1$ des betrachteten Pixelements und die Energieschwellen $S \to S_2$ des zumindest einen weiteren Pixelelements 50 der Vielzahl an Pixelelementen 50 auf jeweils unterschiedliche Energieschwellwerte eingestellt sind, bedeutet dies, dass für das zweite Zählen V2 der zumindest einen Anzahl C an Koinzidenz-Zählsignalen die einstellbare Energieschwelle S des zumindest einen Komparators 19 der Anzahl an Komparatoren des jeweiligen Pixelelements 50 der Teilzahl an Pixelelementen 50 und die einstellbare Energieschwelle S des zumindest einen Komparators 19 der Anzahl an Komparatoren des zumindest einen weiteren Pixelelements 50 der Vielzahl an Pixelelementen 50, auf welchen ein Koinzidenz-Zählsignal basiert, unterschiedliche Energieschwellwerte aufweisen. Das heißt, erfindungsgemäß wird eine Anzahl $C \to C_{12}$ an Koinzidenz-Zählersignalen basierend auf unterschiedlichen Energieschwellen $S \to S_1$ und $S \to S_2$ der jeweils beteiligten Pixelelemente 50 der Vielzahl an Pixelelementen 50 gezählt. Dies kann auch als (energie-)unsymmetrische Überschreitung der Energieschwellen S bezeichnet werden.

Weiterhin ist in dem in Fig. 3 gezeigten Beispiel gekoppelt mit den Komparatoren 19 jeweils ein Ausgang 31 vorgesehen, mittels dessen ein entsprechendes Ausgangssignal an ein oder mehrere weitere Pixelelemente 50 der Teilzahl an Pixelelementen 50 ausgegeben werden kann, um gleichermaßen als Eingangssignal einer Koinzidenzlogik 21 eines der weiteren, hier nicht dargestellten Pixelelemente 50 der Teilzahl an Pixelelementen 50 zu dienen.

Die Anzahlen $T \to T_1$ und $T \to T_2$ an Zählsignalen und die Anzahlen $C \to C_{11}$ und $C \to C_{12}$ an Koinzidenz-Zählsignalen können dann mittels eines Ausleseelements 14 von den Zählelementen 13 ausgelesen und gemeinsam mit den gezählten Anzahlen der Vielzahl an Pixelelementen 50 des Röntgendetektors 1, gegebenenfalls bereits vorverarbeitet, beispielweise an eine Erzeugungseinheit 71 für das Erzeugen V3 des Röntgenbilddatensatzes basierend auf den Anzahlen ausgegeben werden. Vorteilhaft können gezählte Anzahlen T an Zählsignalen und gezählte Anzahlen C an Koinzidenz-Zählsignale für das Erzeugen des Röntgenbilddatensatzes bereitgestellt werden.

Im gezeigten Beispiel ist eine jeweilige Koinzidenzlogik 21 exemplarisch mit vier weiteren Pixelelementen 50 der Vielzahl an Pixelelementen 50 signaltechnisch über die Signaleingänge 29 verknüpft. Das heißt, es werden Koinzidenz-Zählsignale basierend auf koinzidierend auftretenden Signalen von vier weiteren Pixelelementen 50 der Vielzahl an Pixelelementen 50 gebildet. Beispielsweise können die vier weiteren Pixelelemente 50 in Fig. 3 die vier direkt benachbarten Pixelelemente 50 des betrachteten Pixelelements 50 in einer matrixartigen Anordnung der Vielzahl an Pixelelementen 50 umfassen. Die direkt benachbarten Pixelelemente 50 eines betrachteten Pixelelements 50 können insbesondere diejenigen Pixelelemente 50 der Vielzahl an Pixelelementen umfassen, welche in einem durch die matrixartige Anordnung der Vielzahl an Pixelelementen 50 definiertes Pixelraster jeweils eine gemeinsame Kante aufweisen. Es kann jedoch auch eine andere Auswahl und/oder Anzahl an weiteren Pixelelementen 50 vorgesehen sein. Beispielsweise können alternativ oder zusätzlich Koinzidenz-Zählsignale basierend auf diagonal benachbarten Pixelelementen 50 gebildet werden. Es können beispielsweise auch übernächste Nachbarn, d.h. benachbarte Pixelelemente der direkt benachbarten Pixelelemente des betrachteten Pixelelements, hinsichtlich koinzidierend auftretender Signale betrachtet werden. Vorteilhaft werden zumindest mit denjenigen weiteren Pixelelementen 50 Koinzidenz-Zählsignale gebildet, in welchen mit hoher Wahrscheinlichkeit koinzidierende Signale auftreten. In bevorzugten Ausführungsvarianten werden insbesondere Koinzidenz-Zählsignale in jedem Pixelelement 50 der Teilzahl an Pixelelementen 50 mit zwischen einem und 24 weiteren Pixelelementen 50 der Vielzahl an Pixelelementen 50 gebildet.

Die Anzahl und Auswahl an weiteren Pixelelementen 50, auf welchen das Koinzidenz-Zählsignal basiert, kann auch innerhalb der Teilzahl der Vielzahl an Pixelelementen 50 variieren. Beispielsweise kann für randseitig in einer matrixartigen Anordnung der Vielzahl an Pixelelementen 50 angeordnete Pixelelemente 50 eine andere Anzahl und Auswahl an weiteren Pixelelementen 50 hinsichtlich koinzidierend auftretender Signale berücksichtigt werden als mittig in einer matrixartigen Anordnung der Vielzahl an Pixelelementen 50 angeordnete Pixelelemente 50. So weisen randseitig angeordnete Pixelelemente beispielsweise nur drei direkt benachbarte Pixelelemente auf.

In anderen Ausgestaltungsvarianten kann vorgesehen sein, dass alternativ oder zusätzlich eine Anzahl $C \to C_{22}$ und/oder eine Anzahl $C \to C_{21}$ basierend auf der Energieschwelle $S \to S_2$ des betrachteten Pixelelements 50 und entsprechenden Ausgangsignalen der Komparatoren der weiteren verknüpften Pixelelemente 50 der Vielzahl an Pixelelementen 50 gezählt wird. Entsprechend kann in dem in Fig. 3 gezeigten Beispiel alternativ oder zusätzlich zumindest eine weitere Koinzidenzlogik 21 vorgesehen sein, welche mit dem Komparator 19 mit der Energieschwelle $S \to S_2$ des betrachteten Pixelelements 50 gekoppelt ist. Ebenso kann in Ausgestaltungen vorgesehen sein, dass nur eine Anzahl C an Koinzidenz-Zählsignalen, beispielsweise eine Anzahl $C \to C_{12}$, gezählt wird. Weiterhin kann in alternativen Ausgestaltungsvarianten vorgesehen sein, dass nur ein Komparator 19 oder mehr als zwei Komparatoren 19 mit jeweils einer einstellbaren Energieschwelle S zur Bildung von Zählsignalen bzw. Koinzidenz-Zählsignalen vorgehalten werden.

Im Rahmen der Erfindung kann je nach Verschaltung des betrachteten Pixelelements 50 der Teilzahl an Pixelelementen zumindest eine Anzahl $C \to C_{nm}$ an Koinzidenz-Zählsignalen in dem betrachteten Pixelelement 50 gezählt werden, wobei $n \in \{1,...,N\}$ mit der Anzahl N der bereitgestellten Energieschwellen $S \to S_n$ im betrachteten Pixelelement 50 und wobei $m \in \{1,...,M\}$ mit der Anzahl M der bereitgestellten Energieschwellen $S \to S_m$ des zumindest einen weiteren Pixelelements 50 der Vielzahl an Pixelelementen, auf welchem die Koinzidenz-Zählsignale basieren. Bevorzugt werden mehrere Anzahlen $C \to C_{nm}$ an Koinzidenz-Zählsignalen in jedem Pixelelement 50 der Teilzahl an Pixelelementen 50 gezählt. Vorteilhaft erlaubt eine Mehrzahl an gezählten Anzahlen an Koinzidenz-Zählsignalen eine weitergehende, verbesserte Korrektur des Röntgenbilddatensatzes. Insbesondere bei Berücksichtigung sowohl symmetrischer als auch unsymmetrischer Überschreitungen kann außerdem auch eine verbesserte Ortsauflösung verbessert erreicht werden.

Es kann auch vorgesehen sein, dass in einem jeweiligen Pixelelement 50 der Teilzahl an Pixelelementen neben für das Zählen von Zählsignalen vorgesehene Komparatoren jeweils ein oder mehrere gesonderte Komparatoren für das Zählen von Koinzidenz-Zählsignalen 19 vorgesehen sind, basierend auf welchen nur Koinzidenz-Zählsignale gezählt werden.

Liegen neben Pixelelementen 50, welche Teil der Teilzahl der Vielzahl an Pixelelementen 50 sind, außerdem auch Pixelelemente 50 vor, welche lediglich zum Zählen von Zählsignalen ausgebildet sind, können diese insbesondere jeweils zumindest einen Signalverstärker 17, eine Anzahl an damit gekoppelten Komparatoren 19 und jeweils ein mit einem Signalausgang 7 eines Komparators 19 der Anzahl an Komparatoren 19 signaltechnisch verschaltetes Zählelement 13 aufweisen. Gegebenenfalls kann außerdem das Ausgangssignal zumindest eines Komparators 19 in ein Pixelelement 50 der Teilzahl der Vielzahl geführt werden, worauf basierend in dem Pixelelement 50 der Teilzahl der Vielzahl an Pixelelementen 50 ein Koinzidenz-Zählsignal gebildet werden kann.

In dem in Fig. 3 gezeigten Beispiel wird beim Zählen der Anzahlen C an Koinzidenz-Zählsignalen nicht zwischen den einzelnen weiteren verknüpften Pixelelementen 50 der Vielzahl an Pixelelementen 50 unterschieden. Das heißt, in diesem Beispiel basiert die im Schritt des zweiten Zählens V2 für jedes Pixelelement 50 der Teilzahl der Vielzahl an Pixelelementen 50 gezählte zumindest eine Anzahl C an Koinzidenz-Zählsignalen auf koinzidierend auftretenden Signalen in allen weiteren Pixelelementen 50. In anderen Worten, die jeweils gezählte Anzahl C an Koinzidenz-Zählsignalen in einem jeweiligen Zählelement 13 wird in dem gezeigten Beispiel dann um eine Zähleinheit erhöht, wenn zumindest in einem der weiteren verknüpften Pixelelementen 50 ein koinzidierend auftretendes Signal auftritt. In anderen Worten, die gezählte zumindest eine Anzahl C an Koinzidenz-Zählsignalen in dem gezeigten Beispiel repräsentiert eine Summe von koinzidierend auftretenden Signalen in allen weiteren verknüpften Pixelelementen 50. In anderen Ausführungen kann jedoch vorgesehen sein, dass für jedes der weiteren verknüpften Pixelelemente 50 jeweils eine Anzahl C an Koinzidenz-Zählsignalen gezählt wird. Das heißt, es kann vorgesehen sein, dass im Schritt des zweiten Zählens V2 jeweils zumindest eine Anzahl C an Koinzidenz-Zählsignalen mit jedem der weiteren Pixelelementen 50 gezählt wird.

In Fig. 4 ist eine exemplarische Verschaltung eines Pixelelements 50 zum Zählen von Zählsignalen bzw. Koinzidenz-Zählsignalen veranschaulicht, wobei in diesem Fall neben einer Anzahl $T \to T_1$ an Zählsignalen basierend auf einer Energieschwelle $S \to S_1$ und einer Anzahl $T \to T_2$ an Zählsignalen basierend auf einer Energieschwelle $S \to S_2$ des betrachteten Pixelelements 50 für jedes der, in dem gezeigten Beispiel vier, weiteren verknüpften Pixelelemente 50 (Index i = 1,...,4) jeweils Anzahlen $C \to C_{nm}^i$ an Koinzidenz-Zählsignalen gezählt werden, basierend auf den Energieschwellen $S \to S_n$ mit n = 1,2, des betrachteten Pixelelements 50 und den Energieschwellen $S \to S_m$ des jeweiligen verknüpften weiteren Pixelelements 50 mit Index i mit m = 1,2. Analog zu dem Beispiel in Fig. 3 gibt die Anzahl $C \to C_{nm}^i$ an Koinzidenz-Zählsignalen entsprechend die Anzahl an koinzidierend auftretenden Signalen wieder, welche im betrachteten Pixelelement 50 die Energieschwelle $S \to S_n$ und in dem weiteren Pixelelement 50 mit Index i die Energieschwelle $S \to S_m$ überschritten haben.

Die Übertragung auf eine andere Anzahl an bereitgestellten Komparatoren 19 mit jeweils einstellbaren Energieschwellen S und eine andere Zahl an weiteren verknüpften Pixelelementen 50 ist dabei leicht möglich.

Es kann in der Verschaltung der Pixelelemente 50 außerdem vorgesehen sein, dass eine redundante Erhebung von Koinzidenz-Zählsignalen vermieden wird. Dem liegt die Überlegung zugrunde, dass Koinzidenzen (pixel-)symmetrisch auftreten. So kann beispielsweise erwartet werden, dass eine Anzahl C an Koinzidenz-Zählsignalen, welche zwischen einem Pixelelement 50 mit Index p und einem Pixelelement 50 mit Index i erhoben wird, gleich der korrespondierenden Anzahl C an Koinzidenz-Zählsignalen ist, welche im Pixelelement 50 mit Index i mit dem Pixelelement p gezählt wurde. Beispielsweise kann angenommen werden, dass eine Anzahl $C \to {}^p C_{11}^i$ an Koinzidenz-Zählsignalen gleich einer Anzahl $C \to {}^i C_{11}^p$ an Koinzidenz-Zählsignalen ist. Beispielsweise kann angenommen werden, dass eine Anzahl $C \to {}^p C_{12}^i$ an Koinzidenz-Zählsignalen gleich einer Anzahl $C \to {}^i C_{21}^p$ an Koinzidenz-Zählsignalen ist. Für die Vervollständigung der Koinzidenzinformation in einem jeweiligen Pixelelement kann dann eine Übertragung von Anzahlen an Koinzidenz-Zählsignalen zwischen den Pixelelementen vorgesehen sein. Derart kann zumindest teilweise eine vereinfachte Verschaltung bereitgestellt werden.

Eine vorteilhafte Repräsentation der gezählten oder übertragenen Anzahlen C an Koinzidenz-Zählsignalen in einem Pixelelement 50 kann mittels einer Koinzidenzmatrix Ĉ bereitgestellt werden, welche jeweils die gezählte Anzahl bzw. die gezählten und/oder übertragenen Anzahlen C an Koinzidenz-Zählsignalen eines Pixelelements 50 beschreibt.

Ausgehend von der Annahme, dass wie in dem Beispiel in Fig. 4 dargestellt, für jedes mit dem betrachteten Pixelelement 50 verknüpfte, weitere Pixelelement 50 mit Index i Anzahlen C an Koinzidenz-Zählsignalen gezählt oder gegebenenfalls auch übertragen werden, d.h. dass eine pixelweise Koinzidenzinformation vorliegt, kann ein betrachtetes Pixelelement 50 eine dreidimensionale Koinzidenzmatrix C aufgestellt werden, wobei

$$\hat{C}(:,:,i) = \hat{C}_i = \begin{bmatrix} C_{11}^i & C_{12}^i & \dots & C_{1M}^i \\ C_{21}^i & C_{22}^i & \dots & C_{2M}^i \\ \vdots & \vdots & \ddots & \vdots \\ C_{N1}^i & C_{N2}^i & \dots & C_{NM}^i \end{bmatrix}$$

wobei i = 1, ..., I mit I = Anzahl an mit dem betrachteten Pixelelement 50 verknüpften weiteren Pixelelementen 50, basierend auf welchen Anzahlen an Koinzidenz-Zählsignale ermittelt werden, und N = Anzahl der Energieschwellen S in dem betrachteten Pixelelement 50 der Vielzahl an Pixelelementen 50 bzw. M = Anzahl der Energieschwellen S in dem weiteren Pixelelement mit Index i, basierend auf welchen Anzahlen C an Koinzidenz-Zählsignalen ermittelt werden.

Entsprechend zu der vorherigen Beschreibung entspricht die Anzahl $C \rightarrow C_{11}^i$ an Koinzidenz-Zählsignalen den Koinzidenzen der Energieschwelle $S \rightarrow S_1$ des betrachteten Pixelelements 50 mit der Energieschwelle $S \rightarrow S_1$ des weiteren Pixelelements mit Index i, die Anzahl $C \rightarrow C_{12}^i$ an Koinzidenz-Zählsignalen den Koinzidenzen der Energieschwelle $S \rightarrow S_1$ des betrachteten Pixelelements 50 mit der Energieschwelle $S \rightarrow S_2$ des weiteren Pixelelements mit Index i, die Anzahl $C \rightarrow C_{22}^i$ an Koinzidenz-Zählsignalen den Koinzidenzen der Energieschwelle $S \rightarrow S_2$ des betrachteten Pixelelements mit der Energieschwelle $S \rightarrow S_2$ des weiteren Pixelelements mit Index i, etc.

Je nach Ausgestaltung und Verschaltung der Pixelelemente 50 bestimmt sich die Dimension der Koinzidenzmatrix C. Ebenso kann die Koinzidenzmatrix C je nach Ausgestaltung und Verschaltung lediglich teilweise mit Einträgen basierend auf gezählten oder übertragenen Anzahlen an Koinzidenz-Zählsignalen befüllt sein.

Bezogen auf das konkrete Beispiel einer signaltechnischen Verschaltung in Fig. 4 resultiert beispielsweise eine dreidimensionale Koinzidenzmatrix C mit den Dimensionen 2x2x4, wobei

$$\hat{C}(:,:,i) = \hat{C}_i = \begin{bmatrix} C_{11}^i & C_{12}^i \\ C_{21}^i & C_{22}^i \end{bmatrix}$$

mit i = 1,...,4.

Wird beim Zählen der Koinzidenz-Zählsignale nicht zwischen den verknüpften weiteren Pixelelementen 50 unterschieden, kann die Koinzidenzmatrix C eines Pixelelements 50 durch eine zweidimensionale Matrix repräsentiert werden:

$$\hat{C} = \begin{bmatrix} C_{11} & C_{12} & \dots & C_{1M} \\ C_{21} & C_{22} & \dots & C_{2M} \\ \vdots & \vdots & \ddots & \vdots \\ C_{N1} & C_{N2} & \dots & C_{NM} \end{bmatrix}$$

wobei die einzelnen Einträge $C_{nm}$ jeweils auf koinzidierend auftretenden Signalen aller weiteren verknüpften Pixelelemente 50 basiert, d.h. der Summe an koinzidierend auftretenden Signalen in allen weiteren verknüpften Pixelelementen 50 entspricht.

Im Fall des in Fig. 3 dargestellten konkreten Beispiels reduziert sich diese zweidimensionale Matrix C für das betrachtete Pixelelement 50 auf:

$$\hat{C} = \begin{bmatrix} C_{11} & C_{12} \end{bmatrix}$$

Gleichermaßen kann auch aus einer dreidimensionalen Koinzidenzmatrix C eine zweidimensionale Koinzidenzmatrix C bestimmt werden, insofern dies für eine Weiterverarbeitung der Koinzidenzinformation vorteilhaft ist, wobei die Einträge der zweidimensionalen Koinzidenzmatrix C dann jeweils aus der Summe $C_{nm} = \sum_i C_{nm}^i$ bestimmt werden können.

Wie bereits im Rahmen von Fig. 2 beschrieben, kann die ermittelte Koinzidenzinformation auf unterschiedliche Weisen in das Erzeugen V3 des Röntgenbilddatensatzes eingebracht werden. Dabei kann bereits eine über die weiteren Pixelelemente 50 summierte Koinzidenzinformation, repräsentierbar durch eine zweidimensionale Koinzidenzmatrix, ausreichend für die Bereitstellung einer verbesserten Bildqualität des Röntgenbilddatensatzes sein. So kann die Anzahl der Koinzidenzen eines Pixelelements mit beliebigen Nachbarn bereits einen Großteil der Information tragen. Die Bereitstellung einer detaillierteren Koinzidenzinformation, repräsentierbar durch eine dreidimensionale Koinzidenzmatrix, erlaubt dagegen einen besonders vielfältigen Einsatz und insbesondere durch die Hinzunahme detaillierter Richtungsinformationen verbesserte Möglichkeiten eine verbesserte Ortsauflösung zu erreichen, wobei eine weniger detaillierte Koinzidenzinformation zumeist einen geringeren schaltungstechnischen Aufwand bedeutet.

Im Folgenden sollen mit Bezug auf das Verfahren zur Erzeugung eines Röntgenbilddatensatzes in Fig. 2 konkrete Ausführungsbeispiele (im Folgenden Variante 1 bis Variante 5) veranschaulicht werden, wie ermittelte Koinzidenzinformation in den Schritt des Erzeugen V3 des Röntgenbilddatensatz eingehen kann.

Für die weitere Veranschaulichung wird dabei davon ausgegangen, dass mit Hilfe einer geeigneten signaltechnischen Verschaltung der jeweiligen Pixelelemente 50 der Vielzahl an Pixelelementen 50 die jeweilige für die beispielhaft ausgeführte Anwendungsvariante notwendige Koinzidenzinformation für die Pixelelemente 50 der Vielzahl an Pixelelementen durch Zählen oder Übertragen ermittelt werden kann.

Ebenso wird für die folgenden Veranschaulichungen davon ausgegangen, dass eine Energieschwelle bezeichnet mit $S \to S_1$ einen niederenergetischeren Energieschwellwert annimmt als eine Energieschwelle bezeichnet mit $S \to S_2$. Weiterhin wird für die folgende Veranschaulichung davon ausgegangen, dass jedes der Pixelelemente 50 der Vielzahl an Pixelelementen 50 Energieschwellen S mit jeweils den gleichen Energieschwellwerten aufweist.

Diese exemplarischen Bedingungen dienen dabei lediglich der leichteren Veranschaulichung und besseren Verständlichkeit der beschriebenen Varianten. Davon ausgehend sind weitere, auch komplexere Ausführungsvarianten ableitbar. Es sind darüber hinaus auch beliebige Kombinationen der hier beschriebenen Varianten möglich.

Bevorzugt wird im Folgenden zumindest mit den vier direkt benachbarten Pixelelementen 50 eine oder mehrere Anzahlen C an Koinzidenz-Zählsignalen ermittelt. Noch bevorzugter werden zumindest mit den vier direkt benachbarten und den diagonal benachbarten Pixelelementen 50 Anzahlen C an Koinzidenz-Zählsignalen ermittelt.

Variante 1:

Gemäß einer Variante des Verfahrens wird in einem Schritt V31 des Vorverarbeitens in jedem Pixelelement 50 der Teilzahl der Vielzahl an Pixelelementen 50 die zumindest eine gezählte Anzahl T an Zählsignalen mittels der zumindest einen Anzahl C an Koinzidenz-Zählsignalen angepasst. Beispielsweise wird in dem Teilschritt V31 des Vorverarbeitens zumindest in jedem Pixelelement 50 der Teilzahl an Pixelelementen 50 die zumindest eine Anzahl C an Koinzidenz-Zählsignalen von der zumindest einen Anzahl T an gezählten Zählsignalen subtrahiert oder zu der zumindest einen Anzahl T an gezählten Zählsignalen addiert. Dies kann auch eine gewichtete Addition oder Subtraktion umfassen. Das Subtrahieren oder Addieren kann also dabei einen Faktor umfassen, so dass nur ein Anteil oder ein Mehrfaches der zumindest einen Anzahl C an Koinzidenz-Zählsignalen subtrahiert oder addiert wird. Gleichermaßen kann eine auf ein Pixelelement 50 der Vielzahl an Pixelelemente 50 übertragene zumindest eine Anzahl C an Koinzidenz-Zählsignalen von einer in dem Pixelelement 50 gezählten Anzahl an Zählsignalen subtrahiert oder zu der zumindest einen Anzahl an Zählsignalen addiert werden. Werden in einem jeweiligen Pixelelement 50 der Vielzahl an Pixelelementen 50 mehrere Anzahlen T an Zählsignalen und/oder mehrere Anzahlen C an Koinzidenz-Zählsignalen ermittelt, kann beispielsweise zumindest eine der Anzahlen C an Koinzidenz-Zählsignalen von zumindest einer der Anzahlen T an Zählsignalen addiert oder subtrahiert werden.

Ein besonders einfaches, nicht-beanspruchtes Beispiel kann dabei vorsehen, dass in jedem Pixelelement 50 der Vielzahl an Pixelelementen 50 von einer Anzahl $T \to T_1$ an Zählsignalen in Abhängigkeit einer Energieschwelle $S \to S_1$ ein Anteil einer Anzahl $C \to C_{11}$ an Koinzidenz-Zählsignalen subtrahiert wird. Dabei kann die Anzahl $C \to C_{11}$ an Koinzidenz-Zählsignalen insbesondere der Summe aller verknüpften weiteren I Pixelelemente 50 entsprechen. Dabei wird angenommen, dass koinzidierend auftretende Signale, welche sowohl in dem betrachteten Pixelelement 50 der Vielzahl an Pixelelementen 50 sowie zumindest in einem weiteren verknüpften Pixelelement 50 die Energieschwelle $S \to S_1$ überschritten haben, nur anteilig dem betrachteten Pixelelement selbst zuzuordnen sind. Beispielsweise kann angenommen werden, dass koinzidierend auftretende Signale nur zur Hälfte dem jeweiligen Pixelelement selbst zuzuordnen sind, so dass eine korrigierte Anzahl $T'_1$ an Zählsignalen in jedem Pixelelement 50 der Vielzahl an Pixelelementen 50 gegeben werden kann durch:

$$T'_1 = T_1 - \frac{1}{2} * C_{11}$$

Neben dem Faktor $\frac{1}{2}$ kann auch ein anderer Faktor mit anderen Annahmen angewendet werden.

Dabei kann die Anzahl $T \to T_1$ an Zählsignalen bereits vorverarbeitet sein. Beispielsweise kann die Korrektur auf bereits linearisierten oder logarithmierten Daten erfolgen.

In einer weiteren Ausführungsform dieser Variante kann die Anzahl $C \to C_{11}$ an Koinzidenz-Zählsignalen auch genutzt werden, um eine Anzahl $T \to T_2$ an Zählsignalen basierend auf einer zweiten Energieschwelle $S \to S_2$ zu korrigieren. Beispielsweise kann die Anzahl $T \to T_2$ an Zählsignalen ebenso durch Addition bzw. Subtraktion eines Anteils einer Anzahl $C \to C_{11}$ an Koinzidenz-Zählsignalen angepasst werden. Dabei kann angenommen werden, dass zumindest ein Anteil der koinzidierend aufgetretenen Signale, welche sowohl im dem betrachteten Pixelelement 50 als auch in zumindest einem weiteren Pixelelement 50 die Energieschwelle $S \to S_1$ überschritten haben, wenn es keine Aufteilung des Signals auf zumindest zwei Pixelelemente 50 gegeben hätte, eigentlich einer höheren Energieschwelle S zuzuordnen wären. Eine korrigierte Anzahl $T_2$ an Zählsignalen in einem Pixelelement 50 kann dann beispielsweise gegeben sein durch

$$T'_2 = T_2 + a * C_{11}$$

Der Parameter a kann beispielsweise empirisch oder mittels Simulationen festgelegt werden. Der Parameter a kann insbesondere vom Energiespektrums der eintreffenden Röntgenstrahlung und/oder von dem Energieschwellwert der einstellbaren Energieschwellen abhängen und festgelegt werden.

In einer weiteren Ausführungsform dieser Variante kann vorgesehen sein, dass in einem jeweiligen Pixelelement 50 der von einer Anzahl $T \to T_1$ an Zählsignalen in Abhängigkeit einer Energieschwelle $S \to S_1$ zumindest ein Anteil einer Anzahl $C \to C_{12}$ an Koinzidenz-Zählsignalen abgezogen wird. Das heißt, in diesem Fall werden gezielt diejenigen Fälle von der Anzahl $T \to T_1$ basierend auf der Energieschwelle $S \to S_1$ abgezogen, bei welchen im jeweiligen Pixelelement 50 lediglich die Energieschwelle $S \to S_1$ überschritten wurde, in einem weiteren Pixelelement 50 jedoch die Energieschwelle $S \to S_2$. Dies kann als Hinweis gewertet werden, dass im weiteren Pixelelement der Großteil der Energie eines eintreffenden Röntgenphotons deponiert wurde und damit der Treffer mit hoher Wahrscheinlichkeit im weiteren Pixelelement 50 und nicht im betrachteten Pixelelement 50 stattgefunden hat. Eine korrigierte Anzahl $T_1$ an Zählsignalen in einem Pixelelement 50 kann dann beispielsweise gegeben sein durch:

$$T'_1 = T_1 - b * C_{12}$$

Der Parameter b kann in einer einfachsten Variante beispielsweise als b = 1 gewählt werden. Er kann jedoch auch anderweitig gewählt sein. Der Parameter b kann beispielsweise empirisch oder mittels Simulationen festgelegt werden.

In einer weiteren Ausführungsform dieser Variante kann vorgesehen sein, dass sowohl eine Anzahl $C \to C_{11}$ als auch eine Anzahl $C \to C_{12}$ an Koinzidenz-Zählsignalen für eine Korrektur der Anzahl $T \to T_1$ herangezogen wird. Durch eine Kombination der vorherig beschriebenen Ausführungsformen kann dann beispielsweise eine korrigierte Anzahl $T_1$ an Zählsignalen in einem Pixelelement 50 gegeben sein durch

$$T'_1 = T_1 - C_{12} - 0.5 * (C_{11} - C_{12}) = T_1 - 0.5 * (C_{11} + C_{12})$$

, wobei eine doppelte Subtraktion der Anzahl $C \to C_{12}$ an Koinzidenz-Zählsignalen vermieden wird.

In den letzten beiden Fällen geht damit erfindungsgemäß zumindest eine Anzahl C an Koinzidenz-Zählsignalen ein, wobei die einstellbare Energieschwelle S des zumindest einen Komparators 19 der Anzahl an Komparatoren des jeweiligen Pixelelements 50 und die einstellbare Energieschwelle S des zumindest einen Komparators 19 der Anzahl an Komparatoren des zumindest einen weiteren Pixelelements 50 der Vielzahl an Pixelelementen 50, auf welchen die Koinzidenz-Zählsignale basieren, unterschiedliche Energieschwellwerte aufweisen. Das heißt, die Koinzidenz-Zählsignale basierend auf einer unsymmetrischen Überschreitung der Energieschwellen S. In anderen Worten es geht zumindest eine Anzahl C an Koinzidenz-Zählsignalen basierend auf unterschiedlichen Energieschwellen S ein. Diese Anzahlen entsprechen insbesondere den Nebendiagonaleinträgen einer zuvor beschriebenen Koinzidenzmatrix C. Im letzten Fall geht insbesondere sowohl zumindest eine Anzahl C an Koinzidenz-Zählsignalen basierend auf unterschiedlichen Energieschwellen S als auch zumindest eine Anzahl C an Koinzidenz-Zählsignalen basierend auf gleichen Energieschwellen S ein. Daraus resultiert vorteilhaft, dass zusätzlich oder alternativ zu einer Korrektur der Rauscherhöhung, für zumindest einen Teil der Koinzidenzen auch zumindest teilweise eine Zuordnung von Doppelzählungen in das richtige Pixelelement erreicht werden kann. Ohne die Koinzidenz-Zählsignale basierend auf einer unsymmetrischen Überschreitung der Energieschwellen S, d.h. die Nebendiagonaleinträge der Koinzidenzmatrix C, wäre eine solche Korrektur nicht möglich.

Abgesehen von den hier konkret aufgezeigten Beispielen für eine Korrektur der Anzahlen $T \to T_1$ und $T \to T_2$ an Zählsignalen basierend auf der Energieschwelle $S \to S_1$ bzw. $S \to S_2$ können ebenso auch weitere Anzahlen T basierend auf weiteren Energieschwellen S in übertragbarer Weise korrigiert werden. Vorteilhaft kann ein verbesserter Röntgenbilddatensatz basierend auf der Koinzidenzinformation erzeugt werden.

Variante 2:

Eine weitere Ausgestaltungsvariante des Verfahrens zur Erzeugung des Röntgenbilddatensatzes sieht vor, dass der Schritt des Erzeugens V3 das Anwenden einer trainierten Funktion umfasst, wobei die zumindest eine Anzahl C an Koinzidenz-Zählsignalen in zumindest einem Pixelelement 50 der Teilzahl an Pixelelementen 50 als Eingabeparameter in die trainierte Funktion eingeht. Basierend darauf kann im Schritt des Vorverarbeitens V31 beispielsweise eine Korrektur der zumindest einen Anzahl T an Zählsignalen durchgeführt werden, welche auch komplexere Zusammenhänge zwischen der zumindest einen Anzahl C an Koinzidenz-Zählsignalen und der zumindest einen Anzahl T an Zählsignalen und/oder auch weitere Einflussgrößen auf die gezählten Anzahlen berücksichtigt. Weitere Einflussgrößen können beispielweise Temperatureffekte oder zeit- oder strahlungsabhängige Drifteffekte oder ähnliches umfassen. Werden mehrere Anzahlen C an Koinzidenz-Zählsignalen in einem jeweiligen Pixelelement 50 ermittelt, können entsprechend auch die mehreren Anzahlen C an Koinzidenz-Zählsignalen als Eingangsparameter in die trainierte Funktion eingehen.

Variante 3:

Eine weitere Ausgestaltungsvariante des Verfahrens zur Erzeugung des Röntgenbilddatensatzes sieht vor, dass die zumindest eine Anzahl C an Koinzidenz-Zählsignalen in einen iterativen Rekonstruktionsalgorithmus eingeht. Die Koinzidenzinformation kann in der iterativen Rekonstruktion genutzt werden, um eine verbesserte Bildqualität, beispielsweise ein verringertes Rauschen, zu erreichen. Beispielsweise kann die Koinzidenzinformation bei der Vorwärtsprojektion in einer modellbasierten iterativen Rekonstruktion eingesetzt werden. Es kann außerdem eine verbesserte Ortsauflösung erreicht werden, indem insbesondere eine pixelweise Koinzidenzinformation, d.h. eine Unterscheidung der jeweils an einem Koinzidenzsignal beteiligten Pixelelemente, eingesetzt wird. Dadurch kann eine Richtungsinformation der Koinzidenzen miteinbezogen werden. Derart kann beispielsweise im Rahmen der Rekonstruktion eine Übertragung der gesammelten Information auf ein feineres Subpixelrasters, ähnlich wie in der folgend beschriebenen Variante 5, ermöglicht werden, welche im Rahmen der iterativen Rekonstruktion eingesetzt werden kann.

Variante 4:

Eine weitere Ausgestaltungsvariante des Verfahrens zur Erzeugung des Röntgenbilddatensatzes sieht vor, dass der Schritt V3 des Erzeugens umfasst, dass basierend auf der in dem Pixelelementen 50 gezählten zumindest einen Anzahl T an Zählsignalen zumindest ein vorläufiger Bilddatensatz erzeugt wird und basierend auf der in den Pixelelementen 50 der Teilzahl der Vielzahl an Pixelelementen 50 gezählten zumindest einen Anzahl C an Koinzidenz-Zählsignalen zumindest ein Koinzidenz-Bilddatensatz erzeugt wird. In einem Teilschritt V33 des Nachverarbeitens kann dann der zumindest eine Koinzidenz-Bilddatensatz auf den zumindest einen vorläufigen Bilddatensatz angewendet werden. In diesem Fall kann die Degradation der Bildqualität durch auftretende Koinzidenzen durch Korrekturen auf Basis rekonstruierter Bilddaten anstelle von Korrekturen auf Basis gemessener Rohdaten, d.h. der Anzahlen T an Zählsignalen, reduziert werden. In einer einfachen Form kann das Anwenden des zumindest einen Koinzidenz-Bilddatensatzes einer einfachen Linearkombination der rekonstruierten Bilddatensätze, d.h. des Koinzidenz-Bilddatensatzes und des vorläufigen Röntgenbilddatensatzes, umfassen. Es kann auch eine gewichtete Linearkombination umfassen. Ebenso ist beispielsweise eine Kombination des zumindest einen Koinzidenz-Bilddatensatzes und des zumindest einen vorläufigen Bilddatensatzes basierend auf einer polynomiellen Funktion oder ein anderweitiges Anwenden möglich.

Werden in den Pixelelementen 50 mehrere Anzahlen C an Koinzidenz-Zählsignalen ermittelt, können dabei auch mehrere Koinzidenz-Bilddatensätze erzeugt werden, welche auf einen oder mehreren vorläufigen Bilddatensätzen, basierend auf in jedem Pixelelement 50 der Vielzahl an Pixelelementen gezählten Zählsignalen, angewendet werden können. Liegt etwa die Koinzidenzinformation repräsentierbar durch eine dreidimensionale oder zweidimensionale Koinzidenzmatrix C vor, kann der komplette Tensor $\hat{C}$, als auch einzelne Bestandteile wie etwa Teilmatrizen $\hat{C}_i$ oder jeweils Einträge $C_{nm}^i$ bzw. $C_{nm}$ zu Koinzidenz-Bilddaten verarbeitet werden, welche in einem Schritt des Nachverarbeitens zur Verbesserung der Bildqualität des Röntgenbilddatensatzes auf einen vorläufigen Bilddatensatz angewendet werden können. Auch hier kann beispielsweise eine deutlich verbesserte Ortsauflösung erreicht werden, indem eine pixelweise Koinzidenzinformation eingesetzt wird, um auf Basis der rekonstruierten Bilddaten ähnlich wie in nachfolgend beschriebener Variante 5 eine Übertragung auf ein feineres Voxel- oder Bildpixelraster zu erreichen, d.h. die aus den Anzahlen an Zählsignalen und Anzahlen an Koinzidenz-Zählsignalen berechneten Bildwerten der Voxel oder Bildpixel auf neue (kleinere) Voxel oder Bildpixel zu übertragen.

Variante 5

Eine weitere Ausgestaltungsvariante des Verfahrens zur Erzeugung des Röntgenbilddatensatzes sieht vor, die gemessene Koinzidenzinformation zu nutzen, um die Messdaten der Pixelelemente verbessert auf ein feineres Subpixelraster zu übertragen. Damit kann vorteilhaft eine verbesserte Ortsauflösung im Röntgenbilddatensatz ermöglicht werden. In dieser Variante des Verfahrens wird dabei davon ausgegangen, dass insbesondere eine pixelweise Koinzidenzinformation vorliegt, d.h. dass für jedes der weiteren für die Bildung der Koinzidenz-Zählsignale verknüpften Pixelelementen 50 einzeln zumindest eine Anzahl an Koinzidenz-Zählsignalen gezählt wird oder übertragen wird und damit einer Richtungsinformation über registrierten Koinzidenzen vorliegt.

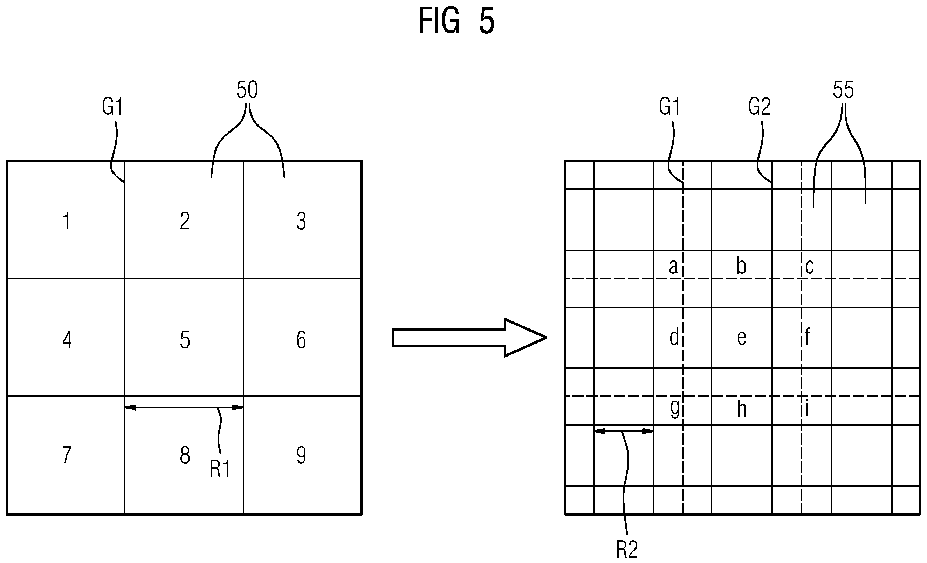

In dieser Ausgestaltungsvariante des Verfahrens wird, wie in Fig. 5 veranschaulicht, basierend auf einem durch die Anordnung der Vielzahl an Pixelelemente 50 im Röntgendetektor 1 definierten ersten matrixartigen Pixelraster G1, wie auf der linken Seite veranschaulicht, ein Subpixelraster G2 an mit den Pixelelementen 50 der Vielzahl an Pixelelementen 50 überlappenden Subpixeln 55, wie auf der rechten Seite in Fig. 5 veranschaulicht, definiert. Im gezeigten Beispiel sind exemplarisch und ausschnitthaft 9 ursprüngliche Pixelelemente 50 der Vielzahl an Pixelelementen 50 mit Indices 1 bis 9, welche das Pixelraster G1 aufstellen, dargestellt. Die neun Pixelelemente 50 werden durch ein zentrales Pixelelement 50 der Vielzahl an Pixelelementen 50 und seinen vier direkt benachbarten und seinen vier diagonal benachbarten Pixelelementen 50 gebildet.

Das Subpixelraster G2 weist dabei zumindest entlang einer Rasterdimension einen relativ zum Pixelraster G1 reduzierten Rasterabstand R2 auf. In der Figur weist das erste Pixelraster G1 beispielsweise einen ersten Rasterabstand R1 in der Horizontalen auf und das Subpixelraster G2 einen relativ dazu geringeren Rasterabstand R2. Die Subpixel können, wie hier dargestellt, müssen jedoch nicht, eine quadratische und regelmäßige Pixelfläche aufweisen. Ebenso kann der Rasterabstand R2 und/oder die Pixelfläche des Subpixelrasters G2 lokal innerhalb des Subpixelrasters G2 variieren.

Die Subpixel 55 des Subpixelrasters G1 überlappen mit den ursprünglichen Pixelelementen 50 im Pixelraster G2. Das Subpixelraster G2 kann wie in diesem Beispiel derart definiert sein, dass zumindest teilweise einzelne Subpixel 55 mit zumindest zwei ursprünglichen Pixelelementen 50 überlappen. Im gezeigten Beispiel überlappen beispielsweise die Subpixel mit Indices b,d,h,f jeweils mit zwei Pixelelementen 50 im Pixelraster G1, die Subpixel mit Indices a,c,g,i jeweils mit vier ursprünglichen Pixelelementen 50 im Pixelraster G1 und das Subpixel mit Index e lediglich mit einem ursprünglichen Pixelelementen 50 im Pixelraster G1.

In einem weiteren Schritt dieser Ausgestaltungsvariante des Verfahrens zur Erzeugung eines Röntgenbilddatensatzes wird jedem Subpixel 55 des Subpixelrasters G2 zumindest eine virtuelle Anzahl Z an Zählsignalen zugeordnet. Dabei basiert zumindest für einen Teil der Subpixel 55 die jeweilige zumindest eine virtuelle Anzahl Z auf zumindest einer gezählten oder übertragenen Anzahl C an Koinzidenz-Zählsignalen eines mit dem jeweiligen Subpixel 55 überlappenden Pixelelements 50. Insbesondere kann die einem jeweiligen Subpixel 55 zugeordnete virtuelle Anzahl Z an Zählsignalen dabei abhängig sein von der relativen Position des virtuellen Subpixels 55 zum zumindest einen überlappenden, ursprünglichen Pixelelement 50 der Vielzahl an Pixelelementen 50.

Anschließend wird der Röntgenbilddatensatz basierend auf der den virtuellen Subpixeln 5 jeweils zugeordneten virtuellen Anzahl Z an Zählsignalen erzeugt.

Beispielsweise kann die zumindest eine virtuelle Anzahl Z an Zählsignalen, welche einem Subpixel 55 mit Index q zugeordnet wird, in Abhängigkeit von einer Energieschwelle $S \to S_j$ beispielsweise auf einer gewichteten Summe aus den Anzahlen T an Zählsignalen und/oder den Anzahlen C an Koinzidenz-Zählsignalen der jeweils überlappenden ursprünglichen Pixelelemente im ursprünglichen Pixelraster G1 berechnet werden. Eine Formel zur Berechnung der zuzuordnenden virtuellen Anzahlen Z an Zählsignalen für ein Subpixel 55 mit Index q hinsichtlich der Energieschwelle $S \to S_j$ kann beispielsweise folgendermaßen bereitgestellt werden:

$$Z_j^q = \sum_p \left( \sum_i a_{ip} T_i^p + \sum_{k,n,m} b_{knmp}\, {}_{\Box}^p C_{nm}^k \right)$$

$Z_j^q$ beschreibt dabei die virtuelle Anzahl Z im Subpixel 55 mit Index q, welche der Energieschwelle $S \to S_j$ zugeordnet werden kann. $T_i^p$ beschreibt die jeweils gemessene Anzahl T an Zählsignalen in Abhängigkeit der Energieschwelle $S \to S_i$ des ursprünglichen Pixelelements 50 mit Index p. $a_{ip}$ und $b_{kmp}$ beschreiben Gewichtungsfaktoren. ${}_{\Box}^p C_{nm}^k$ beschreibt die jeweilige Anzahl $C \to {}_{\Box}^p C_{nm}^k$ an Koinzidenz-Zählsignalen des ursprünglichen Pixelelements 50 mit Index p in Energieschwelle $S \to S_n$ mit dem jeweiligen weiteren Pixelelement 50 mit Index $k$ in dessen Energieschwelle $S \to S_m$.

Auf die Repräsentation der Koinzidenzinformation mittels einer Koinzidenzmatrix $\hat{C}$ übertragen, entsprechen die $^p_{...}C^k_{nm}$ den Einträgen der Koinzidenzmatrix $\hat{C}$ des Pixelelements 50 mit Index p mit dem verknüpften weiteren Pixelelement 50 mit Index k und in Abhängigkeit der jeweiligen beteiligten Energieschwellen S des Pixelelements 50 mit Index p und des verknüpften weiteren Pixelelement 50 mit Index k. Die hier eingehende Anzahl $T \rightarrow T^p_i$ kann dabei einer bereits vorverarbeiten Anzahl T, z.B. nach einer Linearisierung, entsprechen.

Zur Veranschaulichung wird im Folgenden ein konkretes Beispiel anhand des Pixelrasters G1 und des Subpixelrasters G1 gemäß Fig. 5 beleuchtet. Zur besseren Verständlichkeit und zur Betonung ihrer Bedeutung als relative Angabe zum gerade betrachteten Pixelelement 50 mit Index p, wird dazu die Art der Koinzidenz k, d.h. im Wesentlichen das weitere verknüpfte Pixelelement k, auf welchem die Koinzidenz-Zählsignale basieren, mit einer relativen geographischen Angabe angegeben. Das heißt, ein Index N für "north" (Norden), ein Index NE für "northeast" (Nordost), ein Index E für "east" (Osten) etc. Ausgehend vom Pixelelement 50 mit Index 5 in Fig. 5 entspräche der Index N dem Pixelelement mit Index 2, der Index E dem Index 6, der Index NE dem Index usw.

Für das konkrete Beispiel wird hier ein Röntgendetektor 1 mit einer Vielzahl an Pixelelementen 50 mit jeweils zwei Komparatoren 19 aufweisend jeweils eine Energieschwelle S angenommen. Außerdem wird angenommen, dass die Koinzidenzinformation repräsentierbar durch eine vollständig gefüllte 2x2x8 Koinzidenzmatrix $\hat{C}$ für jedes Pixelelement 50 der Vielzahl an Pixelelementen 50 mit seinen direkt benachbarten und diagonal benachbarten Pixelelementen 50 vorliegt.

Der Beitrag des zentralen Pixelelements 50 mit Index 5 in Fig. 5 zu den virtuellen Anzahlen Z der Subpixel 55 mit Indices a-i der Fig. 5 kann dann beispielsweise folgendermaßen berechnet werden:

$$Z^a_1 = \frac{1}{2}C^{NW}_{11}, \ Z^a_2 = \frac{1}{2}\left(C^{NW}_{21} + C^{NW}_{12} - C^{NW}_{22}\right)$$

$$Z^b_1 = \frac{1}{2}C^N_{11}, \ Z^b_2 = \frac{1}{2}\left(C^N_{21} + C^N_{12} - C^N_{22}\right)$$

$$Z^c_1 = \frac{1}{2}C^{NE}_{11}, \ Z^c_2 = \frac{1}{2}\left(C^{NE}_{21} + C^{NE}_{12} - C^{NE}_{22}\right)$$

$$Z^d_1 = \frac{1}{2}C^W_{11}, \ Z^d_2 = \frac{1}{2}\left(C^W_{21} + C^W_{12} - C^W_{22}\right)$$

$$Z^f_1 = \frac{1}{2}C^E_{11}, \ Z^f_2 = \frac{1}{2}\left(C^E_{21} + C^E_{12} - C^E_{22}\right)$$

$$Z^g_1 = \frac{1}{2}C^{SW}_{11}, \ Z^g_2 = \frac{1}{2}\left(C^{SW}_{21} + C^{SW}_{12} - C^{SW}_{22}\right)$$

$$Z^h_1 = \frac{1}{2}C^S_{11}, \ Z^h_2 = \frac{1}{2}\left(C^S_{21} + C^S_{12} - C^S_{22}\right)$$

$$Z^i_1 = \frac{1}{2}C^{SE}_{11}, \ Z^i_2 = \frac{1}{2}\left(C^{SE}_{21} + C^{SE}_{12} - C^{SE}_{22}\right)$$

$$Z^e_1 = T^5_1 - \sum_{k \in NW,N,NW,W,E,SW,S,SE} C^k_{11},$$

$$Z^e_2 = T^5_2 - \sum_{k \in NW,N,NW,W,E,SW,S,SE} \left(C^k_{12} + C^k_{21} - C^k_{22}\right)$$

Diese Berechnung wird für jedes der ursprünglichen Pixelelemente 50 der Vielzahl an Pixelelementen 50 durchgeführt und die erhaltenen, virtuellen Anzahlen Z zu eventuell bereits vorhandenen virtuellen Anzahl Z in den Subpixeln addiert. Der Faktor $\frac{1}{2}$ für alle Subpixel 55 mit Indices a bis i, außer für das Subpixel 55 mit Index e, korrigiert die Tatsache, dass alle Koinzidenzen jeweils in zwei Pixelelementen 50 des Pixelrasters G1 gezählt werden und somit bei einer Übertragung der Anzahlen auf die Subpixel ansonsten doppelt gezählt werden würden.

Aus einer derartigen Berechnung folgt, dass alle Photonen, welche das Pixelelement mit Index 5 im Pixelraster G1 zentral treffen (und damit mit hoher Wahrscheinlichkeit keine koinzidierenden Signale in weiteren Pixelelementen 50 erzeugen), entsprechend in Subpixel 55 mit Index e gezählt werden, und alle Koinzidenzevents in demjenigen Subpixel 55, welcher zwischen den beiden beteiligten ursprünglichen Pixelelementen 50 liegt.

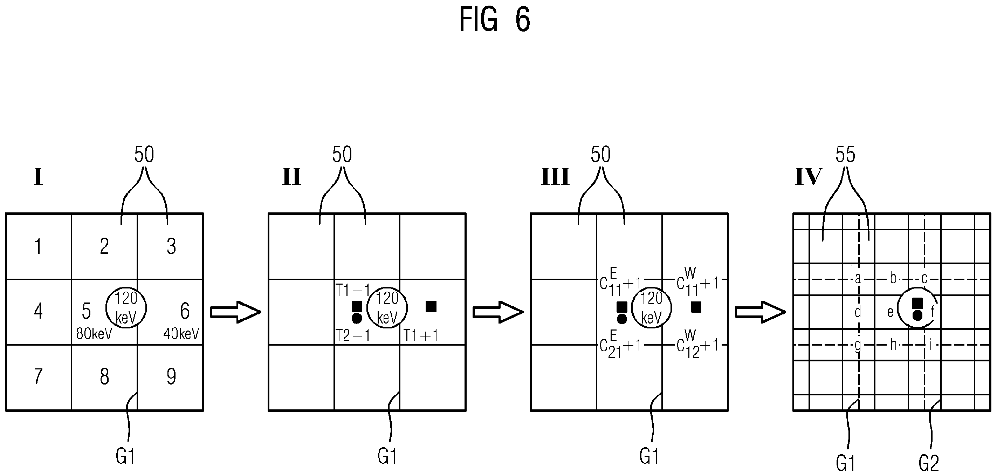

Die Auswirkung der zuvor beschriebenen Methode auf die Ortsauflösung kann anhand der Fig. 6 veranschaulicht werden. Trifft beispielsweise ein 120keV Photon das Pixelelement 50 mit Index 5 in Fig. 6 an seinem rechten Rand (wie in Teilansicht I auf der linken Seite dargestellt), so kann sich die im Konverterelement 3 erzeugte Ladung zwischen den Pixelelementen 50 mit Index 5 und mit Index 6 aufteilen. In dem in Fig. 6 gezeigten Beispiel wird beispielhaft 80keV im Pixelelement mit Index 5 deponiert und 40keV im Pixelelement mit Index 6. In dem gezeigten Beispiel, wie in Teilansicht II der Fig. 6 dargestellt, resultiert dies zu je einem Zählsignal in Abhängigkeit der Energieschwelle $S \to S_1$ der Pixelelemente mit Index 5 und Index 6, sowie zu einem Zählsignal in Abhängigkeit der Energieschwelle $S \to S_2$ des Pixelelements mit Index 5. Die Energieschwelle $S \to S_1$ weist dabei beispielsweise einen Energieschwellwert von 30keV auf und die Energieschwelle $S \to S_2$ einen Energieschwellwert von 60keV. Das heißt, die Anzahl $T \to T_1$ an Zählsignalen basierend auf Energieschwelle $S \to S_1$ erhöht sich in dem Pixelelement mit Index 5 und in dem Pixelelement mit Index 6 jeweils um eins, die Anzahl $T \to T_2$ basierend auf Energieschwelle $S \to S_2$ erhöht sich außerdem im Pixelelement mit Index 5 ebenfalls um eine Zähleinheit (in der Figur jeweils durch $T_1 + 1$ bzw. $T_2 + 1$ angedeutet). Zur besseren Sichtbarkeit soll in den folgenden Teilansichten III und IV der Fig. 6 die Erhöhung der Anzahl $T \to T_1$ jeweils durch das eingezeichnete Quadrat und die Erhöhung der Anzahl $T \to T_2$ jeweils durch den eingezeichneten Kreis visualisiert sein.

Gleichzeitig erzeugt das Röntgenphoton Koinzidenz-Zählsignale in den Pixelelementen mit Indices 5 und 6. Das Ereignis führt insbesondere dazu, dass im Pixelelement mit Index 5 sowohl die Anzahl $C \to C_{11}^E$ als auch die Anzahl $C \to C_{21}^E$ an Koinzidenz-Zählsignalen um eine Zähleinheit erhöht wird und in dem Pixelelement mit Index 6 entsprechend sowohl die Anzahl $C \to C_{11}^W$ als auch die Anzahl $C \to C_{12}^W$ an Koinzidenz-Zählsignalen um eine Zähleinheit erhöht wird (in der Figur jeweils durch $C_{nm}^E + 1$ bzw. $C_{nm}^W + 1$ angedeutet).

Nach Übergang zu dem Subpixelraster G2 in Teilansicht IV in Fig. 6 und der Berechnung der zugeordneten virtuellen Anzahlen Z an Zählsignalen gemäß den obig beschriebenen Formeln ergibt sich jeweils nur noch je ein einziges Zählsignal in beiden Energieschwellen $S \to S_1$ und $S \to S_2$ im neuen Subpixel mit Index f. Dies entspricht einer verbesserten Lokalisation des ursprünglichen Orts des eintreffenden Röntgenphotons.

Zur Veranschaulichung der grundsätzlichen Funktionsweise der Verfahrensvariante zum Erzeugen eines Röntgenbilddatensatzes wurde das Beispiel in Fig. 6 auf ein einzelnes Röntgenphoton reduziert. Das Verfahren arbeitet jedoch bevorzugt nicht eventweise, sondern nachträglich auf der aufgenommenen Koinzidenzinformation, welche einem Integral bzw. einer Summe der gebildeten Signale über ein Belichtungszeitfensters oder Auslesezeitfensters entspricht. Die Lage eines Objekts relativ zu den Pixelraster zeigt sich dabei insbesondere als Muster in den Häufigkeiten der Koinzidenz-Zählsignale. Im feineren Subpixelraster kann diese zusätzliche Information als höhere Auflösung nutzbar gemacht werden. Vorteilhaft kann eine verbesserte Ortsauflösung im Röntgenbilddatensatz erreicht werden.

Zur Vereinfachung wurden 3-fach und höhere Koinzidenzen vernachlässigt, was jedoch in einfacher Weise in das Konzept integrierbar ist. Ebenso kann eine derartige Berechnung auch auf andere Ausgestaltungsvarianten, beispielsweise anderweitig ausgestaltete Subpixelraster und/oder eine andere Anzahl an Energieschwellen und/oder eine andere Anzahl an jeweils verknüpften weiteren Pixelelementen übertragen werden.

Neben dem in Fig. 5 und 6 gezeigten, regelmäßigen Subpixelraster G2 sind auch weitere Varianten des Subpixelrasters G2 möglich. Ein regelmäßiges Subpixelraster stellt eine besonders zweckmäßige und einfache Variante des Verfahrens dar, welche außerdem auf übliche, routinemäßig eingesetzte Bildrekonstruktions- und Bildverarbeitungsalgorithmen in einfacher Weise zurückgreifen kann.

Es kann jedoch auch anderweitige Raster geben. Beispielsweise findet lediglich in eine Rasterdimension ein feineres Sampling, beispielsweise eine Unterteilung der ursprünglichen Pixelelemente 50, statt. Beispielsweise variieren die Rasterabstände des Subpixelrasters entlang zumindest einer Rasterdimension. Das definierte Subpixelraster kann insbesondere auf der zur Verfügung stehenden Koinzidenzinformation in den Pixelelementen der Vielzahl an Pixelelementen basieren. Wird beispielsweise lediglich in Richtung einer Rasterdimension Koinzidenzinformation zwischen den Pixelelementen erhoben, so ist ein feineres Subpixelraster entlang dieser Dimension besonders vorteilhaft umzusetzen.

Steht nicht für jedes Pixelelement der Vielzahl an Pixelelementen Koinzidenzinformation zur Verfügung kann in diesen Pixelelementen lokal auch auf eine Unterteilung oder ein feineres Subpixelraster verzichtet werden.

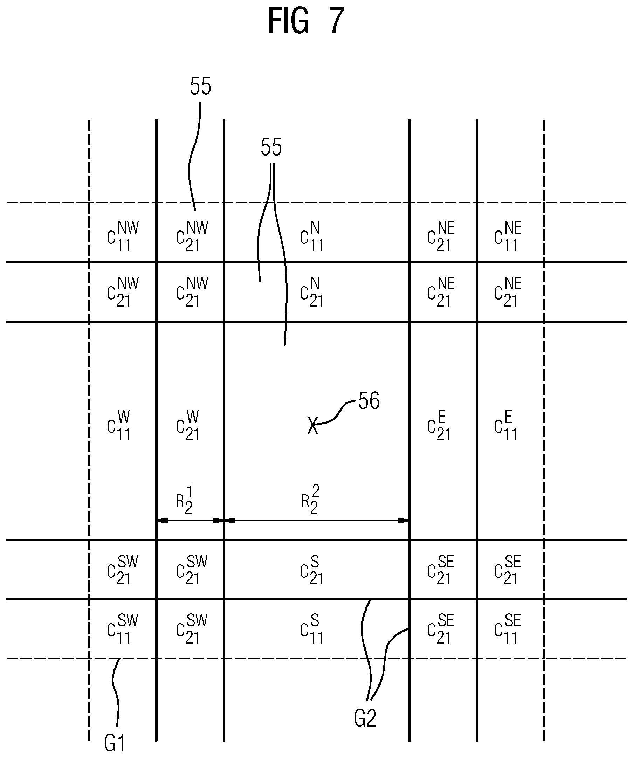

Es kann außerdem beispielsweise vorteilhaft sein die weitere, zusätzliche Koinzidenzinformation aus mehreren Energieschwellen S zu nutzen, um eine weiter verbesserte Lokalisation des Ursprungs koinzidierend auftretender Signale zu ermöglichen. Dafür kann in dem Verfahren vorgesehen sein, basierend auf dem Pixelraster G1 ein Subpixelraster G2 zu definieren, welches, wie in Fig. 7 exemplarisch veranschaulicht, Koinzidenzen, bei denen im Pixelelement eine höhere Energieschwelle S als im jeweiligen benachbarten Pixelelement ausgelöst hat, auch näher am Pixelzentrum 56 des betrachteten Pixelelements 50 zu lokalisieren.

In Fig. 7 ist ein exemplarisches Subpixelraster G2 gezeigt, welches das Pixelraster G1 in ein feineres Raster unterteilt und wobei die zusätzliche Koinzidenzinformation aus mehreren Energieschwellen S genutzt wird, eine weiter verbesserte Lokalisation der koinzidierend auftretenden Signale zu erreichen. Es sind dabei abweichend von der gezeigten Variante auch andere Ausführungsvarianten des Subpixelrasters möglich. Das in Fig. 7 exemplarisch gezeigte Subpixelraster G1 weist ein nichtäquidistantes Sampling auf, d.h. es weist variierende Rasterabstände auf. Insbesondere weisen die durch das in Fig. 7 gezeigte Subpixelraster G2 definierten Subpixel 55 im Gegensatz zu den Subpixel 55 in den Fig. 5 und 6 unterschiedliche Flächen auf.

In Fig. 7 ist außerdem der Beitrag der jeweiligen gezählten Anzahlen an Koinzidenz-Zählsignalen zu den virtuellen Anzahlen an Zählsignalen in den Subpixeln 55 für das exemplarische Subpixelraster G2 verdeutlicht. Die Anzahlen $C \rightarrow C_{nm}^k$ an Koinzidenz-Zählsignalen tragen dabei in Abhängigkeit von der relativen Position und insbesondere in Abhängigkeit des Abstands des virtuellen Subpixels 55 zum Zentrum 56 des in dem von den Pixelelementen 50 definierten Pixelraster G1 überlappenden Pixelelement 50 bei. In dem in Fig. 7 gezeigten Beispiel werden z.B. Koinzidenz-Zählsignale, welche zu einer Anzahl $C \rightarrow C_{21}^k$ beitragen, näher am Pixelzentrum 56 lokalisiert als Koinzidenz-Zählsignale, welche zu einer Anzahl $C \rightarrow C_{11}^k$ beitragen. Dem zentralen Subpixel 55 wird die gezählte Anzahl T an Zählsignalen des überlappenden Pixelelements 50, definiert durch das Pixelraster G1 zugeordnet, wobei entsprechend die Anzahlen C an Koinzidenz-Zählsignalen subtrahiert werden. Der Index k bezeichnet wie im obigen Beispiel Fig. 6 die Art der Koinzidenz k, d.h. im Wesentlichen das weitere verknüpfte Pixelelement k, auf welchem die Koinzidenz-Zählsignale basieren, in Form der relativen geographischen Angabe.

Dieser Variante wird die Annahme zugrunde gelegt, dass die Anzahlen C an Koinzidenz-Zählsignalen mit einer (energie-)symmetrischen Überschreitung an Energieschwellen im betrachteten und benachbarten Pixelelement 50 mit höherer Wahrscheinlichkeit Ereignisse enthalten, welche durch eine grenznahe Interaktion und der damit einhergehenden gleichmäßigeren Aufteilung der deponierten Energie auf mehrere Pixelelemente mit höherer Wahrscheinlichkeit lediglich jeweils die ersten Energieschwelle überschritten haben. Wohingegen eine (energie-)unsymmetrische Koinzidenz auf einen größeren Überlapp der deponierten Energie mit einem der beteiligten ursprünglichen Pixelelementen und damit gegebenenfalls auf einen weiter von der Pixelkante entfernteren Interaktionspunkt hinweist.

Es kann außerdem im Allgemeinen bei der Wahl der Subpixelgrößen des Subpixelrasters G2 vorteilhaft sein, die Pixelgröße d.h. die Pixelfläche der Subpixel 55, in Abhängigkeit von zumindest einem physikalischen Parameter, welcher in Verbindung mit der Ladungsverteilung der eintreffenden Röntgenstrahlung im Konverterelement 3 steht, zu wählen. Ein physikalischer Parameter kann beispielsweise den erwarteten Durchmesser der durch die Absorption eines Röntgenphotons generierten Ladungsverteilung im Konverterelement umfassen. Er kann die erwartete, mittlere Reichweite von durch die Absorption eines Röntgenphotons erzeugten Fluoreszenzen im Sensormaterial umfassen. Dabei kann beispielsweise die Energieverteilung der eintreffenden Röntgenstrahlung berücksichtigt werden. Damit kann vorteilhaft ein gleichmäßigeres Verhältnis Statistik/Pixelfläche und damit ein einheitlichen Signal-zu-Rausch Verhältnis erreicht werden. Dies kann vorteilhaft zu einem gleichmäßigeren Bildeindruck führen.

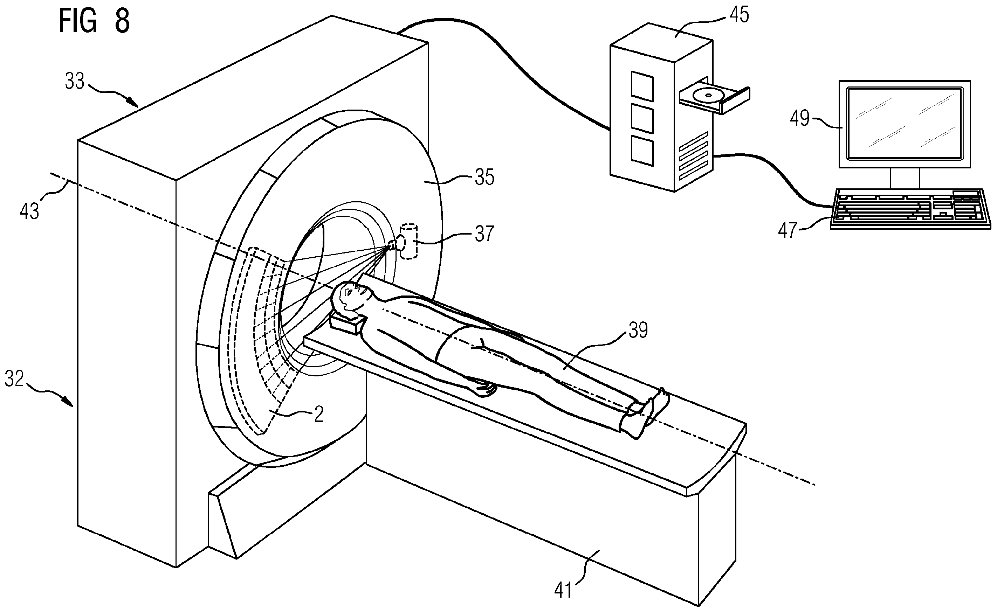

Die Fig. 8 zeigt eine beispielhafte Ausführungsform eines erfindungsgemäßen medizinischen Bildgebungsgerät 32 in Form eines Computertomographie-Systems. Das Computertomographie-System 32 umfasst eine Gantry 33 mit einem Rotor 35. Der Rotor 35 umfasst eine Strahlungsquelle oder Röntgenquelle 37 und eine Detektorvorrichtung 2. Die Detektorvorrichtung 2 weist mindestens einen Röntgendetektor 1 auf. Die Detektorvorrichtung 2 kann insbesondere ein erfindungsgemäßes Röntgendetektorsystem 51 umfassend zumindest einen Röntgendetektor 1 und eine Erzeugungseinheit 71, aufweisen. Das Röntgendetektorsystem 51 kann jedoch auch aufgeteilt an physikalisch unterschiedlichen Orten des medizinischen Bildgebungsgeräts 32 angeordnet sein. Beispielsweise kann der oder die Röntgendetektoren 1 eines Röntgendetektorsystems 51 von der Detektionsvorrichtung 2 umfasst und am Rotor angeordnet sein und die Erzeugungseinheit 71 davon physikalisch getrennt, jedoch signaltechnisch verbunden, beispielsweise in einem statischen Teil des medizinischen Bildgebungsgerät 32 angeordnet sein. Zur Steuerung des Bildgebungsgeräts 32 wird eine Recheneinheit 45 verwendet. Beispielsweise kann die Erzeugungseinheit 71 des erfindungsgemäßen Röntgendetektorsystems 51 auch von der Recheneinheit 45 umfasst sein. Eine Eingabeeinheit 47 und eine Ausgabeeinheit 49 sind mit der Recheneinheit 45 verbunden. Eine Eingabeeinheit 47 und/oder Ausgabeeinheit 49 ermöglicht beispielsweise die manuelle Interaktion eines Anwenders, beispielsweise, das Starten oder Stoppen des erfindungsgemäßen Verfahrens.

Die Erzeugungseinheit 71 und/oder die Recheneinheit 45 kann in Form eines Computers, eines Mikrocontrollers oder eines integrierten Schaltkreises umgesetzt sein. Die Erzeugungseinheit 71 und/oder die Recheneinheit 45 kann Hardware-Elemente oder Software-Elemente aufweisen, beispielsweise einen Mikroprozessor oder ein sogenanntes FPGA (englisches Akronym für "Field Programmable Gate Array"). Es kann sich auch um einen realen oder virtuellen Verbund von Computern handeln (ein englischer Fachbegriff für einen realen Verbund ist "Cluster", ein englischer Fachbegriff für einen virtuellen Verbund ist "Cloud").

Das Untersuchungsobjekt 39, hier der Patient, ist auf der Patientenliege 41 gelagert und ist entlang der Rotationsachse z 43 durch die Gantry 33 bewegbar. Das Objekt kann beispielsweise auch ein tierisches Untersuchungsobjekt sein.

Für die Aufnahme eines Röntgenbilddatensatzes rotiert in der Regel die Detektionsvorrichtung 2 umfassend den Röntgendetektor 1 und die Röntgenquelle 37 um die Rotationsachse z, wobei aus unterschiedlichen Winkelbereichen Messdaten mittels des Röntgendetektors 1 basierend auf der das Objekt 39 passierenden Röntgenstrahlung aufgenommen werden. Basierend auf den ggf. vorverarbeiteten Messdaten können mittels eines Rekonstruktionsalgorithmus, bspw. einer gefilterten Rückprojektion oder einem iterativen Rekonstruktionsalgorithmus, dreidimensionale Volumenbilddaten erzeugt werden. Ausgehend von diesen dreidimensionalen Volumenbilddaten können auch zweidimensionale Schichtbilder, welche jeweils ein Schnittbild durch das abgebildete Volumen darstellen, erzeugt werden.

Im Rahmen der Erfindung können die Messdaten die ermittelten Anzahlen T an Zählsignalen und die ermittelten Anzahlen C an Koinzidenz-Zählsignalen in den Pixelelementen 50 der Vielzahl an Pixelelementen 50 bzw. Teilzahl der Vielzahl an Pixelelementen 50 umfassen, wobei die Anzahlen C an Koinzidenz-Zählsignalen für die Erzeugung des Bilddatensatzes in eine Vorverarbeitung der Messdaten, die Bildrekonstruktion selbst und/oder auch in eine Nachverarbeitung eines rekonstruierten vorläufigen Bilddatensatzes eingehen können.

**Patentansprüche**

1. Verfahren zur Erzeugung eines Röntgenbilddatensatzes mittels eines photonenzählenden Röntgendetektors (1) aufweisend ein Konverterelement (3), ausgebildet Röntgenstrahlung in ein elektrisches Signal umzuwandeln, und mit einer Vielzahl an Pixelelementen (50), wobei jedes Pixelelement (50) der Vielzahl an Pixelelementen (50) eine Anzahl an Komparatoren (19) mit jeweils einer einstellbaren Energieschwelle (S) aufweist und jeweils ausgebildet ist, ein Zählsignal basierend auf einem in einem Pixelelement (50) der Vielzahl an Pixelelementen (50) direkt eingegangenem Signal zu bilden, und wobei mindestens eine Teilzahl der Vielzahl an Pixelelementen (50) ausgebildet ist, ein Koinzidenz-Zählsignal zu bilden, welches auf dem in einem Pixelelement (50) der Teilzahl der Vielzahl an Pixelelementen (50) direkt eingegangenem Signal und auf einem koinzidierend auftretendem Signal zumindest eines weiteren Pixelelements (50) der Vielzahl an Pixelelementen (50) basiert, umfassend die Schritte

   - Erstes Zählen (V1) zumindest einer Anzahl (T) an Zählsignalen in Abhängigkeit der eintreffenden Röntgenstrahlung in jedem Pixelelement (50) der Vielzahl an Pixelelementen (50), wobei die zumindest eine Anzahl (T) an Zählsignalen in jedem Pixelelement (50) der Vielzahl an Pixelelementen (50) basierend auf einem Ausgangssignal zumindest eines Komparators (19) der Anzahl an Komparatoren (19) eines jeweiligen Pixelelements (50) gezählt wird,
   - Zweites Zählen (V2) zumindest einer Anzahl (C) an Koinzidenz-Zählsignalen in jedem Pixelelement (50) der Teilzahl der Vielzahl an Pixelelementen (50) mit zumindest einem weiteren Pixelelement (50) der Vielzahl an Pixelelementen (50), wobei die zumindest eine Anzahl (C) an Koinzidenz-Zählsignalen in jedem Pixelelement (50) der Teilzahl an Pixelelementen (50) basierend auf dem Ausgangssignal zumindest eines Komparators (19) der Anzahl an Komparatoren (19) des jeweiligen Pixelelements (50) und zumindest auf dem Ausgangssignal eines Komparators (19) der Anzahl an Komparatoren (19) des zumindest einen weiteren Pixelelements (50) gezählt wird, und für das zweite Zählen (V2) der zumindest einen Anzahl (C) an Koinzidenz-Zählsignalen die einstellbare Energieschwelle (S) des zumindest einen Komparators (19) der Anzahl an Komparatoren (19) des jeweiligen Pixelelements (50) der Teilzahl der Vielzahl an Pixelelementen (50) und die einstellbare Energieschwelle (S) des zumindest einen Komparators (19) der Anzahl an Komparatoren (19) des zumindest einen weiteren Pixelelements (50), auf welchen die Koinzidenz-Zählsignale basieren, unterschiedliche Energieschwellwerte aufweisen,
   - Erzeugen (V3) eines Röntgenbilddatensatzes basierend auf der in jedem Pixelelement (50) der Vielzahl an Pixelelementen (50) gezählten zumindest einen Anzahl (T) an Zählsignalen und der in jedem Pixelelement (50) der Teilzahl der Vielzahl an Pixelelementen (50) gezählten zumindest einen Anzahl (C) an Koinzidenz-Zählsignalen.

2. Verfahren nach Anspruch 1, wobei im Schritt des Erzeugens (V3) die in den Pixelelementen (50) der Teilzahl der Vielzahl an Pixelelementen (50) gezählte zumindest eine Anzahl (C) an Koinzidenz-Zählsignalen in die Datenvorverarbeitung vor einer Bildrekonstruktion, in die Bildrekonstruktion oder in einen der Bildrekonstruktion nachgelagerten Nachverarbeitungsschritt eingeht.

3. Verfahren nach einem der Ansprüche 1 oder 2, wobei zumindest in jedem Pixelelement (50) der Teilzahl der Vielzahl an Pixelelementen (50) die zumindest eine Anzahl (T) an Zählsignalen mittels der zumindest einen Anzahl (C) an Koinzidenz-Zählsignalen angepasst wird.

4. Verfahren nach einem der Ansprüche 1 bis 3, wobei basierend auf der zumindest einen Anzahl (T) an Zählsignalen in jedem Pixelelement (50) der Vielzahl an Pixelelementen (50) ein vorläufiger Bilddatensatz erzeugt wird und wobei basierend auf der zumindest einen Anzahl (C) an Koinzidenz-Zählsignalen in jedem Pixelelement der Teilzahl an Pixelelementen ein Koinzidenz-Bilddatensatz erzeugt wird, welcher im Schritt des Erzeugens des Röntgenbilddatensatzes auf den vorläufigen Bilddatensatz angewendet wird.

5. Verfahren nach einem der Ansprüche 1 bis 4, wobei der Schritt des Erzeugens (V3) das Anwenden einer trainierten Funktion umfasst, wobei die zumindest eine Anzahl (C) an Koinzidenz-Zählsignale in zumindest einem Pixelelement (50) der Teilzahl der Vielzahl an Pixelelementen (50) als Eingabeparameter in die trainierte Funktion eingeht.

6. Verfahren nach einem der Ansprüche 1 bis 5, wobei in jedem Pixelelement (50) der Teilzahl an Pixelelementen (50) außerdem zumindest eine weitere Anzahl (C) an Koinzidenz-Zählsignalen gezählt wird, wobei die einstellbare Energieschwelle (S) des zumindest einen Komparators (19) der Anzahl an Komparatoren (19) des jeweiligen Pixelelements (50) der Teilzahl der Vielzahl an Pixelelementen (50) und die einstellbare Energieschwelle (S) des zumindest einen Komparators (19) der Anzahl an Komparatoren (19) des zumindest einen weiteren Pixelelements (50), auf welchen die Koinzidenz-Zählsignale basieren, den gleichen Energieschwellwert aufweisen.

7. Verfahren nach einem der Ansprüche 1 bis 6, wobei jedes Pixelelement (50) der Teilzahl der Vielzahl an Pixelelementen (50) eine Mehrzahl an Komparatoren (19) aufweist, wobei basierend auf den jeweiligen Ausgangssignalen von mehr als einem Komparator (19) jeweils eine Anzahl (C) an Koinzidenz-Zählsignalen mit jeweils zumindest einem weiteren Pixelelement (50) der Vielzahl an Pixelelementen (50) gezählt wird.

8. Verfahren nach einem der Ansprüche 1 bis 7, wobei das zumindest eine weitere Pixelelement (50) ein direkt benachbartes oder ein diagonal benachbartes Pixelelement (50) umfasst.

9. Verfahren nach einem der Ansprüche 1 bis 8, wobei Koinzidenz-Zählsignale in jedem Pixelelement (50) der Teilzahl der Vielzahl an Pixelelementen (50) mit zwischen einem weiteren Pixelelement (50) und 24 weiteren Pixelelementen (50) der Vielzahl an Pixelelementen (50) gebildet werden und wobei die im Schritt des zweiten Zählens (V2) für jedes Pixelelement (50) der Teilzahl der Vielzahl an Pixelelementen (50) gezählte zumindest eine Anzahl (C) an Koinzidenz-Zählsignalen auf koinzidierend auftretenden Signalen aller weiteren Pixelelemente (50) basiert.

10. Verfahren nach einem der Ansprüche 1 bis 9, wobei Koinzidenz-Zählsignale in jedem Pixelelement (50) der Teilzahl der Vielzahl an Pixelelementen (50) mit zwischen einem weiteren Pixelelement (50) und 24 weiteren Pixelelementen (50) der Vielzahl an Pixelelementen (50) gebildet werden und wobei im Schritt des zweiten Zählens (V2) jeweils zumindest eine Anzahl (C) an Koinzidenz-Zählsignalen mit jedem der weiteren Pixelelemente (50) gezählt wird.

11. Verfahren nach einem der Ansprüche 1 bis 10, außerdem umfassend den Schritt

- Übertragen (V4) zumindest einer in einem Pixelelement (50) der Teilzahl der Vielzahl an Pixelelementen (50) gezählten Anzahl (C) an Koinzidenz-Zählsignalen auf zumindest eines der weiteren Pixelelemente (50) der Vielzahl an Pixelelementen (50), mit welchem in dem jeweiligen Pixelelement (50) der Teilzahl ein Koinzidenz-Zählsignal gebildet wird, und

wobei der Schritt des Erzeugens (V3) außerdem auf zumindest einer übertragenen Anzahl (C) an Koinzidenz-Zählsignalen basiert.

12. Verfahren nach einem der Ansprüche 1 bis 11, wobei für jedes Pixelelement (50) der Vielzahl an Pixelelementen (50) zumindest eine gezählte oder eine übertragene Anzahl (C) an Koinzidenz-Zählsignalen ermittelt wird.

13. Verfahren nach Anspruch 10 oder nach einem der Ansprüche 11 oder 12 in Kombination mit Anspruch 10, wobei durch die Anordnung der Vielzahl an Pixelelemente (50) ein erstes matrixartiges Pixelraster (G1) definiert ist, und wobei im Schritt des Erzeugens (V3)

- basierend auf dem Pixelraster (G1) ein Subpixelraster (G2) an mit den Pixelelementen (50) der Vielzahl an Pixelelementen (50) überlappenden Subpixeln (55) definiert wird, wobei das Subpixelraster (G2) zumindest entlang einer Rasterdimension einen relativ zum Pixelraster (G1) reduzierten Rasterabstand (R2) aufweist,
- jedem Subpixel (55) zumindest eine virtuelle Anzahl (Z) an Zählsignalen zugeordnet wird, wobei die jeweilige zugeordnete zumindest eine virtuelle Anzahl (Z) zumindest eines Teils der Subpixel (55) auf einer gezählten oder einer übertragenen Anzahl (C) an Koinzidenz-Zählsignalen eines mit dem jeweiligen Subpixel (55) überlappenden Pixelelements (50) basiert, und

wobei der Röntgenbilddatensatz basierend auf der den virtuellen Subpixeln (50) jeweils zugeordneten virtuellen Anzahl (Z) an Zählsignalen erzeugt wird.

14. Verfahren nach Anspruch 13, wobei die einem jeweiligen Subpixel (55) zugeordnete virtuelle Anzahl (Z) an Zählsignalen abhängig ist vom Abstand des virtuellen Subpixels (55) zum Zentrum (56) eines mit dem Subpixel (55) überlappenden Pixelelements (50) der Vielzahl an Pixelelementen (50).

15. Verfahren nach einem der Ansprüche 13 oder 14, wobei die Fläche der virtuellen Subpixel (55) im Subpixelraster (G2) unterschiedlich ist.

16. Röntgendetektorsystem (51), ausgebildet ein Verfahren gemäß einem der Ansprüche 1 bis 15 auszuführen, aufweisend

- Einen photonenzählenden Röntgendetektor (1) mit einem Konverterelement (3), ausgebildet Röntgenstrahlung in ein elektrisches Signal umzuwandeln, und mit einer Vielzahl an Pixelelementen (50), wobei

o jedes Pixelelement (50) der Vielzahl an Pixelelementen (50) eine Anzahl an Komparatoren (19) mit jeweils einer einstellbaren Energieschwelle (S) aufweist,
o jedes Pixelelement (50) der Vielzahl an Pixelelementen (50)jeweils ausgebildet ist, ein Zählsignal basierend auf einem in einem Pixelelement (50) der Vielzahl an Pixelelementen (50) direkt eingegangenem Signal zu bilden, wobei das Zählsignal auf einem Ausgangssignal zumindest eines Komparators (19) der Anzahl an Komparatoren (19) eines jeweiligen Pixelelements (50) basiert, und
o mindestens eine Teilzahl der Vielzahl an Pixelelementen (50) ausgebildet ist, ein Koinzidenz-Zählsignal zu bilden, welches auf dem in einem Pixelelement (50) der Teilzahl der Vielzahl an Pixelelementen (50) direkt eingegangenem Signal und auf einem koinzidierend auftretendem Signal zumindest eines weiteren Pixelelements (50) der Vielzahl an Pixelelementen (50) basiert, wobei das Koinzidenz-Signal auf dem Ausgangssignal zumindest eines Komparators (19) der Anzahl an Komparatoren (19) des jeweiligen Pixelelements (50) und zumindest auf dem Ausgangssignal eines Komparators (19) der Anzahl an Komparatoren (19) des zumindest einen weiteren Pixelelements (50) basiert, und wobei die einstellbare Energieschwelle (S) des zumindest einen Komparators (19) der Anzahl an Komparatoren (19) des jeweiligen Pixelelements (50) der Teilzahl der Vielzahl an Pixelelementen (50) und die einstellbare Energieschwelle (S) des zumindest einen Komparators (19) der Anzahl an Komparatoren (19) des zumindest einen weiteren Pixelelements (50), auf welchen die Koinzidenz-Zählsignale basieren, unterschiedliche Energieschwellwerte aufweisen, und

- eine Erzeugungseinheit (71) ausgebildet einen Röntgenbilddatensatz basierend auf einer in jedem Pixelelement (50) der Vielzahl an Pixelelementen (50) gezählten zumindest einen Anzahl (T) an Zählsignalen und basierend auf einer in jedem Pixelelement (50) der Teilzahl der Vielzahl an Pixelelementen (50) gezählten zumindest einen Anzahl (C) an Koinzidenz-Zählsignalen zu erzeugen.

17. Medizinisches Bildgebungsgerät (32), insbesondere Computertomographie-System, umfassend ein Röntgendetektorsystem (51) nach Anspruch 16.

**Claims**

1. Method for generating an X-ray image dataset by means of a photon-counting X-ray detector (1) having a converter element (3) which is configured to convert X-ray radiation into an electrical signal, and having a multiplicity of pixel elements (50), wherein each pixel element (50) of the multiplicity of pixel elements (50) has a quantity of comparators (19), each with an adjustable energy threshold (S) and each being configured to form a count signal based upon a signal directly entering a pixel element (50) of the multiplicity of pixel elements (50) and wherein at least a subset of the multiplicity of pixel elements (50) is configured to form a coincidence count signal which is based upon the signal directly entering a pixel element (50) of the subset of the multiplicity of pixel elements (50) and upon a coincidentally occurring signal of at least one further pixel element (50) of the multiplicity of pixel elements (50), comprising the steps

- first counting (V1) of at least one quantity (T) of count signals dependent upon the incident X-ray radiation in each pixel element (50) of the multiplicity of pixel elements (50), wherein the at least one quantity (T) of count signals is counted in each pixel element (50) of the multiplicity of pixel elements (50), based upon an output signal of at least one comparator (19) of the quantity of comparators (19) of a respective pixel element (50),
- second counting (V2) of at least one quantity (C) of coincidence count signals in each pixel element (50) of the subset of the multiplicity of pixel elements (50) with at least one further pixel element (50) of the multiplicity of pixel elements (50), wherein the at least one quantity (C) of coincidence count signals in each pixel element (50) of the subset of pixel elements (50) is counted based upon the output signal of at least one comparator (19) of the quantity of comparators (19) of the respective pixel element (50) and at least upon the output signal of a comparator (19) of the quantity of comparators (19) of the at least one further pixel element (50), and, for the second counting (V2) of the at least one quantity (C) of coincidence count signals, the adjustable energy threshold (S) of the at least one comparator (19) of the quantity of comparators (19) of the respective pixel element (50) of the subset of the multiplicity of pixel elements (50) and the adjustable energy threshold (S) of the at least one comparator (19) of the quantity of comparators (19) of the at least one further pixel element (50), upon which the coincidence count signals are based, have different energy threshold values,
- generating (V3) an X-ray image dataset based upon the at least one quantity (T) of count signals counted in each pixel element (50) of the multiplicity of pixel elements (50) and the at least one quantity (C) of coincidence count signals counted in each pixel element (50) of the subset of the multiplicity of pixel elements (50).

2. Method according to claim 1, wherein in the step of generating (V3), the at least one quantity (C) of coincidence count signals counted in the pixel elements (50) of the subset of the multiplicity of pixel elements (50) enters into the data preprocessing before an image reconstruction, into the image reconstruction or into a postprocessing step downstream of the image reconstruction.

3. Method according to one of claims 1 or 2, wherein at least in each pixel element (50) of the subset of the multiplicity of pixel elements (50), the at least one quantity (T) of count signals is adapted by means of the at least one quantity (C) of coincidence count signals.

4. Method according to one of claims 1 to 3, wherein, based upon the at least one quantity (T) of count signals in each pixel element (50) of the multiplicity of pixel elements (50), a preliminary image dataset is generated, and wherein, based upon the at least one quantity (C) of coincidence count signals in each pixel element of the subset of pixel elements, a coincidence image dataset is generated which is applied to the preliminary image dataset in the step of generating the X-ray image dataset.

5. Method according to one of claims 1 to 4, wherein the step of generating (V3) comprises the use of a trained function, wherein the at least one quantity (C) of coincidence count signals in at least one pixel element (50) of the subset of the multiplicity of pixel elements (50) enters into the trained function as an input parameter.

6. Method according to one of claims 1 to 5, wherein in each pixel element (50) of the subset of pixel elements (50), at least one further quantity (C) of coincidence count signals is also chosen, wherein the adjustable energy threshold (S) of the at least one comparator (19) of the quantity of comparators (19) of the respective pixel element (50) of the subset of the multiplicity of pixel elements (50) and the adjustable energy threshold (S) of the at least one comparator (19) of the quantity of comparators (19) of the at least one further pixel element (50), upon which the coincidence count signals are based, have the same energy threshold value.

7. Method according to one of claims 1 to 6, wherein each pixel element (50) of the subset of the multiplicity of pixel elements (50) has a plurality of comparators (19), wherein based upon the respective output signals from more than one comparator (19), in each case, a quantity (C) of coincidence count signals with at least one further pixel element (50) of the multiplicity of pixel elements (50) is counted.

8. Method according to one of claims 1 to 7, wherein the at least one further pixel element (50) comprises a directly adjacent or a diagonally adjacent pixel element (50).

9. Method according to one of claims 1 to 8, wherein coincidence count signals are formed in each pixel element (50) of the subset of the multiplicity of pixel elements (50) with between one further pixel element (50) and 24 further pixel elements (50) of the multiplicity of pixel elements (50) and wherein the at least one quantity (C) of coincidence count signals counted in the step of the second counting (V2) for each pixel element (50) of the subset of the multiplicity of pixel elements (50) is based upon coincidentally occurring signals of all the further pixel elements (50).

10. Method according to one of claims 1 to 9, wherein coincidence count signals are formed in each pixel element (50) of the subset of the multiplicity of pixel elements (50) with between one further pixel element (50) and 24 further pixel elements (50) of the multiplicity of pixel elements (50) and wherein, in the step of the second counting (V2), at least one quantity (C) of coincidence count signals is counted with each of the further pixel elements (50) in each case.

11. Method according to one of claims 1 to 10, also comprising the step

   - transferring (V4) at least one quantity (C) of coincidence count signals counted in a pixel element (50) of the subset of the multiplicity of pixel elements (50) to at least one of the further pixel elements (50) of the multiplicity of pixel elements (50) with which a coincidence count signal is formed in the respective pixel element (50) of the subset, and

   wherein the step of generating (V3) is further based upon at least one transferred quantity (C) of coincidence count signals.

12. Method according to one of claims 1 to 11, wherein for each pixel element (50) of the multiplicity of pixel elements (50), at least one counted or transferred quantity (C) of coincidence count signals is ascertained.

13. Method according to claim 10 or according to one of claims 11 or 12 in combination with claim 10, wherein through the arrangement of the multiplicity of pixel elements (50), a first matrix-like pixel grid (G1) is defined, and wherein in the step of generating (V3)

   - based upon the pixel grid (G1), a subpixel grid (G2) of subpixels (55) overlapping with the pixel elements (50) of the multiplicity of pixel elements (50) is defined, wherein the subpixel grid (G2) has a reduced grid spacing (R2), at least along one grid dimension, relative to the pixel grid (G1),
   - at least one virtual quantity (Z) of count signals is assigned to each subpixel (55), wherein the respectively assigned at least one virtual quantity (Z) of at least one portion of the subpixels (55) is based upon a counted or transferred quantity (C) of coincidence count signals of a pixel element (50) overlapping with the respective subpixel (55), and

   wherein the X-ray image dataset is generated based upon the virtual quantity (Z) of count signals respectively assigned to the virtual subpixels (50).

14. Method according to claim 13, wherein the virtual quantity (Z) of count signals assigned to one respective subpixel (55) is dependent upon the spacing of the virtual subpixel (55) from the centre (56) of a pixel element (50) of the multiplicity of pixel elements (50) overlapping the subpixel (55).

15. Method according to one of claims 13 or 14, wherein the area of the virtual subpixel (55) in the subpixel grid (G2) differs.

16. X-ray detector system (51), configured to carry out a method according to one of claims 1 to 15, having

   - a photon-counting X-ray detector (1) having a converter element (3) which is configured to convert X-ray radiation into an electrical signal, and having a multiplicity of pixel elements (50), wherein

      ∘ each pixel element (50) of the multiplicity of pixel elements (50) has a quantity of comparators (19), each with an adjustable energy threshold (S),
      ∘ each pixel element (50) of the multiplicity of pixel elements (50) is in each case configured to form a count signal based upon a signal directly entering a pixel element (50) of the multiplicity of pixel elements (50), wherein the count signal is based upon an output signal of at least one comparator (19) of the quantity of comparators (19) of a respective pixel element (50), and

o at least a subset of the multiplicity of pixel elements (50) is configured to form a coincidence count signal which is based upon the signal directly entering a pixel element (50) of the subset of the multiplicity of pixel elements (50) and upon a coincidentally occurring signal of at least one further pixel element (50) of the multiplicity of pixel elements (50), wherein the coincidence signal is based upon the output signal of at least one comparator (19) of the quantity of comparators (19) of the respective pixel element (50) and at least upon the output signal of a comparator (19) of the quantity of comparators (19) of the at least one further pixel element (50), and wherein the adjustable energy threshold (S) of the at least one comparator (19) of the quantity of comparators (19) of the respective pixel element (50) of the subset of the multiplicity of pixel elements (50) and the adjustable energy threshold (S) of the at least one comparator (19) of the quantity of comparators (19) of the at least one further pixel element (50), upon which the coincidence count signals are based, have different energy threshold values, and

- a generating unit (71) configured to generate an X-ray image dataset based upon at least one quantity (T) of count signals counted in each pixel element (50) of the multiplicity of pixel elements (50) and upon at least one quantity (C) of coincidence count signals counted in each pixel element (50) of the subset of the multiplicity of pixel elements (50).

17. Medical imaging device (32), in particular a computed tomography system comprising an X-ray detector system (51) according to claim 16.

## Revendications

1. Procédé de production d'un ensemble de données d'image aux rayons X au moyen d'un détecteur (1) de rayons X comportant les photons comptant un élément (3) convertisseur, constitué pour transformer du rayonnement X en un signal électrique, et comprenant une pluralité d'éléments (50) de pixel, dans lequel chaque élément (50) de pixel de la pluralité d'éléments (50) de pixel a un nombre de comparateurs (19) ayant chacun un seuil (S) d'énergie réglable et est constitué respectivement pour former un signal de dénombrement sur la base d'un signal entré directement dans un élément (50) de pixel de la pluralité (50) d'éléments de pixel, et dans lequel au moins un nombre partiel de pluralité d'éléments (50) de pixel est constitué pour former un signal de dénombrement de coïncidence, qui repose sur le signal entré directement dans un élément (50) de pixel du nombre partiel de la pluralité d'éléments (50) de pixel et sur un signal se produisant en coïncidence d'au moins un autre élément (50) de pixel de la pluralité d'éléments (50) de pixel, comprenant les stades

- premier dénombrement (V1) d'au moins un nombre (T) de signaux de dénombrement en fonction du rayonnement X incident dans chaque élément (50) de pixel de la pluralité d'éléments (50) de pixel, dans lequel on dénombre le au moins un nombre (T) de signaux de dénombrement dans chaque élément (50) de pixel de la pluralité d'éléments (50) de pixel sur la base d'un signal de sortie d'au moins un comparateur (19) du nombre de comparateurs (19) d'un élément (50) de pixel respectif,
- deuxième dénombrement (V2) d'au moins un nombre (C) de signaux de dénombrement de coïncidence dans chaque élément (50) de pixel du nombre partiel de la pluralité d'éléments (50) de pixel ayant au moins un autre élément (50) de pixel de la pluralité d'éléments (50) de pixel, dans lequel on dénombre le au moins un nombre (C) de signaux de dénombrement de coïncidence dans chaque élément (50) de pixel du nombre partiel d'éléments (50) de pixel sur la base du signal de sortie d'au moins un comparateur (19) du nombre de comparateurs (19) de chaque élément (50) de pixel et d'au moins le signal de sortie d'un comparateur (19) du nombre de comparateurs (19) du au moins un autre élément (50) de pixel, et pour le deuxième dénombrement (V2) du au moins un nombre (C) de signaux de dénombrement de coïncidence, le seuil (S) d'énergie réglable du au moins un comparateur (19) du nombre de comparateurs (19) de l'élément (50) de pixel respectif du nombre partiel de la pluralité d'éléments (50) de pixel et le seuil (S) d'énergie réglable du au moins un comparateur (19) du nombre de comparateurs (19) du au moins un autre élément (50) de pixel, sur lequel reposent les signaux de dénombrement de coïncidence, ont des valeurs de seuil d'énergie différentes,
- production (V3) d'un ensemble de données d'image aux rayons X sur la base d'au moins un nombre (T) de signaux de dénombrement, dénombré dans chaque élément (50) de pixel de la pluralité d'éléments (50) de pixel, et d'au moins un nombre (C), dénombré dans chaque élément (50) de pixel du nombre partiel de la pluralité d'éléments (50) de pixel, de signaux de dénombrement de coïncidence.

2. Procédé suivant la revendication 1, dans lequel, dans le stade de la production (V3), le au moins un nombre (C), dénombré dans les éléments (50) de pixel du nombre partiel de la pluralité d'éléments (50) de pixel de signaux de

**EP 3 839 577 B1**

dénombrement de coïncidence, entre dans le dispositif de traitement de données avant une reconstruction d'image, dans la reconstruction d'image ou dans un stade de traitement ultérieur en aval de la reconstruction d'image.

3. Procédé suivant la revendication 1 ou 2, dans lequel on adapte au moins dans chaque élément (50) de pixel du nombre partiel de la pluralité d'éléments (50) de pixel le au moins un nombre (T) de signaux de dénombrement, au moyen du au moins un nombre (C) de signaux de dénombrement de coïncidence.

4. Procédé suivant l'une des revendications 1 à 3, dans lequel, sur la base du au moins un nombre (T) de signaux de dénombrement dans chaque élément (50) de pixel de la pluralité d'éléments (50) de pixel, on produit un ensemble de données d'image provisoire et dans lequel, sur la base du au moins un nombre (C) de signaux de dénombrement de coïncidence dans chaque élément de pixel du nombre partiel d'éléments de pixel, on produit un ensemble de données d'image de coïncidence, que l'on applique, dans le stade de la production de l'ensemble de données d'image aux rayons X, à l'ensemble de données d'image provisoire.

5. Procédé suivant l'une des revendications 1 à 4, dans lequel le stade de la production (V3) comprend l'application d'une fonction entraînée, dans lequel le au moins un nombre (C) de signaux de dénombrement de coïncidence entre, comme paramètre d'entrée dans la fonction entraînée, dans au moins un élément (50) de pixel du nombre partiel de la pluralité d'éléments (50) de pixel.

6. Procédé suivant l'une des revendications 1 à 5, dans lequel on dénombre, dans chaque élément (50) de pixel du nombre partiel d'éléments (50) de pixel en outre au moins un autre nombre (C) de signaux de dénombrement de coïncidence, dans lequel le seuil (S) d'énergie réglable du au moins un comparateur (19) du nombre de comparateurs (19) de l'élément (50) de pixel respectif du nombre partiel de la pluralité d'éléments (50) de pixel et le seuil (S) d'énergie réglable du au moins un comparateur (19) du nombre de comparateurs (19) du au moins un autre élément (50) de pixel, sur lesquels reposent les signaux de dénombrement de coïncidence, ont la même valeur de seuil d'énergie.

7. Procédé suivant l'une des revendications 1 à 6, dans lequel chaque élément (50) de pixel du nombre partiel de la pluralité d'éléments (50) de pixel a une multiplicité de comparateurs (19), dans lequel, sur la base des signaux de sortie respectifs de plus d'un comparateur (19), on dénombre respectivement un nombre (C) de signaux de dénombrement de coïncidence avec respectivement au moins un autre élément (50) de pixel de la pluralité d'éléments (50) de pixel.

8. Procédé suivant l'une des revendications 1 à 7, dans lequel le au moins un autre élément (50) de pixel comprend un élément (50) de pixel directement voisin ou voisin en diagonale.

9. Procédé suivant l'une des revendications 1 à 8, dans lequel on forme des signaux de dénombrement de coïncidence dans chaque élément (50) de pixel du nombre partiel de la pluralité d'éléments (50) de pixel avec entre un autre élément (50) de pixel et 24 autres éléments (50) de pixel de la pluralité d'éléments (50) de pixel et dans lequel le au moins un nombre (C), dénombré dans le stade du deuxième dénombrement (V2) pour chaque élément (50) de pixel du nombre partiel de la pluralité d'éléments (50) de pixel, de signaux de dénombrement de coïncidence repose sur des signaux se produisant en coïncidence de tous les autres éléments (50) de pixel.

10. Procédé suivant l'une des revendications 1 à 9, dans lequel on forme des signaux de dénombrement de coïncidence dans chaque élément (50) de pixel du nombre partiel de la pluralité d'éléments (50) de pixel avec entre un autre élément (50) de pixel et 24 autres éléments (50) de pixel de la pluralité d'éléments (50) de pixel et dans lequel, dans le stade du deuxième dénombrement (V2), on dénombre respectivement au moins un nombre (C) de signaux de dénombrement de coïncidence avec chacun des autres éléments (50) de pixel.

11. Procédé suivant l'une des revendications 1 à 10, comprenant en outre le stade

- transfert (V4) d'au moins un nombre (C), dénombré dans un élément (50) de pixel du nombre partiel de la pluralité d'éléments (50) de pixel, de signaux de dénombrement de coïncidence à au moins l'un des autres éléments (50) de pixel de la pluralité d'éléments (50) de pixel, par lequel on forme, dans l'élément (50) de pixel respectif du nombre partiel, un signal de dénombrement de coïncidence, et

dans lequel le stade (V3) de la production repose en outre sur au moins un nombre (C) transféré de signaux de dénombrement de coïncidence.

**12.** Procédé suivant la revendication 1 à 11, dans lequel, pour chaque élément (50) de pixel de la pluralité d'éléments (50) de pixel, on détermine au moins un nombre (C), dénombré ou transféré de signaux de dénombrement de coïncidence.

**13.** Procédé suivant la revendication 10 ou suivant l'une des revendications 11 ou 12 en combinaison avec la revendication 10, dans lequel, par l'agencement de la pluralité d'éléments (50) de pixel, une première trame (G1) de pixel matricielle est définie, et dans lequel, dans le stade de la production (V3)

- sur la base de la trame (G1) de pixel, on définit une trame (G2) de sous-pixel (55) à chevauchement avec l'élément (50) de pixel de la pluralité d'éléments (50) de pixel, dans lequel la trame (G2) de sous-pixel a au moins le long d'une dimension de la trame une distance (R2) de trame réduite relativement à la trame (G1) de pixel,
- on affecte à chaque sous-pixel (55) au moins un nombre (Z) virtuel de signaux de dénombrement, dans lequel le au moins un nombre (Z) virtuel affecté respectivement d'au moins une partie des sous-pixels (55) repose sur un nombre (C), dénombré ou transmis, de signaux de dénombrement de coïncidence d'un élément (50) de pixel à chevauchement avec le sous-pixel (55) respectif, et

dans lequel on produit l'ensemble de données d'image aux rayons X sur la base du nombre (Z) virtuel, associé respectivement au sous-pixel (50) virtuel, de signaux de dénombrement.

**14.** Procédé suivant la revendication 13, dans lequel le nombre (Z) virtuel, affecté à un sous-pixel (55) respectif de signaux de dénombrement, dépend de la distance du sous-pixel (55) virtuel au centre (56) d'un élément (50) de pixel à chevauchement avec le sous-pixel (55) de pluralité d'éléments (50) de pixel.

**15.** Procédé suivant l'une des revendications 13 ou 14, dans lequel la surface des sous-pixels (55) virtuels dans la trame (G2) de sous-pixels est différente.

**16.** Système (51) de détecteur de rayons X, constitué pour exécuter un procédé suivant l'une des revendications 1 à 15, comportant

- un détecteur (1) de rayons X comptant les photons comprenant un élément (3) de convertisseur constitué pour transformer du rayonnement X en un signal électrique et comprenant une pluralité d'éléments (50) de pixel, dans lequel

o chaque élément (50) de pixel de la pluralité d'éléments (50) de pixel a un nombre de comparateurs (19) ayant respectivement un seuil (S) d'énergie réglable,
o chaque élément (50) de pixel de la pluralité d'éléments (50) de pixel est constitué respectivement pour former un signal de dénombrement reposant sur un signal entré directement dans un élément (50) de pixel de la pluralité d'éléments (50) de pixel, dans lequel le signal de dénombrement repose sur un signal de sortie d'au moins un comparateur (19) du nombre de comparateurs (19) d'un élément (50) de pixel respectif, et
o au moins un nombre partiel de la pluralité d'éléments (50) de pixel est constitué pour former un signal de dénombrement de coïncidence, qui repose sur le signal entré directement dans un élément (50) de pixel du nombre partiel de la pluralité d'éléments (50) de pixel et sur un signal se produisant en coïncidence d'au moins un autre élément (50) de pixel de la pluralité d'éléments (50) de pixel, dans lequel le signal de coïncidence repose sur le signal de sortie d'au moins un comparateur (19) du nombre de comparateurs (19) de l'élément (50) de pixel respectif et sur le signal de sortie d'un comparateur (19) du nombre de comparateurs (19) du au moins un autre élément (50) de pixel, et dans lequel le seuil (S) d'énergie réglable du au moins un comparateur (19) du nombre de comparateurs (19) de l'élément (50) de pixel respectif du nombre partiel de la pluralité d'éléments (50) de pixel et le seuil (S) d'énergie réglable du au moins un comparateur (19) du nombre de comparateur (19) du au moins un autre élément (50) de pixel, sur lequel repose les signaux de dénombrement de coïncidence, ont des valeurs de seuil d'énergie différentes, et

- une unité (71) de production constituée pour produire un ensemble de données d'image aux rayons X sur la base d'au moins un nombre (T), dénombré dans chaque élément (50) de pixel de la pluralité d'éléments (50) de pixel, de signaux de dénombrement et sur la base d'au moins un nombre (C), dénombré dans chaque élément (50) de pixel du nombre partiel de la pluralité d'éléments (50) de pixel, de signaux de dénombrement de coïncidence.

**17.** Appareil (32) d'imagerie médicale, en particulier système de tomodensitométrie assistée par ordinateur comprenant un système (51) de détection de rayons X suivant la revendication 16.

FIG 1

# FIG 2

V1

V2

V4

V31

V32

V3

V33

FIG 3

FIG 4

# FIG 5

50

G1

| 1 | 2 | 3 |
| 4 | 5 | 6 |
| 7 | 8 | 9 |

R1

⇒

55

G1  G2

| | a | b | c |
| | d | e | f |
| | g | h | i |

R2

EP 3 839 577 B1

# FIG 6

I

| 1 | 2 | 3 |
| 4 | 5 / 80keV | 120 keV / 6 / 40keV |
| 7 | 8 | 9 |

50

G1

II

T1+1 ■ ● / 120 keV / ■ T1+1 / T2+1

50

G1

III

$C_{11}^{E}+1$ ■ ● / 120 keV / $C_{11}^{W}+1$ ■
$C_{21}^{E}+1$ / $C_{12}^{W}+1$

50

G1

IV

a b c / d e f ■ ● / g h i

55

G1  G2

EP 3 839 577 B1

41

# FIG 7

55

55

$c_{11}^{NW}$  $c_{21}^{NW}$  $c_{11}^{N}$  $c_{21}^{NE}$  $c_{11}^{NE}$

$c_{21}^{NW}$  $c_{21}^{NW}$  $c_{21}^{N}$  $c_{21}^{NE}$  $c_{21}^{NE}$

56

$c_{11}^{W}$  $c_{21}^{W}$  X  $c_{21}^{E}$  $c_{11}^{E}$

$R_2^1$  $R_2^2$

$c_{21}^{SW}$  $c_{21}^{SW}$  $c_{21}^{S}$  $c_{21}^{SE}$  $c_{21}^{SE}$

$c_{11}^{SW}$  $c_{21}^{SW}$  $c_{11}^{S}$  $c_{21}^{SE}$  $c_{11}^{SE}$

G2

G1

FIG 8

EP 3 839 577 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011077859 B4

- DE 102021224209 A1

**In der Beschreibung aufgeführte Nicht-Patentliteratur**

- **VON SCOTT S. HSIEH**. Coincidence counters for charge sharing compensation in spectroscopic photon counting detectors. *IEEE Transactions on Medical Imaging*